# EUROPÄISCHE PATENTSCHRIFT

(11) **EP 2 349 964 B1**
(45) Veröffentlichungstag und Bekanntmachung des Hinweises auf die Patenterteilung: **09.09.2015**
(21) Anmeldenummer: 09783915.3
(22) Anmeldetag: 09.10.2009
(51) Int. Cl.: C07C 39/42, C07C 49/747, C07C 49/755, C07C 69/16, C07C 255/22, C07C 233/33, C07C 309/65, C07D 307/12, C07D 309/12, C07D 407/10, C07D 413/12, C07D 295/088, C07D 313/12, A61K 31/35, A61P 29/00

(54) **DIBENZOCYCLOHEPTANONDERIVATE UND PHARMAZEUTISCHE MITTEL, WELCHE DIESE VERBINDUNGEN ENTHALTEN**
DIBENZOCYCLOHEPTANONE DERIVATIVES AND PHARMACEUTICAL AGENTS CONTAINING SAID COMPOUNDS
DÉRIVÉS DIBENZOCYCLOHEPTANONE ET AGENTS PHARMACEUTIQUES CONTENANT CES COMPOSÉS

(30) Priorität: 09.10.2008 EP 08166223
(43) Veröffentlichungstag der Anmeldung: 03.08.2011
(73) Patentinhaber: c-a-i-r biosciences GmbH, 72076 Tübingen (DE)
(72) Erfinder: LAUFER, Stefan, 72070 Tübingen (DE); ALBRECHT, Wolfgang, 89075 Ulm (DE)
(74) Vertreter: Reitstötter Kinzebach
(86) Internationale Anmeldenummer: PCT/EP2009/063215
(87) Internationale Veröffentlichungsnummer: WO 2010/040843

(56) Entgegenhaltungen:
- WO-A2-2006/120010
- CA-A1- 2 175 287
- US-A- 4 576 960

## Beschreibung

Die vorliegende Erfindung betrifft Verbindungen der Formel I 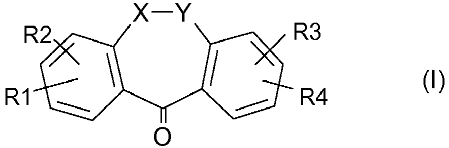 worin X, Y und R1 bis R4 die unten angegebenen Bedeutungen besitzen, sowie pharmazeutische Mittel, welche die Verbindungen der Formel I enthalten. Bei den Verbindungen handelt es sich um Interleukin-1β (IL-1β)- und Tumor-Nekrose-Faktor-α (TNF-α)-Inhibitoren, die zur Behandlung von inflammatorischen Erkrankungen brauchbar sind.

IL-1β und TNF-α schützen den Körper vor infektiösen Agenzien, Tumoren oder Gewebeschädigung. Bei Autoimmunerkrankungen kommt es jedoch zu einer erhöhten Produktion von IL-1β und TNF-α, was beispielsweise Knochen- und Knorpelabbau zur Folge haben kann. Arzneimittel, welche die Freisetzung von IL-1β und TNF-α regulieren, sind daher zur Behandlung von inflammatorischen Erkrankungen brauchbar.

Eine Gruppe von Verbindungen, welche die Freisetzung von IL-1β und TNF-α inhibieren, sind aus der WO 98/32730 bekannt. Sie entsprechen der allgemeinen Formel 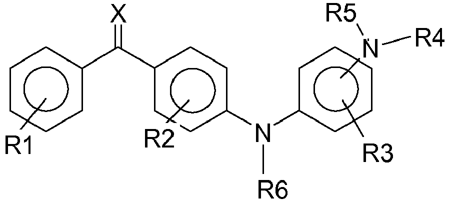 und sind zur Behandlung und Prophylaxe von Asthma, Allergien, rheumatoider Arthritis, Spondyloarthritis, Gicht, Atherosklerose, chronische entzündliche Darmerkrankung (inflammatory bowel disease), proliferative und inflammatorische Hauterkrankungen, wie Psoriasis und atopische Dermatitis brauchbar.

Weitere Verbindungen dieser Gruppe sind beschrieben in WO 01/05744, WO 01/05745, WO 01/05746, WO 01/05749, WO 01/05751, WO 01/42189, WO 02/45752, WO 02/076447 und WO 03/018535 sowie in J. Med. Chem. 2003, 46, 5651-5662 und Bioorganic & Medicinal Chemistry Letters 14 (2004) 3601-3605.

Die WO 2006/120010 beschreibt Dibenzocycloheptanonderivate der Formel I 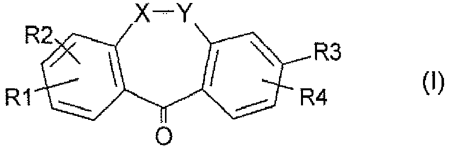 worin eines der Ringatome X und Y für CH₂ und das andere für O, S, SO, SO₂ oder NR5 steht; oder -X-Y- für -CH₂-CH₂- oder -CH=CH- steht; R1 für H oder C₁-C₆-Alkyl steht; und R2 für H, Halogen oder C₁-C₄-Alkyl-C≡C-, das ggf. mit einer Aminogruppe substituiert ist, steht. R3 steht für Halogen oder einen gegebenenfalls substituierten Aminorest. Die Verbindungen sind Interleukin-1β (IL-1β)- und Tumor-Nekrose-Faktor-α (TNF-α)-Inhibitoren, die zur Behandlung von inflammatorischen Erkrankungen brauchbar sind.

Die Wirkung der Verbindungen ist jedoch nicht zufriedenstellend.

Der vorliegenden Erfindung liegt daher die Aufgabe zugrunde, anti-inflammatorisch wirkende Verbindungen zur Verfügung zu stellen, die verbesserte Wirkung besitzen.

Diese Aufgabe wird durch die Verbindungen der Formel I gelöst. Die Erfindung betrifft somit Verbindungen der Formel I 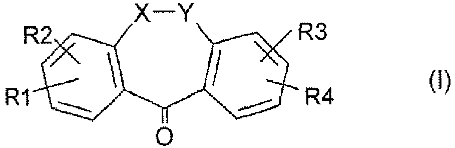 worin
eines der Ringatome X und Y für CH₂ und das andere für O, S, SO, SO₂ oder NR5 steht; oder -X-Y- für -CH₂-CH₂- oder -CH=CH- steht;
R1 für RO- steht, wobei R ausgewählt ist unter:
a) C₁-C₆-Alkyl, das mit 1, 2 oder 3 Hydroxy- oder C₁-C₆-Alkoxygruppen substituiert ist;
b) C₁-C₆-Alkyl, das substituiert ist mit einem gesättigten oder ungesättigten, aromatischen oder nicht-aromatischen Heterocyclus mit 5 oder 6 Ringatomen, der 1, 2 oder 3 Heteroatome aufweist, die unabhängig voneinander ausgewählt sind unter O, N und S, wobei der Heterocyclus gegebenenfalls 1 oder 2 Hydroxy-, C₁-C₆-Alkoxy- oder C₁-C₆-Alkylsubstituenten aufweisen und mit einem Phenylring oder einem gesättigten oder ungesättigtenCarbocyclus mit 5 oder 6 Ringatomen kondensiert sein kann;
c) C₁-C₆-Alkyl; und
d) C₁-C₆-Alkyl, das mit NR6R7 substituiert ist;
R2 für H oder C₁-C₆-Alkyl steht;
R3 ausgewählt ist unter:
a) 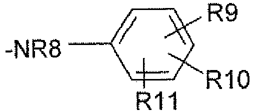
b) 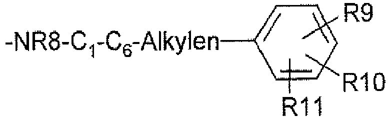
c) 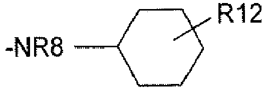
d) 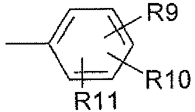
e) -NH-C₁-C₆-Alkylen-NR6R7
f) Tetrazolo;
R4 für H, Halogen oder C₁-C₆-Alkyl steht;
R5 für H oder C₁-C₆-Alkyl, das mit 1, 2 oder 3 Hydroxy- oder C₁-C₆-Alkoxygruppen substituiert ist, steht;
R6 und R7, die gleich oder verschieden sein können, für H oder C₁-C₆-Alkyl, das mit 1, 2 oder 3 Hydroxy- oder C₁-C₆-Alkoxygruppen substituiert ist, stehen;
R8 für H oder C₁-C₆-Alkyl steht;
R9, R10 und R11, die gleich oder verschieden sein können, ausgewählt sind unter:
a) H,
b NH₂,
c) mono-C₁-C₆-Alkylamino,
d) di-C₁-C₆-Alkylamino,
e) C₁-C₉-Alkyl,
f) C₁-C₆-Alkoxy,
g) Hydroxy,
h) Halogen,
i) C₁-C₆-Alkyl, das mit 1, 2 oder 3 Halogenatomen substituiert ist;
j) CONR6R7; und
k) NO₂;
R12 für H oder NH₂ steht;
R13 und R14, die gleich oder verschieden sein können, für H oder C₁-C₆-Alkyl stehen oder zusammen mit dem Stickstoffatom an das sie gebunden sind, einen nicht-aromatischen Heterocyclus mit 5 oder 6 Ringatomen, der 1 oder 2 Heteroatome aufweist, die unabhängig voneinander ausgewählt sind unter O und N, bilden;
und die optischen Isomeren, physiologisch verträglichen Salze und Solvate davon.

Der Ausdruck "Alkyl" (auch in Verbindung mit anderen Gruppen, wie Alkoxy, Halogenalkyl etc.) umfasst geradkettige und verzweigte Alkylgruppen mit vorzugsweise 1 bis 6 bzw. 1 bis 4 Kohlenstoffatomen, wie Methyl, Ethyl, n- und i-Propyl, n-, i- und t-Butyl, sec-Butyl, n-Pentyl und n-Hexyl.

Der Ausdruck "Halogen" steht für ein Fluor-, Chlor-, Brom- oder Jodatom, insbesondere für ein Fluor- oder Chloratom.

Bei C₁-C₆-Alkoxy, das mit 1, 2 oder 3 Hydroxy- oder C₁-C₆-Alkoxygruppen substituiert ist, handelt es sich bevorzugt um C₂-C₆-Alkoxy, insbesondere um 2-Hydroxyethoxy, 3-Hydroxypropoxy, 2-Hydroxypropoxy, 1,2-Dihydraxyethoxy, 2,3-Dihydroxypropoxy oder 2,3-Dimethoxypropoxy.

Bei einem gesättigten nicht-aromatischen Heterocyclus handelt es sich insbesondere um Pyrrolidinyl, Piperidinyl, Hydroxypiperidinyl, Piperazinyl, Tetrahydropyranyl, Tetrahydrofuranyl, Dioxolanyl, 2,2-Dimethyldioxoianyl, Dioxanyl, Morpholinyl oder Thiomorpholinyl, Der Piperidinylrest kann durch 1, 2, 3 oder 4 C₁-C₄-Alkylgruppen, insbesondere Methylgruppen substituiert sein. Ein bevorzugter Piperidinylrest ist 2,2,6,6-Tetramethylpiperidinyl. Die Stickstoffheterocyclen können über ein Stickstoffatom oder ein Kohlenstoffatom gebunden sein.

Bei einem ungesättigten nicht-aromatischen Heterocyclus handelt es sich insbesondere um Pyrrolinyl, Di- oder Tetrahydropyridinyl.

Bei einem aromatischen Heterocyclus handelt es sich insbesondere um Pyridyl, bevorzugt 3-oder 4-Pyridyl, Pyrimidinyl, Pyrrolyl, Imidazolyl, Pyrazolyl, Oxazolyl, Isoxazolyl, Furyl, Thienyl, Thiazolyl, Thiadiazolyl, Isothiazolyl oder die entsprechenden Benzoderivate davon.

Gemäß einer Ausführungsform steht R1 in Position 7 (Position 1, bezogen auf X). Gemäß einer weiteren Ausführungsform steht R1 in Position 8 (Position 2, bezogen auf X). Gemäß einer weiteren Ausführungsform steht R1 in Position 9 (Position 3, bezogen auf X). Positionen 7 und 8 und insbesondere Position 7 sind bevorzugt.

R2 steht vorzugsweise für H.

Der Rest R3 steht vorzugsweise in Position 3 (bezogen auf Y). Gemäß einer Ausführungsform steht R3 für die oben genannten Bedeutungen b) und g).

Der Rest R3 steht vorzugsweise in Position 3 (bezogen auf Y). Gemäß einer Ausführungsform steht R3 für die oben genannten Bedeutungen b) und e).

R3 steht gemäß einer weiteren Ausführungsform für die oben genannte Formel (b). Gemäß einer weiteren Ausführungsform steht R3 für 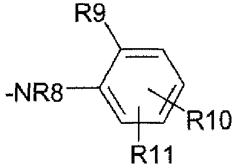 wobei R8, R9, R10 und R11 die oben angegebenen Bedeutungen besitzen. R10 und R11 stehen vorzugsweise in 3- und 5-Position.

Gemäß einer weiteren Ausführungsform steht R3 für 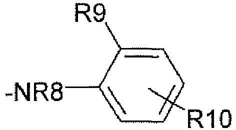 worin R8, R9 und R10 die oben angegebenen Bedeutungen besitzen. R10 steht vorzugsweise in 4-Position.

R4, R5, R6, R7 und R8 stehen vorzugsweise für H.

R9, R10 und R11 stehen vorzugsweise für die oben genannten Bedeutungen a), b), e), f),g) und h) und insbesondere für H, NH₂, C₁-C₆-Alkoxy oder Halogen.

R12 steht vorzugsweise für H.

Eine Ausführungsform der Erfindung sind die Verbindungen der Formel Ia: 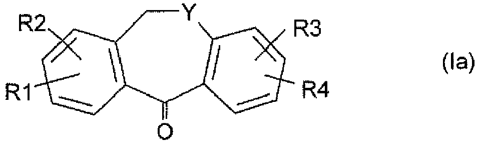 worin Y für O oder S steht und R1, R2, R3 und R4 die oben angegebenen Bedeutungen besitzen.

Eine weitere Ausführungsform sind die Verbindungen der Formel Iaa 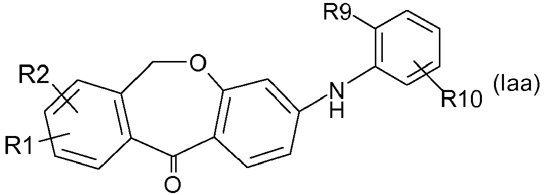 worin R1, R2, R9 und R10 die oben angegebenen Bedeutungen besitzen.

Gemäß einer Ausführungsform stehen in den Formeln Ia und laa R2 und R4 für H. Gemäß einer Ausführungsform stehen R1 in Position 1 oder 2 (relativ zur Methylengruppe des 7-Rings) und R3 in Position 2 (relativ zu Y). Gemäß einer weiteren Ausführungsform stehen R2 und R4 für H und R1 in Position 1 oder 2 (relativ zur Methylengruppe des 7-Rings) und R3 in Position 2 (relativ zu Y).

Eine weitere Ausführungsform sind die Verbindungen der Formeln lab und lac 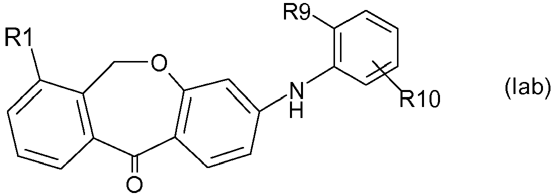 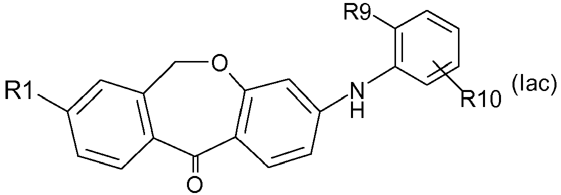 worin R1, R9 und R10 die oben angegebenen Bedeutungen besitzen.

Eine weitere Ausführungsform der Erfindung sind die Verbindungen der Formel Ib 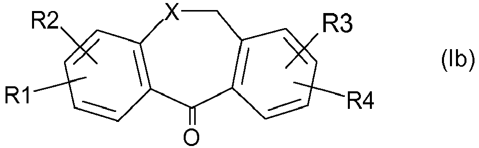 worin X für O oder S steht und R1, R2, R3 und R4 die oben angegebenen Bedeutungen besitzen.

Eine weitere Ausführungsform sind die Verbindungen der Formel Iba:

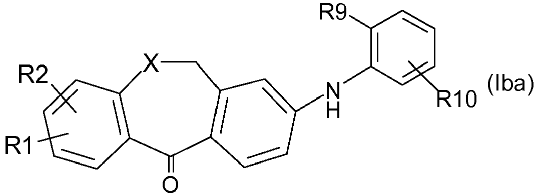

Gemäß einer Ausführungsform stehen in den Formeln Ib und Iba R2 und R4 für H. Gemäß einer Ausführungsform stehen R1 in Position 1 oder 2 (relativ zu X) und R3 in Position 2 (relativ zur Methylengruppe des 7-Rings). Gemäß einer weiteren Ausführungsform stehen R2 und R4 für H und R1 in Position 1 oder 2 (relativ zu X) und R3 in Position 2 (relativ zur Methylengruppe des 7-Rings).

Eine weitere Ausführungsform sind die Verbindungen der Formel Ibb 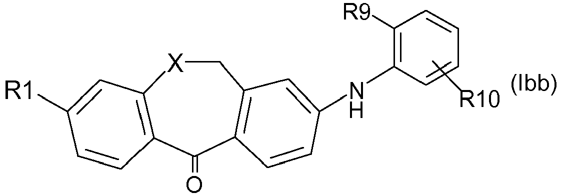 worin X für O oder S steht und R1, R9 und R10 die oben angegebenen Bedeutungen besitzen.

Eine weitere Ausführungsform sind die Verbindungen der Formel Ibc 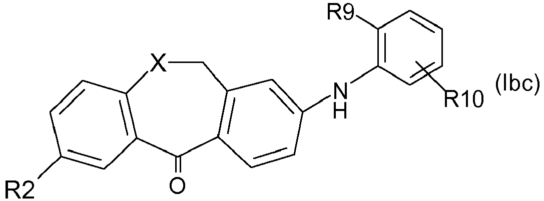 worin X für O oder S steht und R2, R9 und R10 die oben angegebenen Bedeutungen besitzen.

Eine weitere Ausführungsform sind die Verbindungen der Formel Ic 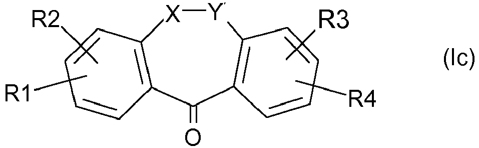 worin -X-Y- für -CH₂-CH₂- oder -CH=CH- steht und R1, R2, R3 und R4 die oben angegebenen Bedeutungen besitzen.

Eine weitere Ausführungsform sind die Verbindungen der Formel Ica: 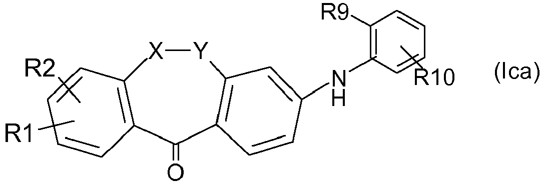 worin -X-Y- für -CH₂-CH₂- oder -CH=CH- steht und R1, R2, R9 und R10 die oben angegebenen Bedeutungen besitzen.

Gemäß einer Ausführungsform stehen in den Formeln Ic und Ica R2 und R4 für H. Gemäß einer Ausführungsform stehen R1 in Position 1 oder 2 (relativ zu X) und R3 in Position 2 (relativ zu Y). Gemäß einer weiteren Ausführungsform stehen R2 und R4 für H und R1 in Position 1 oder 2 (relativ zu X) und R3 in Position 2 (relativ zu Y).

Eine weitere Ausführungsform sind die Verbindungen der Formeln Icb und Icc 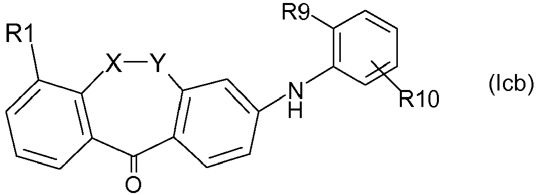 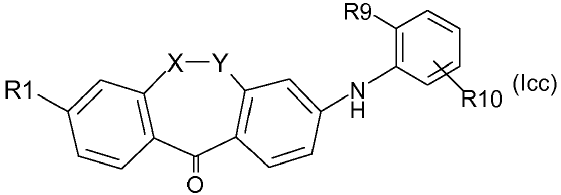 worin -X-Y- für -CH₂-CH₂- oder -CH=CH- steht und R1, R2, R9 und R10 die oben angegebenen Bedeutungen besitzen.

Die Erfindung umfasst auch die physiologisch verträglichen Salze der Verbindungen der Formel I. Im vorliegenden Fall handelt es sich insbesondere um die Säureadditionssalze. Zur Bildung der Säureadditionssalze werden anorganische Säuren, wie Salzsäure, Schwefelsäure oder Phosphorsäure oder organische Säuren, wie Weinsäure, Zitronensäure, Maleinsäure, Fumarsäure, Äpfelsäure, Mandelsäure, Ascorbinsäure, Gluconsäure, Methansulfonsäure, Benzolsulfonsäure oder Toluensulfonsäure und dergleichen, eingesetzt.

Soweit die erfindungsgemäßen Verbindungen Asymmetriezentren aufweisen, sind die Racemate sowie die einzelnen optischen Isomere (Enantiomere, Diastereomere) ebenfalls Gegenstand der Erfindung.

Gegenstand der Erfindung sind auch die Solvate der Verbindungen der Formel I oder der Salze davon, insbesondere die Hydrate.

Die Herstellung der Verbindungen der Formel I, die an der Position 7 substituiert sind, wobei X-Y für CH₂-CH₂ oder CH = CH steht, erfolgt nach den in den Schemata I bis III beispielhaft erläuterten Verfahren.

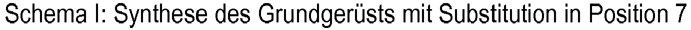

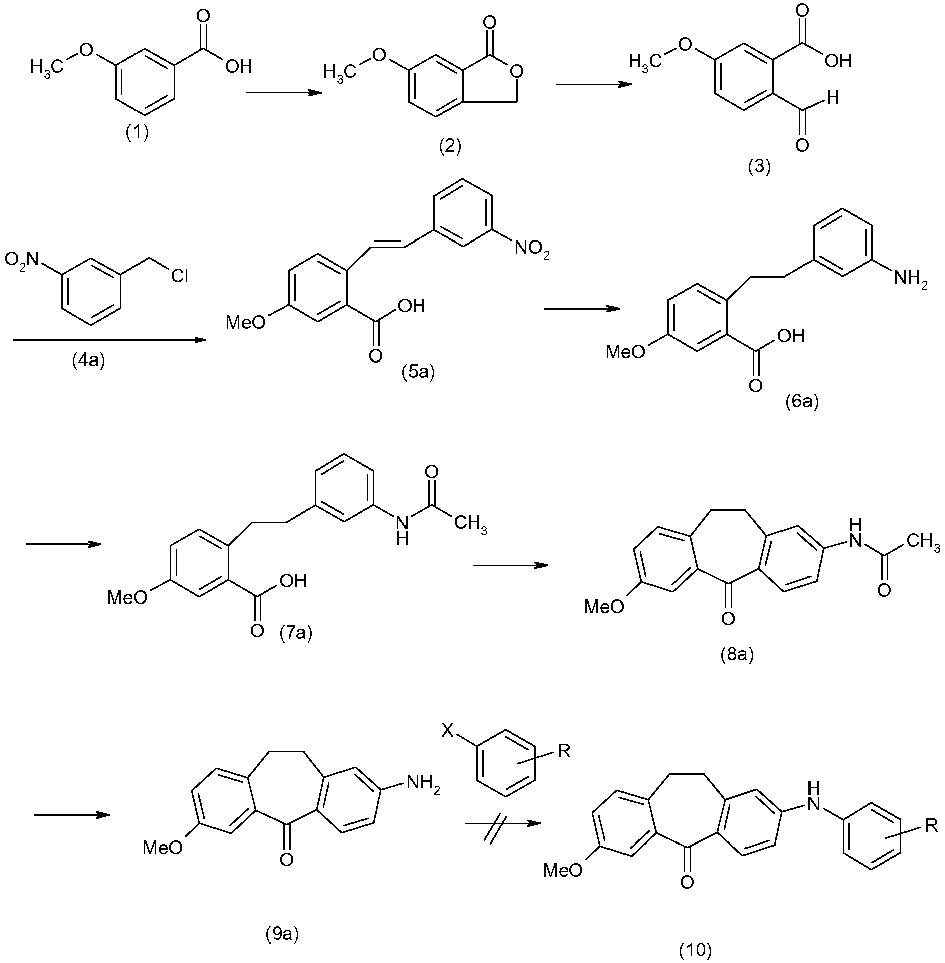

Ausgehend von 3-Methoxybenzoesäure (1) wird über das Phthalid (2) eine Formylgruppe eingeführt. Mittels einer Wittig-Reaktion erhält man die ungesättigte Verbindung (5a), die in üblicher Weise, beispielsweise unter Verwendung eines Edelmetallkatalysators, wie Pt oder Pd, hydriert werden kann. Vor dem Ringschluss durch Friedel-Crafts-Acylierung wird die Aminogruppe durch eine geeignete Schutzgruppe geschützt, die nach der Friedel-Crafts-Acylierung entfernt wird. Nach Umsetzung mit einem Arylhalogenid erhält man Verbindung (10).

Schema II zeigt ein alternatives Verfahren zu Herstellung von (10), mit dem sich die Verwendung der Schutzgruppe vermeiden lässt:

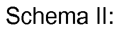

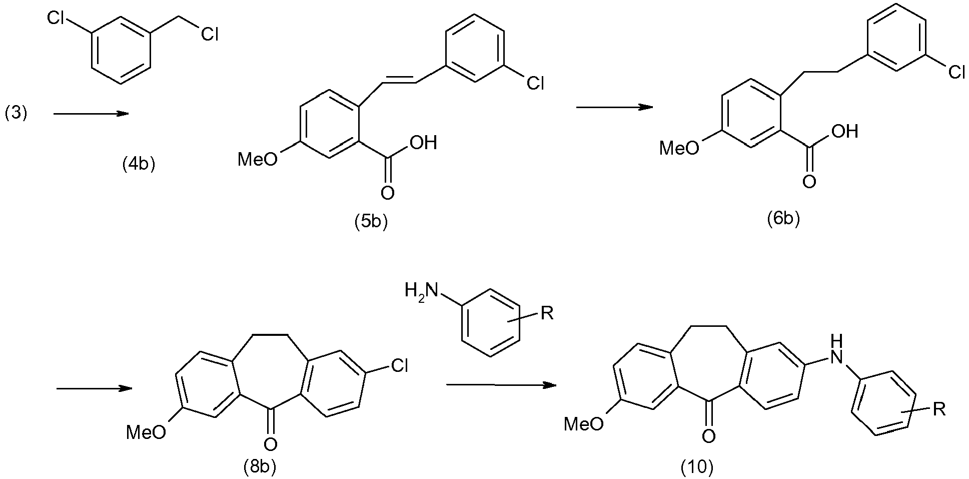

Die an der Position 7 methoxy-substituierten Verbindungen (10) lassen sich durch Etherspaltung, etwa mittels wässriger HBr, in die Hydroxyverbindungen (11) überführen, siehe Schema III.

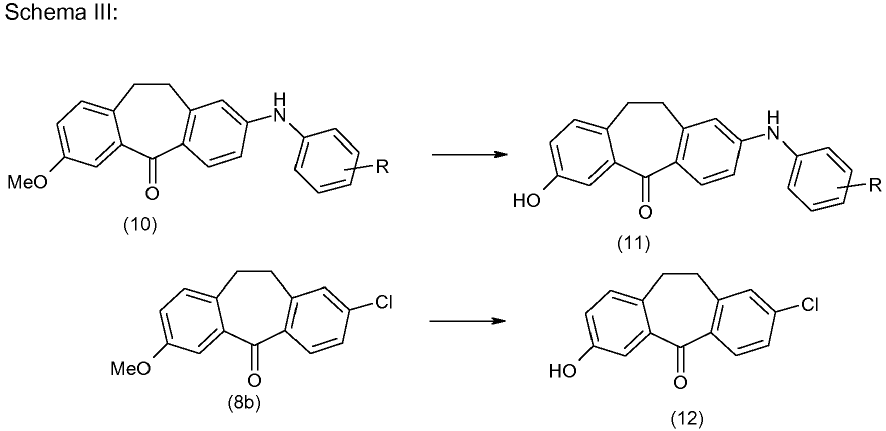

Die Hydroxygruppe an Position 7 erlaubt weitere Substitutionen, etwa über die Ethersynthese nach Williams oder die Mitsunobu-Reaktion, siehe Schema IV:

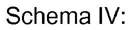

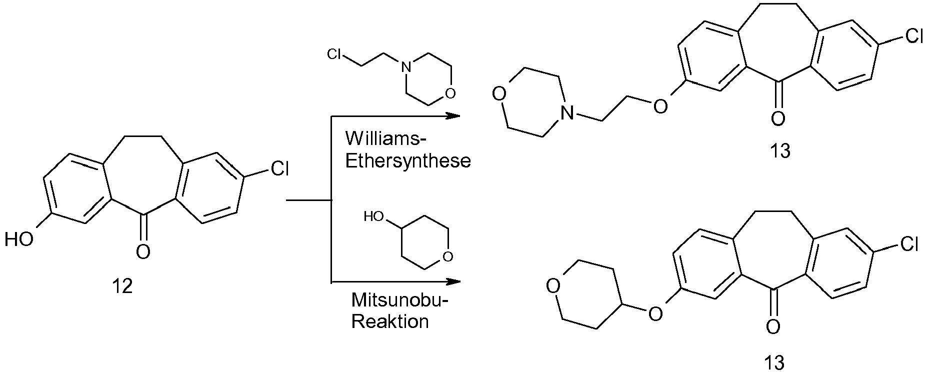

Die Herstellung der Verbindungen der Formel I, die an der Position 8 oder 9 substituiert sind, wobei X-Y für CH₂-CH₂ oder CH = CH steht, erfolgt nach den in den Schemata V bis X beispielhaft erläuterten Verfahren.

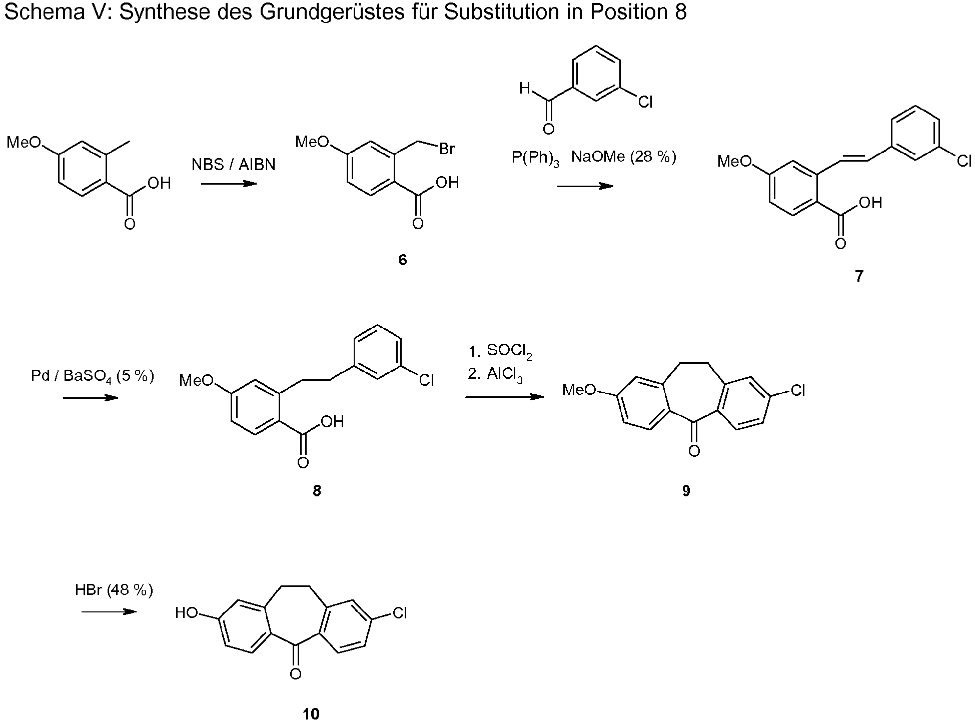

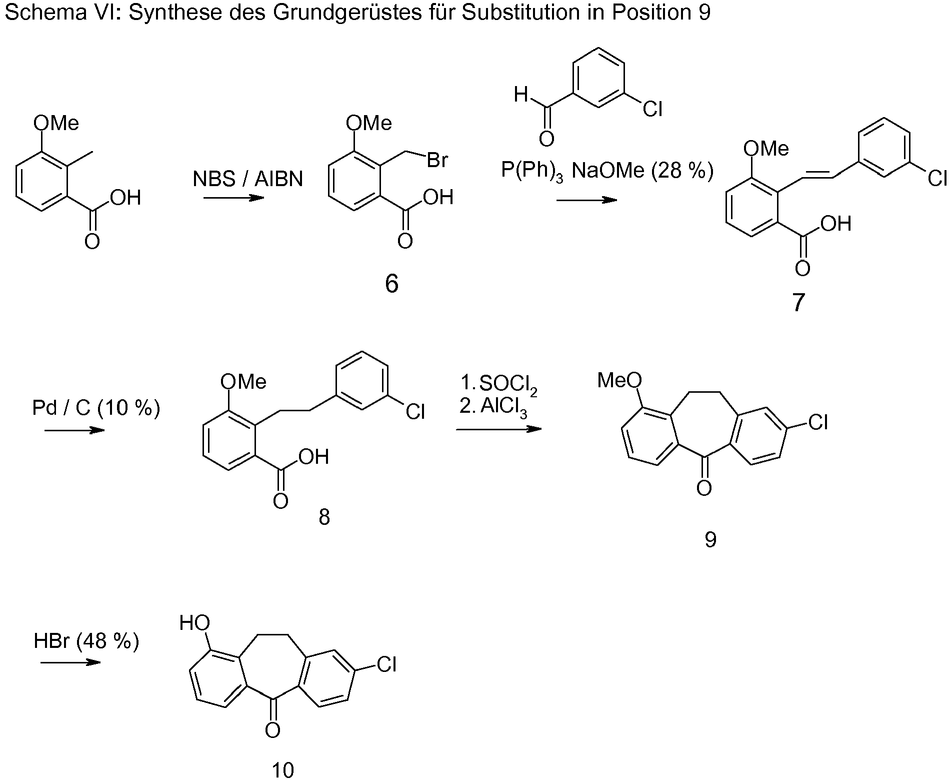

Ausgehend von Methoxymethylbenzoesäure erfolgt zunächst eine Bromierung mit N-Bromsuccinimid (NBS). Die entstandene Brommethylmethoxybenzoesäure (6) wird in einer Wittig-Reaktion mit 3-Chlorbenzaldehyd zu Chlorphenylvinylmethoxybenzoesäure (7) umgesetzt. Die nachfolgende Hydrierung der Chlorphenylvinylmethoxy-benzoesäure erfolgt in üblicher Weise unter Verwendung von Edelmetalkatalysatoren, wie Pd/BaSO₄ (Substitution in Position 8) bzw. Pd/C (Substitution in Position 9). Die Carbonsäure (8) wird in das Säurechlorid überführt, zum Beispiel durch Umsetzung mit Thionylchlorid. Der anschließende Ringschluss zu (9) erfolgt durch Friedel-Crafts-Acylierung mit geeigneten Lewis-Säuren, wie AlCl₃. Verbindung (10) wird dann durch Etherspaltung, beispielsweise mit wässrigem Bromwasserstoff, erhalten.
Schema VII: Substitution mit Alkoholen

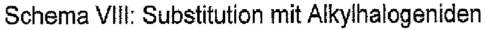

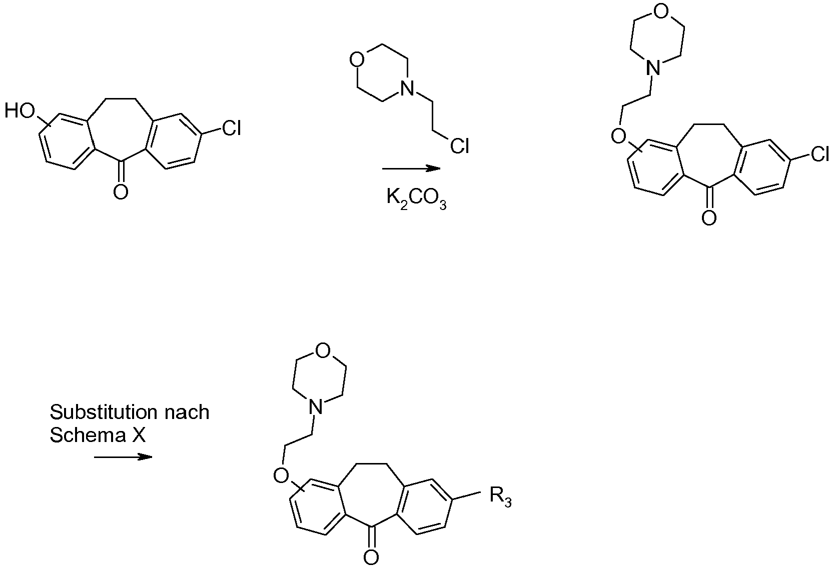

Auf diese Weise wurden folgende Verbindungen erhalten:

| Verbindungen in Position 9 | | | Verbindungen in Position 8 | | |
|---|---|---|---|---|---|
| 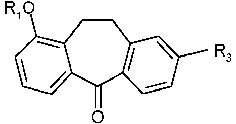 | | | 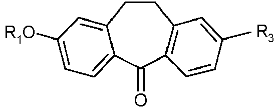 | | |

| Verbindung des Beispiels Nr. | R₁ | R₃ | Verbindung des Beispiels Nr. | R₁ | R₃ |
|---|---|---|---|---|---|
| 73 (11j) | 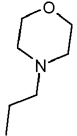 | 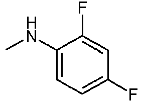 | 56 (12e) | 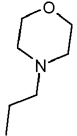 | 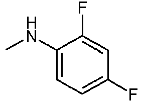 |
| 89 (16) | 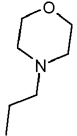 | -Cl | 90 (17) | 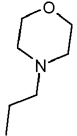 | -Cl |

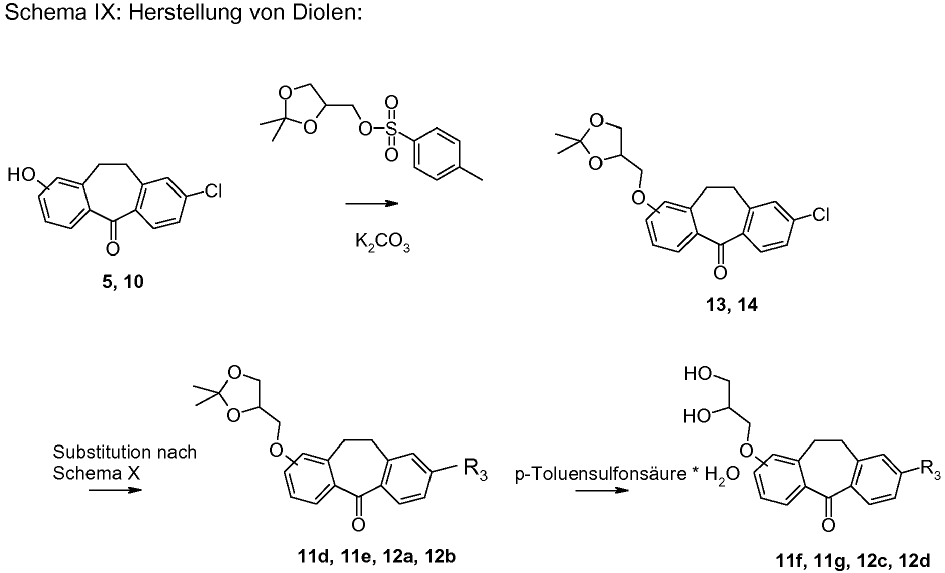

Auf diese Weise wurden folgende Verbindungen erhalten:

| Verbindungen in Position 9 | | | Verbind ungen in Position 8 | | |
|---|---|---|---|---|---|
| 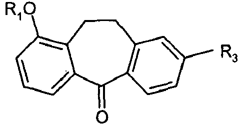 | | | 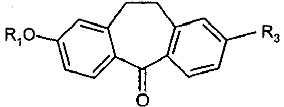 | | |

| Verbindung des Beispiels Nr. | R₁ | R₃ | Verbindung des Beispiels Nr. | R₁ | R₃ |
|---|---|---|---|---|---|
| 66 (11d) | 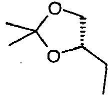 | 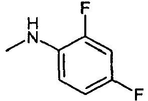 | 51 (12a) | 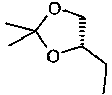 | 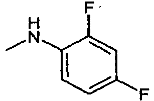 |
| 68 (11e) | 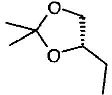 | 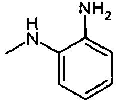 | 53 (12b) | 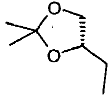 | 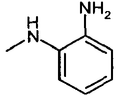 |
| 67 (11f) | 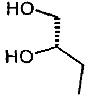 | 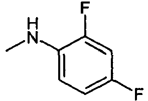 | 52 (12c) | 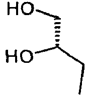 | 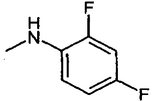 |
| 69 (11g) | 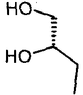 | 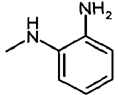 | 54 (12d) | 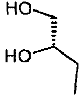 | 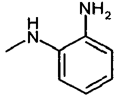 |
| 65 (13) | 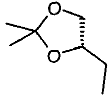 | -Cl | 87 (14) | 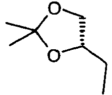 | -Cl |

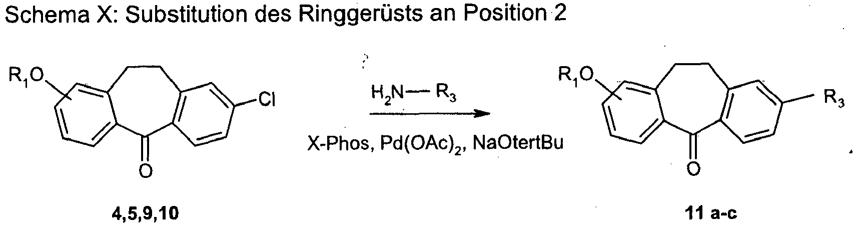

Die Mischung aus Chlordihydrodibenzosuberenon, Pd(OAc)₂, Phosphinligand (2-(Dicyclohexylphosphino)-2',4',6'-triisopropylbiphenyl), NaOtert-Bu, Toluol, tert-BuOH und dem Amin R₃NH₂ wird bei erhöhten Temperaturen mehrere Stunden gerührt. Nach dem Abkühlen wird das gewünschte Produkt in üblicher Weise gewonnen.

Auf diese Weise wurden folgende Verbindungen erhalten:

| | | |
|---|---|---|
| 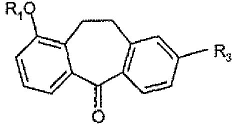 | | |

| Verbindung des Beispiels Nr. | R₁ | R₃ |
|---|---|---|
| 63 (11a) | -CH₃ | 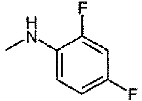 |
| 64 (11b) | -CH₃ | 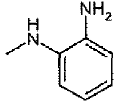 |
| 62 (11c) | -OH | 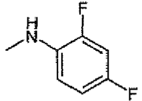 |

Die Herstellung der Verbindungen der Formel I, die an der Position 7 substituiert sind, wobei eines der Ringatome X oder Y für CH₂ steht, erfolgt nach dem in Schema XI erläuterten Verfahren.

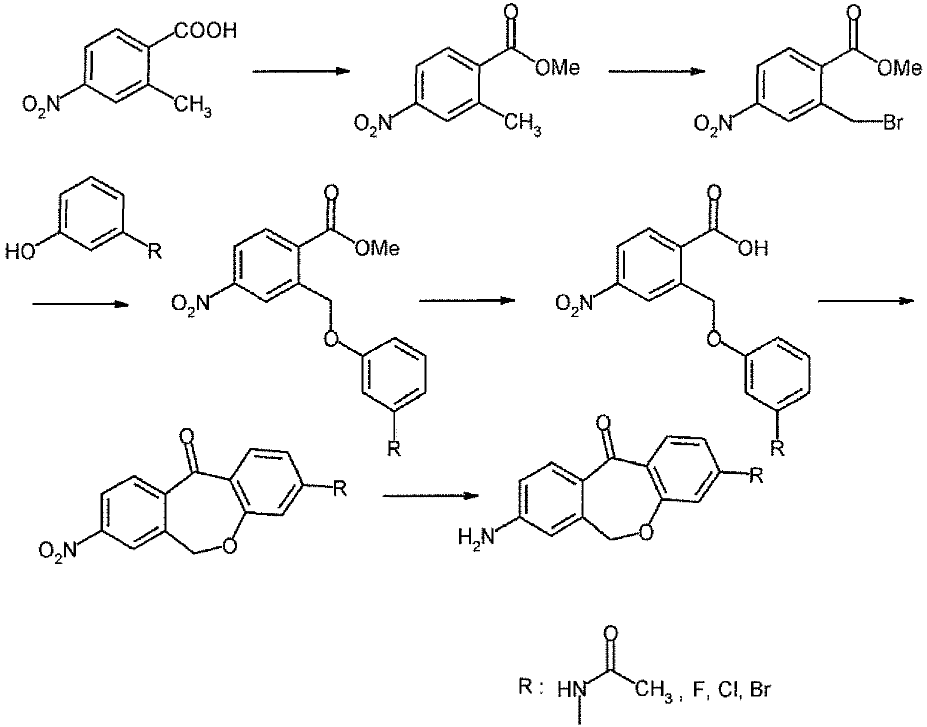

Nach Veresterung der Ausgangsverbindung 2-Methyl-4-nitrobenzoesäure wird in üblicher Weise mit N-Bromsuccinimid (NBS) bromiert. Das bromierte Edukt wird in Gegenwart von Kaliumcarbonat mit dem Phenol umgesetzt. Nach Verseifung des Esters, bspw. mit KOH, erfolgt Ringschluss, beispielsweise mit Polyphosphorsäure in einem organischen Lösungsmitten, wie Sulfolan. Die Nitrogruppe wird anschließend in üblicher Weise, beispielsweise mit Sn/HCl, zur Aminogruppe reduziert. Es empfiehlt sich, bei erhöhten Temperaturen zu arbeiten, zweckmäßigerweise bei der Siedetemperatur des Lösungsmittels bzw. am Rückfluss.

Die Herstellung der Verbindungen der Formel I, worin eines der Ringatome X oder Y für SO oder SO₂ steht, erfolgt durch Oxidation der Verbindungen, worin Y für S steht, in üblicher Weise, beispielsweise mit Perverbindungen, wie m-Chlorperbenzoesäure.

Die erfindungsgemäßen Verbindungen zeigen in vitro und in vivo immunmodulierende und die Freisetzung von TNF-α und IL-1β hemmende Wirkung. Die erfindungsgemäßen Verbindungen eignen sich daher zur Behandlung von Erkrankungen, die im Zusammenhang mit einer Störung des Immunsystems stehen. Sie eignen sich beispielsweise zur Behandlung von Autoimmunerkrankungen, Krebs, rheumatischer Arthritis, Gicht, septischem Schock, Osteoporose, neuropathischem Schmerz, HIV-Ausbreitung, HIV-Demenz, viraler Myokarditis, insulinabhängiger Diabetes, Periodontalerkrankungen, Restenose, Alopezie, T-Zell-Depletion bei HIV-Infektionen oder AIDS, Psoriasis, akuter Pankreatitis, Abstoßungsreaktionen bei allogenen Transplantaten, allergisch bedingter Lungenentzündung, Arteriosklerose, Multipler Sklerose, Kachexie, Alzheimer Erkrankung, Schlaganfall, Ikterus, Colitis ulcerosa, Morbus Crohn, Inflammatory-Bowel-Disease (IBD), Ischämie, kongestive Herzinsuffizienz, Lungen-Fibrose, Hepatitis, Glioblastom, Guillain-Barré-Syndrom, systemischer Lupus erythematodes, Adult-respiratory-distress-Syndrom (ARDS) und Atemnotsyndrom.

Die erfindungsgemäßen Verbindungen können entweder als einzelne therapeutische Wirkstoffe oder als Mischungen mit anderen therapeutischen Wirkstoffen verabreicht werden.

Die Verbindungen können alleine verabreicht werden, im Allgemeinen werden sie jedoch in Form pharmazeutischer Mittel dosiert und verabreicht, d.h. als Mischungen der Wirkstoffe mit geeigneten pharmazeutischen Trägern oder Verdünnungsmittel. Die Verbindungen oder Mittel können oral oder parenteral verabreicht werden, vorzugsweise werden sie in oralen Dosierungsformen gegeben.

Die Art des pharmazeutischen Mittels oder Trägers bzw. des Verdünnungsmittels hängt von der gewünschten Verabreichungsform ab. Orale Mittel können bspw. als Tabletten oder Kapseln vorliegen und können übliche Exzipienten enthalten wie Bindemittel (z.B. Sirup, Akazia, Gelatine, Sorbit, Tragant oder Polyvinylpyrrolidon), Füllstoffe (z.B. Lactose, Zucker, Maisstärke, Calciumphosphat, Sorbit oder Glycin), Gleitmittel (z.B. Magnesiumstearat, Talcum, Polyethylenglycol oder Siliciumdioxid), desintegrierende Mittel (z. B. Stärke) oder Netzmittel (z. B. Natriumlaurylsulfat). Flüssige Oralpräparate können in Form wässriger oder öliger Suspensionen, Lösungen, Emulsionen, Sirupe, Elixiere oder Sprays und dergleichen sein. Sie können auch als Trockenpulver vorliegen, das zur Rekonstitution mit Wasser oder einem anderen geeigneten Träger aufbereitet wird. Derartige flüssige Präparate können übliche Additive, beispielsweise Suspendiermittel, Geschmacksstoffe, Verdünnungsmittel oder Emulgatoren enthalten. Für die parenterale Verabreichung kann man Lösungen oder Suspensionen mit üblichen pharmazeutischen Trägern einsetzen.

Die erfindungsgemäßen Verbindungen oder Mittel können an Säuger (Mensch oder Tier) in einer Dosis von etwa 0,5 mg bis 100 mg pro kg Körpergewicht pro Tag verabreicht werden. Sie können in einer Einzeldosis oder in mehreren Dosen gegeben werden.

Das Wirkungsspektrum der Verbindungen als Inhibitoren der TNF-α und IL-1β Freisetzung wurde anhand der Testsysteme wie beschrieben von Donat C. und Laufer S. in Arch. Pharm. Pharm. Med. Chem. 333, Suppl. 1, 1-40, 2000, untersucht. Die erhaltenen IC₅₀-Werte wurden in Relation zu den Werten für die Verbindung SB-203580 der Formel 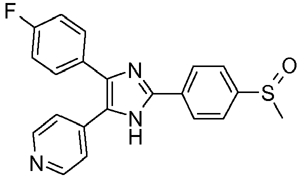 gesetzt. Bei den in der nachfolgenden Tabelle angegebenen Zahlenwerten handelt es sich also um die relative Aktivität, errechnet aus dem Verhältnis von IC₅₀ (SB-203580)/ IC₅₀ (Testverbindung). Je höher der Zahlenwert, umso wirksamer die Verbindung. Die Verbindungen, bei denen R1 für H steht, repräsentieren den Stand der Technik gemäß WO2006/120010.

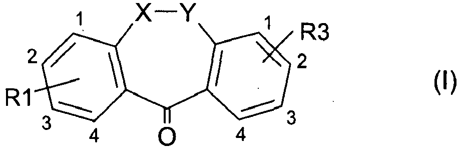

**Tabelle: Inhibierung von p38 MAP Kinase und TNF-α.**

| R1 | R3 | X | Y | p38 MAP-Kinase | Inhibierung von TNF- α |
|---|---|---|---|---|---|
| H¹⁾ | 2-[NH-(2-NH₂-phenyl)] | CH₂ | O | 0.23 | |
| 2-NH₂¹⁾ | 2-[NH-(2-NH₂-phenyl)] | CH₂ | O | 0.93 | |
| 2-OCH₃ | 2-[NH-(2-NH₂-phenyl)] | CH₂ | O | 0.57 | |
| 2-(OCH₂CH₂-morpholin) | 2-[NH-(2-NH₂-phenyl)] | CH₂ | O | 0.32 | |
| H¹⁾ | 2-[NH-(2,4-di-F-phenyl)] | CH₂ | O | 0.31 | 0.09 |
| 2-(NHCO-morpholin)¹⁾ | 2-[NH-(2,4-di-F-phenyl)] | CH₂ | O | 0.51 | |
| 2-(OCH₂CH₂-morpholin) | 2-[NH-(2,4-di-F-phenyl)] | CH₂ | O | 1.92 | 0,02 |
| 1-OCH₃ | 2-[NH-(2,4-di-F-phenyl)] | CH₂ | O | 1.11 | 0,04 |
| 2-(OCH₂CH₂-N(CH₃) | 2-[NH-(2,4-di-F-phenyl)] | CH₂ | O | 1.02 | |
| H¹⁾ | 2-[NH-(2-NH₂-phenyl)] | CH₂ | CH₂ | 0.54 | 0,51 |
| 3-OCH₃ | 2-[NH-(2-NH₂-phenyl)] | CH₂ | CH₂ | 1.00 | 0,91 |
| (S)-3-[OCH₂-CH(OH)-CH₂OH] | 2-[NH-(2-NH₂-phenyl)] | CH₂ | CH₂ | 1.54 | >5 |
| (S)-3-(OCH₂-3,3-di-CH₃-2,4-dioxolan) | 2-[NH-(2-NH₂-phenyl)] | CH₂ | CH₂ | 1.31 | >5 |
| 3-(OCH₂-3,3-di-CH₃-2,4-dioxolan) | 2-[NH-(2-NH₂-phenyl)] | CH₂ | CH₂ | 0.72 | >5 |
| H ¹⁾ | 2-[NH-(2,4-di-F-phenyl)] | CH₂ | CH₂ | 0.52 | 0,49 |
| 1-OH ¹⁾ | 2-[NH-(2,4-di-F-phenyl)] | CH₂ | CH₂ | 2.26 | 0,06 |
| 1-[OCH₂-CH(OH)-CH₂OH] | 2-[NH-(2,4-di-F-phenyl)] | CH₂ | CH₂ | 1.76 | |
| 1-(OCH₂CH₂-morpholin) | 2-[NH-(2,4-di-F-phenyl)] | CH₂ | CH₂ | 1.26 | |
| 2-OCH₃ | 2-[NH-(2,4-di-F-phenyl)] | CH₂ | CH₂ | 2.60 | |
| 2-(OCH₂CH₂-morpholin) | 2-[NH-(2,4-di-F-phenyl)] | CH₂ | CH₂ | 2.53 | |
| 2-(OCH₂-CH₂OH) | 2-[NH-(2,4-di-F-phenyl)] | CH₂ | CH₂ | 2.60 | |
| 3-OH¹⁾ | 2-[NH-(2,4-di-F-phenyl)] | CH₂ | CH₂ | 2.03 | 1.80 |
| 3-OCH₃ | 2-[NH-(2,4-di-F-phenyl)] | CH₂ | CH₂ | 1.59 | 0.70 |
| (S)-3-(OCH₂-3,3-di-CH₃-2,4-dioxolan) | 2-[NH-(2,4-di-F-phenyl)] | CH₂ | CH₂ | 1.86 | 3.79 |
| 3-(O-tetrahydropyran-4-yl)¹⁾ | 2-[NH-(2,4-di-F-phenyl)] | CH₂ | CH₂ | 0.74 | 1.98 |
| (S)-3-(OCH₂-3,3-di-CH₃-2,4-dioxolan) | 2-[NH-(2,4-di-F-phenyl)] | CH₂ | CH₂ | 1.04 | >5 |
| 3-(OCH₂-CH₂OH) | 2-[NH-(2,4-di-F-phenyl)] | CH₂ | CH₂ | 3.00 | 4.98 |
| 3-(OCH₂-CH₂CH₂OH) | 2-[NH-(2,4-di-F-phenyl)] | CH₂ | CH₂ | 4.85 | >5 |
| 3-(OCH₂-3,3-di-CH₃-2,4-dioxolan) | 2-[NH-(2,4-di-F-phenyl)] | CH₂ | CH₂ | 0.32 | 3.75 |
| 3-(O-CH₂-tetrahydro-pyran-4-yl) | 2-[NH-(2,4-di-F-phenyl)] | CH₂ | CH₂ | 3.04 | 4.90 |
| H ¹⁾ | 2-[NH-(2-NH₂-4F-phenyl)] | CH₂ | CH₂ | 0.37 | 0,67 |
| 3-OCH₃ | 2-[NH-(2-NH₂-4F-phenyl)] | CH₂ | CH₂ | 0.92 | 0.73 |
| H ¹⁾ | 2-[NH-(2-NH₂-4F-phenyl)] | CH₂ | O | 0.52 | 0.08 |
| 2-NH₂ ¹⁾ | 2-[NH-(2-NH₂-4F-phenyl)] | CH₂ | O | 1.15 | 0.24 |
| H ¹⁾ | 2-[NH-(2-OCH₃-phenyl)] | CH₂ | O | 0.09 | 0,13 |
| 2-NH₂ ¹⁾ | 2-[NH-(2-OCH₃-phenyl)] | CH₂ | O | 0.14 | 0,04 |
| 3-OCH₃ | 2-[NH-(2-OCH₃-phenyl)] | CH₂ | CH₂ | 0.11 | 0,18 |

| | | | | | |
|---|---|---|---|---|---|
| ¹⁾ Referenzverbindung | | | | | |

Es ist ersichtlich, dass die erfindungsgemäßen Verbindungen ausgezeichnete Wirksamkeit besitzen und den Verbindungen der WO 2006/120010 überlegen sind.

Die nachfolgenden Beispiele erläutern die Erfindung ohne sie zu begrenzen.

### Geräte und Methoden

- Schmelzpunkte: Büchi Melting Point B-545 (thermodynamische Korrektur)
- NMR-Spektroskopie: Bruker Avance 200 (200 MHz)
- IR-Spektroskopie: Perkin-Elmer Spectrum One (ATR)
- GC-MS: Hewlett Packard HP 6890 Series GC-System Hewlett Packard HP 5973 Mass Selective Detector GC-Säule: HP-5MS 5% Phenylmethylsiloxan ESI-MS: 70eV

### GC-Methode

| Temperatur [°C] | Laufzeit [min] |
|---|---|
| 160 | 1 |
| 160 → 240 | 10 |
| 240 | 5 |
| 240 → 270 | 10 |
| 270 | 35 |

### Abkürzungen:

- MeOH: Methanol
- NaOMe: Natriummethylat
- KOtert-Bu: Kalium-tert.-butylat
- Tert-BuOH: tert.-Butanol
- EtOH: Ethanol
- DMF: Dimethylformamid
- THF: Tetrahydrofuran
- DCM: Dichlormethan
- i.Vak.: im Vakuum
- EA: Ethylacetat

### Allgemeine Methode A

Zur Synthese der Stilbene mittels Wittig-Reaktion wird die angegebene Menge Triphenylphosphin in MeOH gelöst. Unter Rühren wird das entsprechende Benzylchlorid zugetropft und 2 Stunden unter Rückfluss erhitzt. Die Reaktionsmischung wird auf 0°C abgekühlt und mit dem entsprechenden Aldehyd versetzt. Anschließend wird bei 0°C innerhalb ca. 45 min die angegebene Menge NaOMe-Lösung zugetropft. Nach beendigtem Zutropfen wird 3 h bei 0°C nachgerührt und das Reaktionsgemisch auf ein gerührtes Gemisch aus 75 g Eis und 175 ml Wasser gegossen. Es wird abgesaugt, der Filterrückstand wird mit ca. 50 ml Wasser gewaschen und die vereinigten wässrigen Phasen werden im Vakuum mehrmals mit Dichlormethan gewaschen. Das Produkt erhält man durch Ansäuern der wässrigen Phase in Form eines Niederschlags, der abfiltriert wird. Die Aufreinigung erfolgt durch Umkristallisieren aus MeOH/H₂O.

### Allgemeine Methode B

Zur Synthese der Ketone mittels Friedel-Crafts-Acylierung wird die angegebene Menge Säure in Dichlormethan suspendiert und der Ansatz mit Argon gespült. Nach Erhitzen zum Rückfluss wird unter Rühren innerhalb 1 Stunde eine Lösung aus Thionylchlorid in 50 ml Dichlormethan zugetropft und eine weitere Stunde gerührt. Nach dem Abkühlen auf Raumtemperatur wird innerhalb von 45 min eine Suspension von AlCl₃ in Dichlormethan zugetropft, 30 min bei Raumtemperatur nachgerührt und das Reaktionsgemisch unter Rühren auf ein Gemisch aus 30 g Eis und 50 ml Wasser gegossen. Das Hydrolysegemisch wird ca. 30 min gerührt, der Niederschlag abfiltriert und die beiden Phasen des Filtrats werden getrennt. Das Produkt erhält man durch Einengen der organischen Phase im Vakuum. Die Aufreinigung erfolgt durch Umkristallisieren mit MeOH.

### Allgemeine Methode C

Zur Herstellung der Testverbindungen wird eine Mischung aus Arylhalogenid, Amin, Pd(OAc)₂, Phosphinligand, KOtert-Bu, Toluol und tert-BuOH unter Argon auf 100 °C erhitzt und die angegebene Zeit bei dieser Temperatur gerührt. Danach wird auf Raumtemperatur abgekühlt, mit 150 ml H₂O hydrolysiert, mit je 3x 200 ml Diethylether extrahiert, die organische Phase filtriert und im Vakuum eingeengt. Die Aufreinigung erfolgt über Flash-Chromatographie.

### Allgemeine Methode D

Zu einer Lösung der Nitroverbindung in EtOH wird SnCl₂ x 5 H₂O hinzugefügt. Die Mischung wird 5 h refluxiert. Dann wird abgekühlt und mit 20%iger NaOH alkalisiert. Es wird mit je 3x 200 ml Ethylacetat extrahiert und die organische Phase im Vakuum eingeengt.

### Allgemeine Methode E

Zur Herstellung von 2-Chlor-7-(S-1,2-isopropylidenglycer-3-yl)-10,11-dihydro-dibenzo[a,d]cyclohepten-5-on werden 0,57 mmol 2-Chlor-7-hydroxy-10,11-dihydro-dibenzo[a,d]cyclohepten-5-on in 10 ml wasserfreiem DMF gelöst, 0,82 mmol des entsprechenden α,β-isopropylideneglycerol-γ-tosylates und 1,65 mmol K₂CO₃ hinzugefügt. Das Reaktionsgemisch wird 24 h unter Argon-Atmosphäre bei 80°C gerührt. Die Reaktionsmischung wird auf Raumtemperatur abgekühlt in 50 ml Wasser gelöst, mit Diethylether extrahiert und die organische Phase wird im Vakuum eingeengt. Das Produkt fällt in Form eines hellgelben Öls an.

### Allgemeine Methode F

Zur Lösung von 2 mmol Diisopropylazodicarboxylat in 20 ml THF wird eine Lösung von 2 mmol P(Ph)₃ in 10 ml THF bei 0°C zugetropft und 1 h gerührt. Anschließend wird eine Lösung aus 2 mmol 2-Chlor-7-hydroxy-10,11-dihydro-dibenzo[a,d]cyclohepten-5-on und 2 ml des entsprechenden Alkohols in THF zugetropft und 2 h bei Raumtemperatur gerührt. Es wird mit Wasser hydrolysiert, mit Ethylacetat extrahiert und die organische Phase im Vakuum eingeengt. Die Aufreinigung erfolgt an SiO₂ (Hexan/Ethylacetat 7+3).

### Allgemeine Methode G

1,7 mmol 2-Chlor-7-hydroxy-10,11-dihydro-dibenzo[a,d]cyclohepten-5-on, 1,86 mol Alkylhalogenid und 6,75 mmol K₂CO₃ werden entweder in DMF oder Acetonitril gelöst, für die angegebene Zeit bei 80°C gerührt, auf Raumtemperatur abgekühlt und in Diethylether und Wasser gelöst. Die organische Phase wird mit Wasser und Lauge gewaschen, über MgSO₄ getrocknet und im Vakuum eingeengt.

### Beispiel 1

### 6-Methoxyphthalid (2)

Die Mischung aus 20 g (131 mmol) 3-Methoxybenzoesäure, 13 ml (160 mmol) 37%ige Formalin-Lsg., 16 ml (162 mmol) 37%ige HCl und 150 ml 150 ml 100%ige Essigsäure wird unter Rühren auf 90°C erhitzt. Nachdem eine klare Lösung entstanden ist, wird der Rührer abgeschaltet und der Ansatz 14 Stunden bei dieser Temperatur belassen. Die Essigsäure wird bei 80°C im Vakuum abgezogen, der Rückstand in 150 ml Toluol aufgenommen und auf 80 ml eingeengt. Die 80°C heiße Lösung wird mit je 40 ml 20%iger Na₂SO₄-Lösung gewaschen, bis der pH der wässrigen Lösung alkalisch ist, dann wird mit 40 ml H₂O gewaschen. Die organische Phase wird nach Zugabe von 6 ml Morpholin 2 h bei 80°C gerührt, dann mit je 50 ml 10%iger H₂SO₄, bis die wässrige Phase sauer ist, sowie mit je zwei mal 50 ml H₂O gewaschen. Zum Kristallisieren des Produkts wird auf 50 ml eingeengt, ggf. ein Impfkristall zugefügt und bis zum Abkühlen auf Raumtemperatur gerührt. Man erhält das Produkt durch Abfiltrieren in Form von weißen Kristallen.
Ausbeute:13,8 g (64%); Smp.: 107,6 °C
IR (ATR): 2945(C-H)ₐₗᵢₚₕ, 1745 (C=O), 1489, 1320, 1278, 1247, 1054, 1017, 990, 9056, 769. ¹H-NMR (DMSO-d6) δ in ppm: 3.84 (s, 3 H, -OCH₃), 5.33 (s, 2 H, C³-H), 7.28 (s, 1 H, C⁷-H), 7.31 (d, J = 8.3 Hz, C⁴-H), 7.56 (d, 1 H, J = 8.3 Hz, C⁵-H).

### Beispiel 2

### 2-Formyl-5-methoxy-benzoesäure (3)

10 g (61 mmol) 6-Methoxyphtalid, 11,4 g (64,0 mmol) NBS und 200 ml Chlorbenzol werden unter Rühren gemischt und die Suspension auf 85°C erhitzt und mit 2 ml der Lösung von 100 mg AIBN in 10 ml Chlorbenzol versetzt. Innerhalb weniger Minuten steigt die Temperatur auf 110°C und es entsteht eine rote Lösung. Nach dem Abflauen des Temperaturanstiegs werden die restlichen 8 ml zugegeben und 40 min bei 85 °C gerührt. Nach dem Abkühlen auf 0°C wird vom ausgefallenen Succinimid abfiltriert und mit Chlorbenzen nachgewaschen. Das Filtrat wird eingeengt, bis ein öliger Rückstand entsteht, der mit 10%iger NaOH-Lösung aufgenommen und mit je drei mal 300 ml Dichlormethan gewaschen wird. Nach dem Ansäuern der wässrigen Phase mit konzentrierter HCl wird 1 Stunde bei 0°C gerührt. Dabei fällt die Säure in Form eines weißen Niederschlags aus, von dem abfiltriert wird.
Ausbeute: 9,6 g (87%); Schmelzpunkt: 166,2 °C
IR (ATR): 2903 (COOH), 2601 (C-H)ₐₗᵢₚₕ, 1703, 1586 (C=O), 1497, 1278, 1210, 1187, 1142, 1072, 1025, 897, 823, 736.
H₁-NMR (DMSO-d6) δ in ppm: 3.64 (s, 3 H, -OCH₃), 6.59 (s, 1 H, -CHO), 7.23-7.86 (m, 3 H, C³-H, C⁴-H, C⁶-H). 10.3 (s, 1 H, -COOH).

### Beispiel 3

### 2-(3-Nitrophenethenyl)-5-methoxybenzoesäure (5a)

Zur Synthese der Verbindung 5a nach Methode A werden 15,3 g (0,058 mol) Triphenylphosphin, in 100 ml MeOH, 10,0 g (0,058 mol) Nitrobenzylchlorid (gelöst in 50 ml MeOH), 9,6 g (0,058 mol) 2-Formyl-5-methoxybenzoesäure und 28,0 g (0,145 mol) 28%iger NaOMe-Lösung verwendet. Ausbeute: 13.5 g (78 %); Schmelzpunkt: 174,7 °C
IR (ATR): 2838 (COOH), 2627 (C-H)ₐₗᵢₚₕ, 1687 (C=O), 1530, 1348, 1266, 1237, 810, 734, 671. H₁-NMR (DMSO-d6) δ in ppm: 3.80 (s, 3 H, -OCH₃), 6.67 (d, 1 H, J = 12.1 Hz, CH=CH), 6.98-7.00 (m, 2 H, C³H, C⁴-H, 7.12 (d, 1 H, J = 12.0 Hz, CH=CH), 7.43-7.47 (m, 3 H, C⁶-H, C⁵'-H, C⁶'-H), 7.88 (s, 1 H, C²'-H), 7.98 (m, 1 H, C⁴'-H).

### Beispiel 4

### 2-(3-Chlorphenethenyl)-5-methoxybenzoesäure (5b)

Zur Synthese der Verbindung 5b nach Methode A werden 15,3 g (0,058 mol) Triphenylphosphin, in 100 ml MeOH, 9,4 g (0,058 mol) Chlorbenzylchlorid (gelöst in 50 ml MeOH), 9,6 g (0,058 mol) 2-Formyl-5-methoxybenzoesäure und 28,0 g (0,145 mol) 28%iger NaOMe-Lösung verwendet. Ausbeute: 6,7 g (40 %); Schmelzpunkt: 142,3 °C.
IR (ATR) 2835 (COOH), 2563 (C-H)ₐₗᵢₚₕ, 1681 (C=O), 1604, 1594, 1419, 1265, 1251, 1218, 840, 779, 756.
H₁-NMR (DMSO-d6) δ in ppm: 3.79 (s, 3 H, -OCH₃), 7.04 (d, 1 H, J = 16.3 Hz, CH=CH), 7.16 (dd, 1 H, J₁ = 2.7 Hz, J₂ = 7.9 Hz), 7.31-7.36 (m, 2 H, C²'-H, C⁴'-H), 7.41 (d, 1 H, J = 7.6, C⁴-H), 7.46-7.55 (m, 2 H, C⁶-H, C⁶'-H), 7.76 (d, 1 H, J = 8.8 Hz, C³-H), 7.86 (d, 1 H, J = 16.4 Hz, CH=CH).

### Beispiel 5

### 2-(3-Aminophenethyl)-5-methoxybenzoesäure (6a)

10,0 g (0,033 mol) 2-(3-Nitrophenethenyl)-5-methoxybenzoesäure werden in 150 ml EtOH suspendiert und 100 mg Pd/Kohle zugefügt. Der Ansatz wird mehrmals mit H₂ gespült und 72 Stunden bei Raumtemperatur gerührt. Nach dem Abfiltrieren der Pd/Kohle wird im Vakuum eingeengt und der Rückstand mit MeOH/H₂O umkristallisiert.
Ausbeute: 8,5 g (95 %); Schmelzpunkt 108,5 °C
H₁-NMR (DMSO-d6) δ in ppm: 2.61 (2 H, m, -CH₂-CH₂-), 3.05 (m, 2 H, -CH₂-CH₂), 3.75 (s, 3 H, -OCH₃), 6.36-6.44 (m, 3 H, C²'-H, C⁴'-H, C⁶'-H), 6.90 (t, 1 H, J₁ = 7.6 Hz, J₂ = 7.7 Hz, C⁵'-H), 7.01 (d, 1 H, J = 8.5 Hz, C⁴-H), 7.20 (d, 1 H, J = 8.5 Hz, C³-H), 7.3 (s, 1 H, C⁶-H).

### Beispiel 6

### 2-(3-Chlorphenethyl)-5-methoxybenzoesäure (6b)

5,0 g (0,017 mol) 2-(3-Chlorphenethenyl)-5-methoxybenzoesäure werden in einem Gemisch aus 75 ml Ethylacetat und 75 m1 Acetonitril gelöst und 100 mg Pd/Kohle zugefügt. Der Ansatz wird mehrmals mit H₂ gespült und 5 Stunden bei Raumtemperatur gerührt. Nach dem Abfiltrieren der Pd/Kohle wird im Vakuum eingeengt und der Rückstand mit MeOH/H₂O umkristallisiert. Ausbeute: 4,7 g (95 %)
H₁-NMR (DMSO-d6) δ in ppm: 2.73-2,81 (m, 2 H, -CH₂-CH₂-), 3.05-3,13 (m, 2 H, -CH₂-CH₂-), 3,75 (s, 3 H, OCH₃), 6.99-7.35 (m, 6 H, C³-H, C⁴-H, C⁶-H, C²'-H, C⁴'-H, C⁵'-H, C⁶'-H).

### Beispiel 7

### 2-(3-Acetamidophenethyl)-5-methoxybenzoesäure (7a)

20 g (0,074 mol) 2-Aminophenethyl-5-methoxybenzoesäure werden in 50 ml Acetanhydrid gelöst und 15 Stunden bei Raumtemperatur gerührt. Es wird mit 200 ml Eiswasser hydrolysiert mehrmals mit je 3x 200 ml Ethylacetat extrahiert. Nach dem Einengen der organischen Phase im Vakuum erhält man ein gelbes Öl, aus dem das Produkt durch Umkristallisieren mit MeOH/H₂O erhalten wird.
Ausbeute: 16.4 g (0,058 mol) (79,0%); Schmelzpunkt: 145,6 °C
IR (ATR) 3265-2933, 2562 (C-H)ₐₗᵢₚₕ, 1622, 1693, 1593, 1567, 1437, 1420, 1289, 1235,789,747.
H₁-NMR (DMSO-d6) δ in ppm: 2.01 (s, 3 H, -CO-CH₃), 2.74 (m, 2 H, -CH₂-CH₂), 3.08 (m, 2 H, -CH₂-CH₂), 3.74 (s, 3 H, -OCH₃), 6.89 (d, 1 H, J= 7.6 Hz, C⁴'-H), 6.95 (d, 1 H, J = 8.5 Hz, C⁶'-H), 7.12-7.20 (m, 2 H, C²'-H, C⁶'-H), 7.28 (d, 1 H, J = 2.8 Hz, C⁴-H), 7.44-7.46 (m, 2 H, C³-H, C⁶-H), 9.89 (s, 1 H, -NH-).

### Beispiel 8

### 2-Nitro-7-methoxydibenzosuberenon (8d)

Zur Herstellung des 2-Nitro-7-methoxydibenzosuberenons nach Methode B werden 5,1 g (0,019 mol) 2-(3-Nitrophenylethenyl)-5-methoxybenzoesäure, 150 ml Dichlormethan, 2,42 g (0,02 mol) Thionylchlorid und 3,33 g (0,025 mol) AlCl₃ verwendet.
Ausbeute: 58%; Schmelzpunkt 163,7 °C
IR (ATR) 3093-3005 (C-H)ₐᵣₒₘ, 2921-2838, 1703 (C=O), 1521, 1482, 1431, 1347, 1290, 1223, 1257, 1019, 872, 806, 730, 674.
H₁-NMR (DMSO-d6) δ in ppm: 3.81 (s, 3 H, -OCH₃), 6.92 (dd, 1 H, J₁ = 2.4 Hz, J₂ = 8.0 Hz), 7.04 (d. 1 H, J = 2.5, C⁸-H), 7.21 (d, 1 H, J = 7.9, -CH=CH-), 7.70 (d, 1 H, J = 8.0, C³-H), 8.13-8.24 (m, 2 H, C²-H, C⁴-H), 8.34 (s, 1 H, C¹⁰-H), 8.67 (t, 1 H, J = 2.0 H_{z}, C⁷-H).

### Beispiel 9

### 2-Chlor-7-methoxysuberenon (8c)

Zur Herstellung der Verbindung 8c nach Methode B werden 5,5 g (0,019 mol) 2-(3-Chlorphenylethenyl)-5-methoxybenzoesäure, 150 ml Dichlormethan, 2,42 g (0,02 mol) Thionylchlorid und 3,33 g ( 0,025 mol) AlCl₃ verwendet. Schm.133,0 °C; Ausbeute:4,8 g (93 %)
IR (ATR) 2923-2843 (C-H)ₐₗᵢₚₕ, 1636, 1605, 1583 (C=O), 1556, 1503, 1298, 1245,1225, 972, 878, 863, 835, 786.
H₁-NMR (DMSO-d6) δ in ppm: 3.89 (s, 3 H, -OCH₃), 7.06 (d, 1 H, J = 12.1 Hz, -CH=CH-), 7.25 (d, 1 H, J = 12.1 Hz, -CH=CH-), 7.37 (dd, 1 H, J₁ = 2.9 Hz, J₂ = 8.5 Hz, C³-H),7.35-7.40 (m, 2 H, C²-H, C¹⁰-H, 7.72 (d, 1 H, J = 8.7 Hz, C⁸-H), 7.85 (d, 1 H, J = 2.2 Hz, C⁴-H), 8.11 (d, 1 H, J = 8.6 Hz, C⁷-H).

### Beispiel 10

### 2-Acetamido-7-methoxydibenzosuberon (8a)

Zur Herstellung der Verbindung 8a nach Methode B werden 6,0 g (0,019 mol) 2-(3-Acetamidophenethyl)-5-methoxybenzoesäure, 150 ml Dichlormethan, 2,42 g (0,02 mol) Thionylchlorid und 6,66 g ( 0,05 mol) AlCl₃ verwendet. Ausbeute 4,66 g (83%
MS m/z (%): 295 (100, M⁺), 253 (60, M⁺-COCH₃), 238 (12, 253-CH₃), 224 (36, 238-NH), 210 (10), 194 (10), 180 (9), 165 (14), 152 (8).
H₁-NMR 2.07 (s, 3 H, -COCH₃), 3.05 (s, 4 H, -CH₂-CH₂), 3.75 (s, 3 H, -OCH₃), 7.05 (d, 1 H, J = 8.4 Hz, C⁸-H), 7.22 (d, 1 H, J = 8.4 Hz, C⁹-H), 7.38 (s, 1 H, C⁵-H), 7.52-7.56 (m, 2 H, C¹-H, C³-H), 7.92 (d, 1 H, J = 9.3 Hz, C⁴-H), 10.22 (s, 1 H, -NH-).

### Beispiel 11

### 2-Chlor-7-methoxydibenzodibenzosuberon (8b)

Zur Herstellung der Verbindung 8b nach Methode B werden 5,5 g (0,019 mol) 2-(3-Chlorphenylethyl)-5-methoxybenzoesäure, 150 ml Dichlormethan, 2,42 g (0,02 mol) Thionylchlorid und 3,33 g (0,025 mol) AlCl₃ verwendet. Ausbeute 58%
MS m/z (%):274/272 (35/100, M⁺), 259/257 ((5/17, M⁺-CH₃), 243, 241 (19/21, M⁺-OCH₃), 208 (29), 194 (17), 178 (23, 208-CO), 165 (49).
H₁-NMR (DMSO-d6) δ in ppm: 3.09 (s, 4 H, -CH₂-CH₂), 3.77 (s, 3 H, -OCH₃), 7.06 (dd, 1 H, J₁ = 2.8Hz, J₂ = 8.4 Hz, C⁸-H), 7.25 (d, 1 H, J = 8.4 Hz, C³-H), 7.38-7.45 (m, 3 H, C¹-H, C⁶-H, C⁹-H), 7.85 (d, 1 H, J = 8.3, C⁴-H).

### Beispiel 12

### 2-Amino-7-methoxydibenzosuberon (9a)

4,0 g (0,0135 mol) 2-Acetamido-7-methoxydibenzosuberon werden in 100 ml 20%iger HCl suspendiert und das Reaktionsgemisch 5 Stunden unter Rückfluss erhitzt. Beim Abkühlen tritt ein Niederschlag auf, von dem abfiltriert wird. Nach dem Trocknen im Vakuum erhält man ein sandfarbenes Pulver.
Ausbeute: 1,85 g (0,0073 mol) (54,0 %); Schmelzpunkt: 185,3 °C
MS m/z (%): 253 (100, M⁺), 238 (51, M⁺-CH₃), 224 (41, 238-NH), 210 (14), 194 (12), 180 (13), 165 (13), 152 (6).
H₁-NMR 2.96 (s, 4 H, -CH₂-CH₂), 3.75 (s, 3 H, -OCH₃), 6.06 (s, 2 H, -NH₂), 6.34 (s, 1 H, C¹-H), 6.49 (d, 1 H, J = 8.7 Hz, C³-H), 7.00 (d, 1 H, J = 8.3 Hz, C⁸-H), 7.19 (d, 1 H, J = 8.3 Hz, C⁹-H), 7.36 (s, 1 H, C⁶-H), 7.87 (d, 1 H, J = 8.7 Hz, C⁴-H).

### Beispiel 13

### 2-Chlor-7-hydroxydibenzosuberon (12)

Zu einer Lösung von 1,0 g (2,67 mmol) 2-Chlor-7-methoxydibenzosuberon in 10 ml DCM wird unter Argon-Schutzgasatmosphäre bei Raumtemperatur 0,13 ml (1,34 mmol) Bortribromid zugetropft. Die Reaktionsmischung wird bei Raumtemperatur 6 h gerührt, anschließend mit 20 ml Wasser versetzt und die org. Phase im Vakuum eingeengt. Man erhält das Produkt in Form eines orangenen Feststoffes.
Ausbeute: 0,66 g (95%); Schmelzpunkt: 191,6 °C
IR (ATR) 2926, 1589 (C=O), 1561, 1459, 1422, 1363, 1312, 1160, 1080, 1006,837,798. H₁-NMR (DMSO-d6) δ in ppm: 3.05 (dd, 4 H, J₁ = 9.0, J₂ = 13.5, -CH₂-CH₂-), 6.91 (d, 1 H, J = 8.2, C⁸-H), 7.13 (d, 1 H, J = 8.3, C⁹-H), 7.30-7.80 (m, 3 H, C¹-H, C³-H, C⁶-H), 7.82 (d, 1 H, J = 9.1, C⁴-H).
¹³C-NMR (DMSO-d6) δ in ppm: 33.4 (C¹¹), 34.4 (C¹⁰), 116.2 (C⁶), 120.7 (C⁸), 126.9 (C³), 129.3 (C¹), 131.5 (C⁹), 132.5 (C⁴), 133.1 (C^{4a}), 137.1 (C^{5a}), 137.3 (C^{9a}), 138.5 (C²), 144.7 (C^{11a}) 156.1 (C⁷), 193.5 (C⁵).

### Beispiel 14

### 2-Chlor-7-(S-1,2-isopropylidenglycer-3-yl)-10,11-dihydro-dibenzo[a,d]cyclohepten-5-on (13a)

Die Herstellung der Titelverbindung erfolgt nach Methode E. Ausbeute: 89 % C₂₁H₂₁ClO₄ (Mr = 372,85)
MS m/z (%): 374/372 (15/43, M⁺), 359/357 (6/17), 299/297 (26/76), 273/271 (5/13), 260/258 (4/10), 194 (11), 178 (24), 165 (37), 145 (24), 115 (46), 101 (100), 73 (16), 59 (16). ¹H-NMR (DMSO-d6) δ in ppm: 1.29 (s, 3 H, -CH₃), 1.34 (s, 3 H, -CH₃), 3.06 (s, 4 H, -CH₂-CH₂-), 3.73-3.80 (m, 1 H, -OCH₂-), 4.03-4.12 (m, 3 H, -CH₂O-, -OCH₂-), 4.37-4.42 (m, 1 H, - CH-), 7.13 (d, 1 H, J = 8.3, C⁸-H), 7.27 (d, 1 H, J = 8.4, C⁹-H), 7.39-7.47 (m, 3 H, C¹-H, C³-H, C⁶-H), 7.85 (d, 1 H, J = 8.3, C⁴-H).
¹³C-NMR (DMSO-d6) δ in ppm: 25.8 (-CH₃), 26.9 (-CH₃), 33.3 (C¹¹), 34.4 (C¹⁰), 66.0 (-OCH₂-), 69.3 (-CH₂O-), 74.0 (-CH-), 115.0 (C⁶), 120.3 (C⁸), 127.0 (C³), 129.5 (C¹), 131.6 (C⁹), 132.6 (C⁴), 135.1 (C^{4a}), 136.9 (C^{5a}), 137.5 (C^{9a}), 138.6 (C²), 144.8 (C^{11a}), 157.3 (C⁷), 193.2 (C⁵).

### Beispiel 15

### 2-Chlor-7-(R-1,2-isopropylidenglycer-3-yl) -10,11-dihydro-dibenzo[a,d]cyclohepten-5-on (13b)

Die Herstellung der Titelverbindung erfolgt nach Methode E. Ausbeute: 91 %
C₂₁H₂₁ClO₄ (Mr = 372,85); GC 24,6 min
MS m/z (%): 374/372 (16/45, M⁺), 359/357 (6/17), 299/297 (26/76), 273/271 (5/13), 260/258 (4/11), 194 (12), 178 (26), 165 (39), 145 (25), 115 (46), 101 (100), 73 (14), 59 (14). ¹H-NMR (DMSO-d6) δ in ppm: 1.29 (s, 3 H, -CH₃), 1.34 (s, 3 H, -CH₃), 3.10 (s, 4 H, -CH₂-CH₂-), 3.72-3.80 (m, 1 H, -CH₂O-), 4.03-4.09 (m, 3 H, -CH₂O-, -OCH₂-), 4.31-4.46 (m, 1 H, - CH-), 7.13 (d, 1 H, J = 8.6 C⁸-H), 7.27 (d, 1 H, J = 8.5, C⁹-H), 7.39-7.49 (m, 3 H, C¹-H, C³-H, C⁶-H), 7.85 (d, 1 H, J = 7.4, C⁴-H).
¹³C-NMR (DMSO-d6) δ in ppm: 25.7 (-CH₃), 26.9 (-CH₃), 33.3 (C¹¹), 34.3 (C¹⁰), 660 (-CH₂O-), 69.3 (-OCH₂-), 74.0 (-CH-), 115.0 (C⁶), 120.2 (C⁸), 127.0 (C³), 129.5 (C¹), 131.6 (C⁹), 132.6 (C⁴), 135.1 (C^{4a}), 136.9 (C^{5a}), 137.5 (C^{9a}), 138.6 (C²), 144.7 (C^{11a}), 157.3 (C⁷), 193.1 (C⁵).

### Beispiel 16

### 2-Chlor-7-(3-acetoxypropoxy) -10,11-dihydro-dibenzo[a,d]-cyclohepten-5-on (13h)

Die Herstellung der Titelverbindung erfolgt nach Methode G unter Verwendung von 3-lodpropyl-acetat als Edukt in DMF als Solvens. Ausbeute: 87 %
C₂₀H1₉ClO₄(Mr = 358,83); GC24,2 min
MS m/z (%):360/358 (2/6, M⁺), 259/257 (2/5, M⁺-Acetoxypropyl), 243/241 (2/2, M⁺-Acetoxypropoxy), 194 (9, 243/241-Cl), 178 (6), 165 (17), 101 (100, Acetoxypropyl), 73 (11). ¹H-NMR (DMSO-d6) δ in ppm: 1.96-2.08 (m, 5 H, CH₃-Ac, -CH₂-), 3-04-3.13 (m, 4 H, -CH₂-CH₂-), 4.01-4.18 (m, 4 H, -OCH₂-, -CH₂O-), 7.10 (d, 1 H, J = 8.3, C⁸-H), 7.25 (d, 1 H, J = 8.4, C⁹-H), 7.30-7.45 (m, 3 H, C¹-H, C³-H, C⁶-H), 7.57 (d, 1 H, J = 8.3, C⁴-H).
¹³C-NMR (DMSO-d6) δ in ppm: 21.0 (CH₃Ac-), 28.4 (-CH₂-), 33.6 (C¹¹), 34.3 (C¹⁰), 61.1 (AcO-CH₂-), 64.8 (-OCH₂), 114.9 (C⁶), 120.3 (C⁸), 127.6 (C³), 130.3 (C¹), 131.6 (C⁹), 132.6 (C⁴), 135.0 (C^{4a}), 136.9 (C^{5a}), 137.5 (C^{9a}), 138.5 (C²), 144.7 (C^{11a}), 157.3 (C⁷), 170.7 (C=O), 193.1 (C⁵).

### Beispiel 17

### 2-Chlor-7-(3-acetoxyethoxy)-10,11-dihydro-dibenzo[a,d]cyclohepten-5-on (13g)

Die Herstellung der Titelverbindung erfolgt nach Methode G unter Verwendung von 3-lodethyl-acetat als Edukt und DMF als Solvens. Ausbeute: 78 %
C₁₉H₁₇ClO₄ (Mr = 344,80); GC 21,1 min
MS m/z (%): 346/344 (2/4, M⁺), 194 (5, 241/243-Cl), 178 (8), 165 (11), 87 (100, Acetoxyethyl).
¹H-NMR (DMSO-d6) δ in ppm: 2.03 (s, 3 H, -COCH₃), 3.10 (s, 4 H, -CH₂-CH₂-), 4.19-4.23 (m, 2 H, -OCH₂-,), 4.30-4.35 (m, 2 H, -CH₂-O-); 7.11-7.48 (m, 5 H, C¹-H, C³-H, C⁶-H, C⁸-H, C⁹-H), 7.86 (d, 1 H, J = 8.3, C⁴-H).
¹³C-NMR (DMSO-d6) δ in ppm: 21.0 (CH₃-CO-), 33.3 (C¹¹), 34.3 (C¹⁰), 62.8 (AcO-CH₂-), 66.4 (-CH₂O-), 115.0 (C⁶), 120.3 (C⁸), 127.0 (C³), 129.5 (C¹), 131.6 (C⁹), 132.6 (C⁴), 135.2 (C^{11a}), 136.9 (C^{5a}), 137.5 (C^{9a}), 138.6 (C²), 144.8 (C^{4a}), 157.1 C⁷), 170.7 (C=O), 193.1 (C⁵).

### Beispiel 18

### 2-Chloro-7-(2-morpholin-4-yl-ethoxy)-10,11-dihydro-dibenzo[a,d]-cyclohepten-5-on (13c)

Die Herstellung der Titelverbindung erfolgt nach Methode G unter Verwendung von 2-Chlorethylmorpholin-hydrochlorid als Edukt in Acetonitril als Solvens.
C₂₁H₂₂ClNO₃ (Mr = 371,87); GC 32,7 min
MS m/z (%): 373/371 (1/2, M⁺), 330/328 (2/6), 286/284 (2/6, M⁺-Morpholin-4-yl), 178 (6), 165 (4) 100 (100, Morphin-4-yl-methyl),
¹H-NMR (DMSO-d6) δ in ppm: 3.11 (s, 4 H, -CH₂-CH₂), 3.20-3.53 (m, 6 H, -N-CH₂, -CH₂-N-CH₂-), 3.79-4.06 (m, 4 H, -CH₂-O-CH₂-), 4.47-4.50 (m, 2 H, (-OCH₂-), 7.19 (d, 1 H, J = 8.3, C⁸-H), 7.32 (d, 1 H, J = 8.4, C⁹-H), 7.40-7.47 (m, 3 H, C¹-H, C³-H, C⁶-H), 7.86 (d, 1 H, J = 8.4, C⁴-H), 11.57 (s, 1 H, -NH-).
¹³C-NMR (DMSO-d6) δ in ppm: 33.3 (C¹¹), 34.4 (C¹⁰), 57.0 (-CH₂-O-CH₂-), 60.1 (-N-CH₂-), 65.5 (-OCH₂-), 66.1 (-CH₂-O-CH₂), 115.1 (C⁶), 120.3 (C⁸), 127.0 (C³), 129.5 (C¹), 131.6 (C⁹), 132.6 (C⁴), 135.0 (C^{4a}), 136.9 (C^{5a}), 137.5 (C^{9a}), 138.6 (C²), 144.7 (C^{11a}), 157.2 (C⁷), 193.2 (C⁵).

### Beispiel 20

### 2-(2-Aminoanilino)-7-methoxydibenzosuberon (10a)

Zur Herstellung der Verbindung 10a nach Methode C werden 0,5 g (1,8 mmol) 2-Chlor-7-methoxydibenzosuberon, 1,0 (9,2 mmol) g 1,2-Phenylendiamin, 0,05 g Pd(OAc)₂, 0,10 g 2-(Dicyclohexylphosphino)-2'-,4'-, 6'-triisopropyl-biphenyl, 0,70 g KO-tert-Bu, 5 ml Toluol und 1 ml tert-BuOH verwendet. Ausbeute: 0,48 g (78 %); Schmp.: 106,5 °C
C₂₂H₂₀N₂O₂ (Mr = 344,42); GC 49,4 min
MS m/z (%): 344 (100, M⁺), 329 (8), 315 (6), 301 (5), 195 (7), 165 (6), 107 (9).
IR 3418-3343 (C-H)ₐᵣₐₒₘ, 3320 (N-H), 2976-2939 (CH)ₐₗᵢₚₕ, 1546 (C=O), 1516, 1494. 1323, 1284, 1147, 1026, 828, 751, 740.
¹H-NMR (DMSO-d6) δ in ppm: 2.96 (s, 4 H, -CH₂-CH₂), 3.76 (s, 3 H, -OCH₃), 4.84 (s, 2 H, - NH₂), 6.46 (s, 1 H, C¹-H), 6.54-6.64 (m, 2 H, C^{3'}-H, C^{6'}-H), 4,78 (d, 1 H, J = 7.8 Hz, C^{4'}-H), M/49251 6.91-7.04 (m, 3 H, C³-H, C⁵-H, C⁸-H), 7.20 (d, 1 H, J = 8.3 Hz, C⁹-H), 7.39 (s, 1 H, C⁶-H), 7.92-7.97 (m, 2 H, C⁴-H, -NH-).
¹³C-NMR (DMSO-d6) δ in ppm: 33.6 (C¹⁰), 36.6 (C¹¹), 55.5 (-OCH₃), 112.1 (C⁶), 113.1 (C³), 114.8 (C¹), 115.8 (C^{3'}), 116.8 (C^{4'}), 118.3 (C⁸), 125.2 (C^{5'}), 126.2 (C^{6'}), 126.3 (C^{1'}), 126.4 (C^{4a}), 130.4 (C⁹), 133.9 (C^{5a}), 134.5 (C⁴), 140.3 (C^{9a}), 144.0 (C^{2'}), 145.7 (C^{11a}), 151.1 (C²). 158.0 (C⁷), 190.3 (C⁵).

### Beispiel 21

### 2-(2-Amino-4-fluoranilino)-7-methoxydibenzosuberon (10b)

Zur Herstellung der Verbindung 10b nach Methode C werden 0,5 g (1,8 mmol) 2-Chlor-7-methoxydibenzosuberon, 0,3 g (1,9 mmol) 2-Nitro-4-fluoranilin, 0,05 g Pd(OAc)₂, 0,10 g 2-(Dicyclohexylphosphino)-2'-,4'-, 6'-triisopropyl-biphenyl, 0,70 g KO-tert-Bu, 5 ml Toluol und 1 ml tert-BuOH verwendet. Die Aufreinigung der Nitroverbindung erfolgt durch Umkristallisieren mit MeOH. Ohne weitere Aufreinigung werden 0,5 g der Nitroverbindung, 2,9 g ZnCl₂ x2 H₂O in 20 ml EtOH nach Methode D reduziert. Ausbeute:: 0,42 g (65%); Schmp.: 72.2 °C C₂₂H₁₉FN₂O₂ (Mr = 362,41); GC 54,1 min
MS m/z (%): 362 (100, M⁺), 247 (7), 125 (14).
IR (ATR) 3353 (N-H), 2941-2834 (C-H)ₐₗᵢₚₕ, 1622, 1567 (C=O), 1508, 1324, 1273, 1162, 839, 784.
¹H-NMR (DMSO-d6) δ in ppm: 2.49 (s, 4 H, -CH₂-CH₂), 3.76 (s, 3 H, -OCH₃), 5.25 (s, 2 H, - NH₂), 6.30-6.38 (m, 2 H, C^{3,}-H, C^{5'}-H), 6.51-6.58 (m, 2 H, C³-H, C^{6'}-H), 7.94-7.05 (m, 2 H, C¹-H, C⁸-H), 7.21 (d, 1 H, J = 8.3 Hz, C⁹-H), 7.38 (s, 1 H, C⁶-H) 7.89 (s, 1 H, -NH-), 7.93 (d, 1 H, C⁴-H).
¹³C-NMR (DMSO-d6) δ in ppm: 33.6 (C¹⁰), 36.5 (C¹¹), 55.5 (-OCH₃), 101.4 (d, J = 25.5 Hz, C^{3'}), 102.5 (d, J = 22.6 Hz, C^{5'}), 111.9 (C⁶), 112.9 (C³), 114.8 (C¹), 118.4 (C⁸), 121.3 (d, J = 2.3 Hz, C^{1'}), 126.2 (C^{4a}), 128.4 (d, J = 20.8 Hz, C^{6'}), 130.4 (C⁹), 134.0 (C^{5a}), 134.5 (C⁴), 140.3 (C^{9a}), 145.8 (C^{11a}), 146.5 (d, J = 12.0 Hz, C^{2'}), 151.5 (C²), 158.0 (C⁷), 161.2 (d, J = 238.6 Hz, C^{4'}), 190.2 (C⁵).

### Beispiel 22

### 2-(2, 4-Difluoranilino)-7-methoxydibenzosuberon (10c)

Zur Herstellung der Verbindung 10c nach Methode C werden 0,5 g (1,8 mmol) 2-Chlor-7-methoxydibenzosuberon, 0,25 g (1,9 mmol) 2, 4-Difluoranilin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70 g KO-tert-Bu, 5 ml Toluol und 1 ml tert-BuOH verwendet. Ausbeute: 0,57 g (87%); Schmp.: 123-126 °C (Zersetzung); C₂₂H₁₇F₂NO₂ (Mr = 365,38); GC 31,4 min
MS m/z (%): 365 (100, M⁺), 350 (7, M⁺-CH₃), 337 (11), 322 (6), 237 (4, M⁺-2-NH₂, 4-F-Anilin), 208 (15), 194 (5), 178 (6), 165 (15), 152 (5).
IR (ATR) 3327 (N-H), 3074 (C-H)ₐᵣₒₘ, 2941-2842 (C-H)ₐₗᵢₚₕ, 1338, 1551 (C=O), 1525, 1498, 1325, 1281, 1238, 854, 832, 786.
¹H-NMR (DMSO-d6) δ in ppm: 2.99 (s, 4 H, -CH₂-CH₂), 3.76 8s, 3 H, -OCH₃), 6.61 (s, 1 H, C¹-H), 6.74 (d, 1 H, J = 8.7 Hz, C³-H), 7.00-7.47 (m, 6 H, C⁶-H, C⁸-H, C⁹-H, C^{3'}-H, C^{5'}-H, C^{6'}-H), 7.95 (d, 1 H, J = 8.8 Hz, C⁴-H), 8.55 (s, 1 H, -NH-).
¹³C-NMR (DMSO-d6) δ in ppm: 36.2 (C¹⁰), 38.6 (C¹¹), 55.5 (-OCH₃), 105.3 (dd, J = 26.7 Hz, C^{3'}), 112.2 (d, J = 22.1 Hz, C^{5'}), 112.6 (C⁶), 113.9 (C³), 114.7 (C¹), 118.6 (C⁸), 125.3 (d, J = 12.2 Hz, C^{1'}), 126.4 (d, J = 9.7 Hz, C^{6'}), 127.7 (C^{4a}), 130.6 (C⁹), 133.8 (C^{5a}), 134.6 (C⁴), 140.0 (C^{9a}), 145.7 (C^{11a}), 149.4 (C²), 154.9 (d, J = 143.5 Hz, C^{4'}), 158.1 (C⁷), 159.8 (d, J = 138.2 Hz, C^{7'}), 190.8 (C⁵).

### Beispiel 23

### 2-(2-Chlor-4-fluoranilino)-7-methoxydibenzosuberon (10d)

C₂₂H₁₇ClFNO₂ (Mr = 381,84)
Zur Herstellung der Verbindung 10d nach Methode C werden 0,5 g (1,8 mmol) 2-Chlor-7-methoxydibenzosuberon, 0,27 g (1,9 mmol) 2-Chlor, 4-fluoranilin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70 g KO-tert-Bu, 5 ml Toluol und 1 ml tert-BuOH verwendet. Ausbeute: 0,24 g(35%);
Schmp.: 110,2 °C; GC 38,4 min
MS m/z (%): 385/383 (36/100, M⁺), 368/366 (2/6, M⁺-CH₃), 354/352 (4/9), 340/338 (2/4), 274/272 (2/6), 237 (4, M⁺-2-Cl, 4-F-Anilin), 208 (14), 194 (5), 178 (6, 208-CO), 165 (13), 151 (4), 136 (6).
IR (ATR) 3336 (N-H), 3070 (C-H)ₐᵣₒₘ, 2939-2835 (C-H)_{aliph,} 1602, 1554 (C=O), 1520, 1484, 1282, 1255, 1235, 863, 818, 785.
¹H-NMR (DMSO-d6) δ in ppm: 3.00 (s, 4 H, -CH₂-CH₂-), 3.77 (s, 3 H, -OCH₃), 6.60 (s, 1 H, C¹-H), 6.72 (d, 1 H, J = 8.8 Hz, C³-H), 7.03 (d, 1 H, J = 5.6 Hz, C^{6'}-H), 7.18-7.57 (m, 5 H, C⁶-H, C⁸-H, C⁹-H, C^{3'}-H, C^{5'}-H), 7.94 d, 1 H, J = 8.7 Hz, C⁴-H), 8.46 (s, 1 H, -NH-).
¹³C-NMR (DMSO-d6) δ in ppm: 33.5 (C¹²), 36.2 (C¹¹), 55.5 (OCH₃), 112.8 (C⁶), 114.1 (C³), 114.6 (C¹), 115.6 (C^{5'}), 117.7 (C^{3'}), 118.6 (C⁸), 127.3 (C^{1'}), 127.8 (C⁴), 129.4 (C^{2'}), 130.6 (C⁹), 133.7 (C^{5a}), 134.7 (C⁴), 140.0 (C^{9a}), 145,7 (C^{11a}), 149.5 (C²), 158.1 (C⁷), 158.9 (C^{4'}), 190.8 (C⁵).

### Beispiel 24

### 2-(2,4,5-Trifluoranilino)-7-methoxydibenzosuberon (10e)

C₂₂H₁₆F₃NO₂ (Mr = 383,37)
Zur Herstellung der Verbindung 10e nach Methode C werden 0,5 g (1,8 mmol) 2-Chlor-7-methoxydibenzosuberon, 0,27 g (1,8 mmol) 2-, 4-, 5-Trifluoranilin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70 g KO-tert-Bu, 5 ml Toluol und 1 ml tert-BuOH verwendet. Ausbeute: 0,30 g (43%);
Schmp.: 132.1 °C; GC 30,0 min
MS m/z (%): 383 (100, M⁺), 368 (8, M⁺-CH₃), 355 (11), 340 (7), 237 (4, M⁺-2, 3, 4-Trifluoranilin), 208 (13), 194 (5), 178 (8), 165 (13).
IR (ATR) 3337 (N-H), 3298-3016 (C-H)ₐᵣₒₘ, 2912-2846 (C-H)ₐₗᵢₚₕ, 1582, 1567, 1518 (C=O), 1286, 1273, 1221, 1161, 856, 832, 808.
¹H-NMR (DMSO-d6) δ in ppm: 3.01 (s, 4 H, -CH₂-CH₂), 3.76 (s, 3 H, -OCH₃), 6,72 (s, 1 H, C¹-H), 6.82-7.05 (m, 2 H, C^{3'}-H, C^{6'}-H), 7.21 (d, 1 H, J = 8.3 Hz, C³-H), 7.38-7.65 (m, 2 H, C⁸-H, C⁹-H), 7.98 (d, 1 H, J = 7.98Hz, C²-H), 8.65 (s, 1 H, -NH-).
¹³C-NMR (DMSO-d6) δ in ppm: 33.5 (C¹⁰), 36.1 (C¹¹), 55.5 (-OCH₃), 106.4-107.4 (m, C^{3'}), 111.9-112.3 (m, C^{6'}), 113.3 (C⁶), 114.6 (C³), 114.7 (C¹), 118.7 (C⁸), 125.6-126.2 (m, C^{1'}), 126.2 (C^{4a}), 128.6 (C⁹), 130.6 (C^{5a}), 133.6 (C⁴), 134.6 (C^{9a}), 142.6-147.9 (m, C^{4'}), 143.6-148.9 (m, C^{5'}), 145.5 (C^{11a}), 148.3 (C²), 148.3-153,3 (m, C^{2'}), 158.1 (C⁷), 191.1 (C⁵).

### Beispiel 25

### 2-(2-Trifluormethylanilino)-7-methoxydibenzosuberon (10f)

Zur Herstellung der Verbindung 10f nach Methode C werden 0,5 g (1,8 mmol) 2-Chlor-7-methoxydibenzosuberon,0,3 (1,9 mmol) 2-Aminotrifluorid, 0,05 g (0,22 mmol) Pd(OAc)₂, 0,10 g (0,21 mmol) 2-(Dicyclohexylphosphino)-2'-,4'-, 6'-triisopropyl-biphenyl, 0,70 g KO-tert-Bu, 5 ml Toluol und 1 ml tert-BuOH verwendet. Ausbeute: 0,23 g (32%); Schmp.: 152,5 °C; C₂₃H₁₈F₃NO₂ (Mr = 397,40); GC 30,3 min
MS m/z (%): 397 (100, M⁺), 282 (7, M⁺-CH₃), 368 (12), 354 (6), 237 (5, M⁺-2-CF₃-Anilin), 208 (12), 194 (5),178 (6), 165 (14).
IR (ATR) 3319 (N-H), 3072-3007 (C-H)ₐᵣₒₘ, 2958-2836 (C-H)ₐₗᵢₚₕ, 1550 (C=O), 1460, 1353, 1323, 1285, 1264, 1111, 1091, 1050, 692.
¹H-NMR (DMSO-d6) δ in ppm: 3.02 (s, 4 H, -CH₂-CH₂-), 3.76 (s, 3 H, -OCH₃), 6.91 (s, 1 H, C¹-H), 7.01-7.06 (m, 2 H, C³-H, C^{6'}-H), 7.20-7.25 (m, 2 H, C^{2'}-H, C^{4'}-H), 7.37-7.39 (m, 2 H, C⁸-H, C^{5'}-H), 7.47-7.52 (m, 2 H, C⁶-H, C⁹-H), 7.98 (d, 1 H, J = 8.7 Hz, C⁴-H), 9.10 (s, 1H, - NH-).
¹³C-NMR (DMSO-d6) δ in ppm: 33.5 (C¹⁰), 36.1 (C¹¹), 55.5 (-OCH₃), 114.0 (C⁶), 114.5 (C³), 114.9-117.9 (m, C^{2'}), 115.1 (C¹), 118.7 (C^{4'}), 121.7 (C^{4a}), 127.1 (C¹¹), 129.1 (C^{3'}), 129.1-132.6 (m, -CF₃), 130.7 (C⁹), 133.7 (C^{5a}), 134.6 (C⁴), 139.9 (C^{9a}), 142.7 (C^{1'}), 145.7 (C^{11a}), 147.3 (C²), 158.1 (C⁷), 191.2 (C⁵).

### Beispiel 26

### 2-(Anilino)-7-methoxydibenzosuberon (10g)

Zur Herstellung der Verbindung 10g nach Methode C werden 0,5 g (1,8 mmol) 2-Chlor-7-methoxydibenzosuberon, 0,2 g (2,1 mmol) Anilin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70g (6,2 mmol) 0,70 g KO-tert-Bu, 5 ml Toluol und 1 ml tert-BuOH verwendet. Ausbeute: 0,27 g (46%);
Schmp.: 123.5 °C; C₂₂H₁₉NO₂ (Mr = 329,40); GC 35,1 min
MS m/z (%): 329 (100, M⁺), 314 (6, M⁺-CH₃), 300 (11), 286 (5), 270 (3), 254 (3), 237 (2,M⁺-Anilin), 208 (10), 194 (4), 180 (4), 165 (11).
IR (ATR) 3329 (N-H), 2992-2853 (C-H)ₐₗᵢₚₕ, 1560 (C=O), 1520, 1494, 1319, 1280, 1262, 1240, 1035, 817,742.
¹H-NMR (DMSO-d6) δ in ppm: 2.99 (s, 4 H, -CH₂-CH₂), 3.75 (s, 3 H, -OCH₃), 6.93-7.05 (m, 3 H, C³-H, C^{4'}-H, C^{6'}-H), 7.16-7.37 (m, 5 H, C⁶-H, C⁸-H, C⁹-H, C^{3'}-H, C^{5'}-H), 7.95 (d, 1 H, J = 8.7 Hz, C⁴-H), 8.81 (s, 1 H, -NH-).
¹³C-NMR (DMSO-d6) δ in ppm: 34.6 (C¹⁰), 36.3 (C¹¹), 55.5 (-OCH₃), 113.1 (C⁶), 114.5 (C³), 114.6 (C¹), 118.6 (C⁸), 120.0 (C^{2'}), 120.0 (C^{6'}), 122.5 (C^{4'}), 127.7 (C^{4a}), 129.7 (C^{3'}), 129.7 (C^{5'}), 130.6 (C⁹), 133.9 (C^{5a}), 134.7 (C⁴), 140.0 (C^{9a}), 141.4 (C^{1'}), 145.9 (C^{11a}), 148.6 (C²), 158.9 (C⁷), 190.9 (C⁵).

### Beispiel 27

### 2-(2-Methoxyanilino)-7-methoxydibenzosuberon (10h)

Zur Herstellung der Verbindung 10h nach Methode C werden 0,5 g (1,8 mmol) 2-Chlor-7-methoxydibenzosuberon, 0,25 g (2,0 mmol) 2-Methoxyanilin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70 g KO-tert-Bu, 5 ml Toluol und 1 ml tert-BuOH verwendet. Ausbeute: 0,55 g (85%); Schmp.: 145.0 °C; C₂₃H₂₁NO₃ (Mr = 359,43); GC 49,7 min
MS m/z (%): 359 (100, M⁺), 344 (5, M⁺-CH₃), 326 (2), 316 (3), 208 (3), 196 (14), 183 (16), 165 (7), 152 (2), 136 (2), 121 (5), 108 (2).
IR (ATR) 3323 (N-H), 3002 (C-H)ₐᵣₒₘ, 1956-2829 (C-H)_{aliph,} 1557 (C=O), 1520, 1489, 1322, 1289, 1271,1252, 1211, 1111, 1027, 866, 754.
¹H-NMR (DMSO-d6) δ in ppm: 2.98 (s, 4 H, -CH₂-CH₂), 3.76 (s, 3 H, -OCH₃), 3.79 (s, 3 H, - OCH₃), 6.71 (s, 1 H, C¹-H), 6.83 (d, 1 H, J = 8.9 Hz, C³-H), 6.97-7.09 (m, 3 H, C^{3'}-H, C^{4'}-H, C^{5'}-H), 7.19-7.30 (m, 2 H, C⁸-H, C⁹-H), 7.38 (s, 1 H, C⁶-H), 7.93 (d, 1 H, J = 8.8 Hz, C⁴-H), 8.17 (s, 1 H, -NH-).
¹³C-NMR (DMSO-d6) δ in ppm: 33.6 (C¹⁰), 36.4 (C¹¹), 55.5 (-OCH₃), 55.8 (-OCH₃), 112.4 (C⁶), 112.8 (C³), 114.1 (C^{3'}), 114.7 (C¹), 118.4 (C⁸), 121.0 (C^{4'}), 122.7 (C^{6'}), 124.6 (C^{5'}), 127.1 (C^{4a}), 129.6 (C^{1'}), 130.5 (C⁹), 133.7 (C^{5a}), 134.5 (C⁴), 140.2 (C^{9a}), 145.6 (C^{11a}), 149.7 (C^{2'}), 152.3 (C²), 158.0 (C⁷), 190.6 (C⁵).

### Beispiel 28

### 2-(3-Methyl-4-fluoranilino)-7-methoxydibenzosuberon (10i)

Zur Herstellung der Verbindung 10i nach Methode C werden 0,5 g (1,8 mmol) 2-Chlor-7-methoxydibenzosuberon, 0,25 g (2,0 mmol) 3-Methyl, 4-fluoranilin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70 g KO-tert-Bu, 5 ml Toluol und 1 ml tert-BuOH verwendet. Ausbeute: 0,51 g (78%); Schmp.: 158,9 °C; C₂₃H₂₀FNO₂ (Mr = 361,42); GC 39,8 min
MS m/z (%):361 (100, M⁺), 346 (6, M⁺-CH₃), 333 (9), 237 (2, M⁺-3-CH₃, 4-F-Anilin), 208 (10), 178 (3), 165 (10).
IR (ATR) 3363 (N-H), 3013 (C-H)ₐᵣₒₘ, 2934-2838 (C-H)ₐₗᵢₚₕ, 1582, 1561 (C=O), 1501, 1286, 1270, 1210, 1048, 971, 862, 806, 768.
¹H-NMR (DMSO-d6) δ in ppm: 2.21 (s, 3 H, -CH₃), 3.00 (s, 4 H, -CH₂-CH₂), 3.77 (s, 3 H, - OCH₃), 6.75 (s, 1 H, C¹-H), 6.89 (d, 1 H, J = 8.8 Hz, C³-H), 7.00-7.14 (m, 4 H, C⁸-H, C⁹-H, C^{5'}-H, C^{6'}-H), 7.22 (d, 1 H, J = 8.3 Hz, C^{2'}-H), 7.39 (s, 1 H, C⁶-H) 7.96 (d, 2 H, J = 8.8 Hz, C⁴-H), 8.69 (s, 1 H, -NH-).
¹³C-NMR (DMSO-d6) δ in ppm: 14.7 (-CH₃), 33.5 (C¹⁰), 36.3 (C¹¹), 55.7 (-OCH₃), 112.6 (C⁶), 114.0 (C³), 114.7 (C¹), 115.9 (C^{5'}), 118.5 (C⁸), 119.9 (C^{2'}), 123.8 (C^{6'}), 125.4 (C^{3'}), 127.4 (C^{4a}), 130.5 (C⁹), 134.0 (C^{5a}), 134.5 (C⁴), 137.3 (C^{1'}), 140.1 C^{9a}), 145.9 (C^{11a}), 149.2 (C²), 156.8 (C^{4'}), 158.1 (C⁷), 190.6 (C⁵).

### Beispiel 29

### 2-(2-Amino-4-trifluormethylanilino)-7-methoxydibenzosuberon (10j)

Zur Herstellung der Verbindung 10j nach Methode C werden 0,5 g (1,8 mmol) 2-Chlor-7-methoxydibenzosuberon, 0,4 g (1,9 mmol) 2-Nitro-4-trifluormethylanilin, 0,05 g Pd(OAc)₂, 0,10 g 2-(Dicyclohexylphosphino)-2'-,4'-, 6'-triisopropyl-biphenyl, 0,70 g KO-tert-Bu, 5 ml Toluol und 1 ml tert-BuOH verwendet. Die Aufreinigung der Nitroverbindung erfolgt durch Umkristallisieren mit MeOH. Ohne weitere Aufreinigung werden 0,5 g der Nitroverbindung, 2,9 g ZnCl₂ x2 H₂O in 20 ml EtOH nach Methode D reduziert. Schmp.: 78,9 °C; GC: 50,8 min; C₂₃H₁₉F₃N₂O₂ (Mr = 412,42)
MS m/z (%): 412 (100, M⁺), 397 (8, M⁺-CH₃), 383 (4), 369 (6), 263 (6), 237 (M⁺-2-NH₂, 4-CF₃-Anilin), 208 (4), 192 (4), 175 (13).
IR (ATR): 3344 (N-H), 2943 (C-H)ₐₗᵢₚₕ, 1567 (C=O), 1520,1496, 1333, 1283, 1260, 1213, 1162, 1147, 1112, 1036.
¹H-NMR (DMSO-d6) δ in ppm: 3.00 (s, 4 H, -CH₂-CH₂-), 3.77 (s, 3 H, -OCH₃), 5.33 (s, 2 H, - NH₂), 6.62 (s, 1 H, C¹-H), 6.76 (d, 1 H, J = 8.8 Hz, C³-H), 6.85 (d, 1 H, J = 8.1 Hz, C^{6'}-H), 7.01-7.06 (m, 2 H, C^{3'}-H, C^{5'}-H), 7.25-7.21 (m, 2 H, C⁸-H, C⁹-H), 7.39 (s, 1 H, C⁶-H), 7.95 (d, 1 H, J = 8.7 Hz, C⁴-H), 8.09 (s, 1 H, -NH-).
¹³C-NMR (DMSO-d6) δ in ppm: 33.6 (C¹⁰), 36.3 (C¹¹), 55.5 (-OCH₃), 111.7 (C^{3'}), 123.2 (C^{5'}) 113.1 (C⁶), 114.3 (C³), 114.7 (C¹), 118.6 (C⁸), 125.5 (-CF₃), 124.0 (C^{6'}), 125.8 (C^{4'}), 127.5 (C^{4a}), 129.3 (C^{2'}), 130.6 (C⁹), 133.8 (C^{5a}), 134.6 (C⁴), 140.1 (C^{9a}), 143.2 (C^{1'}), 145.7 (C^{11a}), 149.4 (C²), 158.1 (C⁷), 190.7 (C⁵).

### Beispiel 30

### 2-(Phenyl)-7-methoxydibenzosuberon (10k)

Eine Mischung von 0,137 g (0,50 mmol) 2-Chlor-7-methoxydibenzosuberon, 0,092mg (0,75 mmol) Phenylboronsäure, 2,2 mg (2 mol%) Pd(OAc)₂, 0,138 g (1,0 mol) K₂CO₃, 4,0 g PEG-400 werden bei 45°C für 5 h gerührt, bis eine vollständige Umsetzung auf der DC beobachtet wird. Zur Mischung werden 15 ml NaOH zugefügt und 4x mit je 15 ml Diethylether extrahiert. Es wir im Vakuum eingeengt. Die Aufreinigung erfolgt über Flash-Chromatographie (SiO₂, Hexan/Ethylacetat 9+1). Ausbeute: 0,05 g (32%); Schmp.: 97,8 °C;
C₂₂H₁₈O₂ (Mr = 314,39); GC 24,7 min
MS m/z (%): 314 (100, M⁺), 299 (11, M⁺-CH₃), 285 (20), 271 (11), 255 12), 239 (11), 228 (6), 215 (5), 178 (4), 165 (10), 120 (4).
IR (ATR) 3027 (C-H)ₐᵣₒₘ, 2993-2834 (C-H)ₐₗᵢₚₕ, 1640, 1601 (C=O), 1493,1287 (C-O), 1245, 1048, 977,805, 770, 701.
¹H-NMR (DMSO-d6) δ in ppm: 2.47-2.51 (m, 4 H, -CH₂-CH₂), 3.78 (s, 3 H, -OCH₃), 7.08-7.12 (m, 1 H, C^{4'} -H), 7.26 (d, 1 H, J = 8.4 Hz, C³-H), 7.40-7.48 (m, 4 H, C¹-H, C⁶-H, C⁸-H, C⁹-H), 7.64 (m, 4 H, C^{2'}-H, C^{3'}-H, C^{5'}-H, C^{6'}-H), 7.97 (d, 1 H, J = 8.8 Hz, C²-H).
¹³C-NMR (DMSO-d6) δ in ppm: 33.6 (C¹⁰), 35.0 (C¹¹), 55.6 (-OCH₃), 114.2 (C⁶), 119.5 (C⁸), 125.0 (C³), 127.3 (C^{6'}), 127.3 (C^{2'}), 128.1 (C^{4'}), 128.7 (C⁹), 129.4 (C^{5'}), 129.4 (C^{3'}), 131.4 (C¹), 131.5 (C⁴), 134.9 (C^{4a}), 136.9 (C^{5a}), 139.1 (C^{9a}), 139.1 (C^{1'}), 143.4 (C²), 144.3 (C^{11a}), 158.1 (C⁷), 193.7 (C⁵).

### Beispiel 31

### 2-(2, 4-Difluoranilino)-7-methoxydibenzosuberenon (10I)

Zur Herstellung der Verbindung 10I nach Methode C werden 0,5 g (1,8 mmol) 2-Chlor-7-methoxydibenzosuberenon, 0,25 g (1,9 mmol) 2, 4-Difluoranilin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70 g KO-tert-Bu, 5 ml Toluol und 1 ml tert-BuOH verwendet. Ausbeute: 0,53 g (81%); Schmp.: 202,4 °C; C₂₂H₁₅F₂NO₂ (Mr = 363,37); GC 36,2 min
MS m/z (%): 363 (100, M⁺), 335 (52), 292 (48), 272 (6), 152 (13), 13), 145 (10), 136 (5). IR (ATR) 3313 (N-H), 3098-3018 (C-H)ₐᵣₒₘ, 2923-2851 (C-H)ₐₗᵢₚₕ, 1611, 1566 (C=O), 1531, 1498, 1360, 1345, 1280, 1259, 1096, 847, 834
¹H-NMR (DMSO-d6) δ in ppm: 3.87 (s, 3 H, -OCH₃), 6.86-6.92 (m, 2 H, C¹-H, C³-H), 6.99-7.11 (m, 3 H, C^{3'}-H, C^{5'}-H, C^{6'}-H), 7.28-7.47 (m, 3 H, C⁸-H, C¹⁰-H, C¹¹-H), 7.63-7.72 (m, 2 H, C⁶-H, C⁹-H), 8.11 (d, 2 H, C⁴-H), 8.65 (s, 1 H, -NH).
¹³C-NMR (DMSO-d6) δ in ppm: 55.8 (-OCH₃), 105.4 (dd, J = 24.1 Hz, C^{3'}) 112.2 (d, J = 25.7 Hz, C^{5'}), 113.1 (C⁶), 114.0 (C¹), 115.9 (C³), 119.9 (C⁸), 125.3 (d, J = 12.1, C^{1'}), 126.3 (d, J = 9.7 Hz, C^{6'}), 128.6 (C^{4a}), 128,7 (C^{5a}), 130,0 (C¹⁰), 131.7 (C¹¹), 133.1 (C⁹), 134.2 (C⁴), 137.6 (C^{9a}), 139.7 (C^{11a}), 149.1 (C²), 155.0 (d, J = 163.7 Hz, C^{4'}) 160.0 (d, J = 163.7 Hz, C^{2'}), 160.0 (C⁷), 188.1 (C⁵).

### Beispiel 32

### 2-(2, 4-Difluoranilino)-7-(S-1,2-isopropylidenglycer-3-yl)-10,11-dihydro-dibenzo[a,d]-cyclohepten-5-on (14a)

Zur Herstellung der Verbindung nach Methode C werden 0,67 g (1,8 mmol) 2-Chlor-7-(S-1,2-isopropylidenglycer-3-yl)-10,11-dihydrodibenzo[a,d]-cyclohepten-5-on, 0,25 g (1,9 mmol) 2, 4-Difluoranilin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70g (6,2 mmol) KOt-Bu, 5 ml Toluol und 1 ml t-BuOH verwendet. Ausbeute: 0,57 g(68%); Schmp.: 114,6 °C;
C₂₇H₂₅F₂₂O₄ (Mr = 465,50); GC 76,6 min
MS m/z (%): 465 (100, M⁺), 450 (6, M⁺-CH₃), 390 (36), 364 (42), 351, (40), 35 (7), 323 (11), 178 (38), 165 (16), 115 (13),101 (31).
IR (ATR) 3352 (N-H), 2997-2944 (C-H)ₐₗᵢₚₕ, 1560, 1509 (C=O), 1289,1262, 1140, 843, 814, 802
¹H-NMR (DMSO-d6) δ in ppm: 1.29 (s, 3 H, -CH₃), 1.34 (s, 3 H, -CH₃), 3.31 (s, 4 H, -CH₂-CH₂), 3.72-4.45 (m, 5 H, -OCH₂-, -CH-, -CH₂O-), 6.60 (s, 1 H, C¹-H), 6.73 (d, 1 H, J = 9.3, C³-H), 7.00-7.47 (m, 6 H, C⁶-H, C⁸-H, C⁹-H), C^{3'}-H, C^{5'}-H, C^{6'}-H), 7.94 (d, 1 H, J = 8.6, C⁴-H), 8.54 (s, 1 H, -NH-).
¹³C-NMR (DMSO-d6) δ in ppm: 25,8 (-CH₃), 26.9 (-CH₃), 33.5 (C¹¹), 36.2 (C¹⁰), 66.0 (-CH₂-O-), 69.2 (-O-CH₂-), 74.1 (-OCH-), 105.3 (d, J = 24.3 C^{3'}), 109.2 (-C-), 112.1 (d, J = 21.9 (C^{5'}), 112.6 (C⁶), 113.9 (C³), 115.4 (C¹); 119.1 (C⁸), 125.3 (d, J = 12.0, C^{1'}), 126.4 (d, J = 9.8, C^{6'}), 127.7 C^{4a}), 130.6 (C⁹), 133.8 (C^{5a}), 134.9 (C⁴) 140.1 (C^{9a}), 145.7 (C^{11a}), 149.5 (C²), 154.5 (d, J = 152.8, C^{4'}), 157.2 (C⁷); 160.2 (d, J = 169.5, C^{2'}) 190.7 (C⁵).

### Beispiel 33

### 2-(2, 4-Difluoranilino)-7-(R-1,2-isopropylidenglycer-3-yl)-10,11-dihydro-dibenzo[a,d]-cyclohepten-5-on (14b)

Zur Herstellung der Verbindung nach Methode C werden 0,67 g (1,8 mmol) 2-Chlor-7-7-(R-1,2-isopropylidenglycer-3-yl)-10,11-dihydrodibenzo[a,d]-cyclohepten-5-on, 0,25 g (1,9 mmol) 2, 4-Difluoranilin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70g (6,2 mmol) KOt-Bu, 5 ml Toluol und 1 ml t-BuOH verwendet. Ausbeute: 65 %; C₂₇H₂₅F₂₂O₄ (Mr = 465,50)

### Beispiel 34

### 2-(2-Aminoanilino)-7-(S-1,2-isopropylidenglycer-3-yl)-10,11-dihydro-dibenzo[a,d]-cyclohepten-5-on (14c)

Zur Herstellung der Verbindung nach Methode C werden 0,67 g (1,8 mmol) 2-Chlor-7-7-(S-1,2-isopropylidenglycer-3-yl)-10,11-dihydrodibenzo[a,d]-cyclohepten-5-on, 1,0 (9,2 mmol) g 1, 2-Phenylendiamin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70g (6,2 mmol) KOt-Bu, 5 ml Toluol und 1 ml t-BuOH verwendet. C₂₇H₂₈N₂O₄ (Mr = 444,54)
¹H-NMR (DMSO-d6) δ in ppm: 1.29 (s, 3 H, -CH₃), 1.34 (s, 3 H, -CH₃), 2.96 (s, 4 H, -CH₂-CH₂-), 3.72-3.79 (m, 1 H, -CH₂O-), 4.01-4.31 (m, 3 H, -CH₂O-, -CH₂O-), 4.37-4.42 (m, 1 H, - CH-), 4.83 (s, 2 H, -NH₂), 6.45 (s, 1 H, C¹-H), 6.53-6.63 (m, 2 H, C^{3'}-H, C^{6'}-H), 6.77 (d, 1 H, J = 7.4, C^{4'}-H), 6.90-7.05 (m, 3 H, C³-H, C⁸-H, C^{5**'**}-H), 7.20 (d, 1 H, J = 8.4, C⁹-H), 7.39 (s, 1 H, C⁶-H), 7.91-7.96 (m, 2 H, C⁴-H, -NH-).
¹³C-NMR (DMSO-d6) δ in ppm: 25.8 (-CH₃), 26.9 (-CH₃), 33.6 (C¹⁰), 36.6 (C¹¹), 66.1 (C³-Glyceryl), 69.2 (C¹-Glyceryl), 74.1 (C²-Glyceryl), 112.1 (C⁶), 113.1 (C³), 115.5 (C¹), 115.8 (C^{3'}), 116.8 (C^{4'}), 118.8 (C⁸), 125.2 (C^{5'}); 126.2 (C^{6'}), 126.2 (C^{1'}), 126.4 (C^{4a}), 130.4 (C⁹), 133.9 (C^{5a}), 134.8 (C⁴), 140.3 (C^{9a}), 144.0 (C^{2'}), 145.8 (C^{11a}), 151.1 (C²), 157.2 (C⁷), 190.2 (C⁵).

### Beispiel 35

### 2-(2-Aminoanilino)-7-(R-1,2-isopropylidenglycer-3-yl)-10,11-dihydro-dibenzo[a,d]-cyclohepten-5-on (14d)

Zur Herstellung der Verbindung nach Methode C werden 0,67 g (1,8 mmol) 2-Chlor-7-(R-1,2-isopropylidenglycer-3-yl)-10,11-dihydrodibenzo[a,d]-cyclohepten-5-on, 1,0 (9,2 mmol) g 1, 2-Phenylendiamin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70g (6,2 mmol) KOt-Bu, 5 ml Toluol und 1 ml t-BuOH verwendet. Ausbeute: 72 %; C₂₇H₂₈N₂O₄ (Mr = 444,54)
¹H-NMR (DMSO-d6) δ in ppm: 1.29 (s, 3 H, -CH₃), 1.35 (s, 3 H, -CH₃), 2.96 (s, 4 H, -CH₂-CH₂-), 3.76 (q, 1 H, J₁ = 6.5, J₂ = 8.1, -CH₂O-), 3.99-4.12 (m, 3 H, -CH₂O-, -OCH₂-), 4.39 (quin, 1 H, -CH-), 4.83 (s, 2 H, -NH₂), 6.46 (s, 1 H, C¹-H), 6.53-6.63 (m, 2 H, C^{3'}-H, C^{6'}-H), 6.77 (d, 1 H, J = 6.99, C^{4'}-H), 6.92 (d, 1 H, J = 7.0, C^{5'}-H), 7.00-7.06 (m, 2 H, C³-H, C⁵-H), 7.20 (d, 1 H, J = 8.2, C⁹-H), 7.40 (s, 1 H, C⁶-H), 7.92-7.96 (m, 2 H, C⁴-H, -NH-).
¹³C-NMR (DMSO-d6) δ in ppm: 25.8 (-CH₃), 26.9 (-CH₃), 33.6 (C¹⁰), 36.6 (C¹¹), 66.1 (C³-Glyceryl), 69.2 (C¹-Glyceryl), 74.1 (C²-Glyceryl), 112.1 (C⁶), 113.1 (C³), 115.5 (C¹), 115.8 (C^{3'}), 116.6 (C^{4'}), 118.8 (C⁸), 125.2 (C^{5'}), 126.2 (C^{1'}), 126.2 (C^{1'}), 126.4 (C^{4a}), 130.4 (C⁹), 133.9 (C^{5a}), 134.8 (C⁴), 140.3 (C^{9a}), 144.0 (C^{2'}), 145.7 (C^{11a}), 151.1 (C²), 157.2 (C⁷), 190.2 (C⁵).

### Beispiel 36

### 2-(2, 4-Difluoranilino)-7-[2R-, 3-dihydroxypropoxy]-10,11-dihydro-dibenzo[a,d]-cyclohepten-5-on (14 e)

Zu einer Lösung von 1,0 g (2,1 mmol) 2-(2, 4-Difluoranilino)-7-(S-1,2-isopropylidenglycer-3-yl)-10,11-dihydrodibenzo[a,d]-cyclohepten-5-on. in 40 ml MeOH werden 10 ml H₂O und 0,25 g (1,3 mmol) p-Toluolsulfonsäure zugefügt. Die Lösung wird unter Argon-Schutzgasatmosphäre auf 50°C erhitzt. Nach 6 h wird die Lösung auf Raumtemperatur abgekühlt und im Vakuum eingeengt. Man erhält ein gelbes Öl, das in Ethylacetat und 5%iger Na₂HCO₃- Lösung (50 ml) wieder gelöst wird. Die organisch Phase wird abgetrennt und i. Vak. eingeengt. Das Diol fällt als weißer Feststoff aus. Der Feststoff wird in Hexan/ Ethylacetat (1:1) gelöst und 1 h lang gerührt. Es wird abfiltriert und i. Vak. eingeengt.
Ausbeute: 0,85 g (95%); Schmp.: 130,3 °C; C₂₄H₂₁F₂NO₄ (Mr = 425,44)
IR (ATR) 3305 (N-H), 2934 (C-H)ₐₗᵢₚₕ, 1629, 1607, 1569 (C=O), 1510, 1327, 1278, 1218, 1097,847,781.
¹H-NMR (DMSO-d6) δ in ppm: 3.32 (s, 4 H, -CH₂-CH₂), 3.40-3.46 (m, 2 H, -CH₂OH), 3.77-4.05 (m, 3 H, -CHOH-, -OCH₂-), 4.66 (t, 1 H, J = 5.7, -CH-OH), 4.94 (d, 1 H, J = 4.9, -CH₂-OH), 6.60 (s, 1 H, C¹-H), 6.73 (d, 1 H, J = 8.9, C³-H), 7.01-7.10 (m, 2 h, C^{3'}-H, C^{6'}-H), 7.22 (d, 1 H, J = 8.3, C^{4'}, -H), 7.30-7.47 (m, 3 H, C⁶-H, C⁸-H, C⁹-H), 7.94 (d, 1 H, J = 8.8, C⁴-H), 8.54 (s, 1 H, -NH-).
¹³C-NMR (DMSO-d6) δ in ppm: 33.6 (C¹¹), 36.3 (C¹⁰), 63.1 (-CH₂-OH), 70.2 (-O-CH₂-), 70.3 (-OCH-), 105.3 (d, J = 24.3, C^{3'}), 112.1 (d, J = 21.9, C^{5'}), 112.6 (C⁶), 113.8 (C³), 115.4 (C¹), 119.1 (C⁸), 125.3 (d, J = 12.0, C^{1'}), 126.4 (d, J = 9.8, C^{6'}), 127.7 (C^{4a}), 130.6 (C⁹), 133.8 (C^{5a}), 134.6 (C⁴), 139.9 (C^{9a}), 145.7 (C^{11a}), 149.4 (C²), 154.5 (d, J =152.8, C^{4'}), 157.6 (C⁷), 160.2 (d, J = 169.5, C^{2'}), 190.8 (C⁵).

### Beispiel 37

### 2-(2, 4-Difluoranilino)-7-[2S-, 3-dihydroxypropoxy]-10,11-dihydro-dibenzo[a,d]-cyclohepten-5-on (14 f)

Zu einer Lösung von 1,0 g (2,1 mmol) 2-(2, 4-Difluoranilino)-7-(R-1,2-isopropylidenglycer-3-yl)-10,11-dihydrodibenzo[a,d]-cyclohepten-5-on. in 40 ml MeOH werden 10 ml H₂O und 0,25 g (1,3 mmol) p-Toluolsulfonsäure zugefügt. Die Lösung wird unter Argon-Schutzgasatmosphäre auf 50°C erhitzt. Nach 6 h wird die Lösung auf Raumtemperatur abgekühlt und im Vakuum eingeengt. Man erhält ein gelbes Öl, das in Ethylacetat und 5%iger Na₂HCO₃- Lösung (50 ml) wieder gelöst wird. Die organisch Phase wird abgetrennt und im Vakuum eingeengt. Das Diol fällt als weißer Feststoff aus. Der Feststoff wird in Hexan/ Ethylacetat (1:1) gelöst und 1 h lang gerührt. Es wird abfiltriert und i. Vak. eingeengt.
Ausbeute: 95 %; C₂₄H₂₁F₂NO₄ (Mr = 425,44)
¹H-NMR (DMSO-d6) δ in ppm: 2.99 (s, 4 H, -CH₂-CH₂-), 3.41-3.46 (m, 2 H, -CH₂O-, -CH-), 3.77-4.03 (m, 3 H, -CH₂O-, CH₂OH), 4.65 (t, 1 H, J = 5.7, -OH), 4.93 (d, 1 H, J = 4.8, -OH), 6.60 (s, 1 H, C¹-H), 6.73 (d, 1 H, J= 8.6, C³-H), 7.01-7.41 (m, 6 H, C⁶-H, C⁸-H, C⁹-H, C^{3'}-H, C^{5'}-H, C^{6'}-H), 7.94 (d, 1 H, J = 8.7, C⁴-H), 8.53 (s, 1 H, -NH-).
¹³C-NMR (DMSO-d6) δ in ppm: 33.6 (C¹⁰), 36.3 (C¹¹), 63.1 (C³-Propoxy), 70.2 (C¹-Propoxy), 70.3 (C²-Propoxy), 105.3 (dd, 1 C, J₁ = 24.2, J₂ = 26.6, C^{3'}), 112.2 (dd, 1 C, J₁ = 3.6, J₂ = 22.0, C^{5'}), 112.6 (C⁶), 113.8 (C³), 115.4 (C¹), 119.1 (C⁸), 125.1-125.3 (m, 1 C, C^{1'}), 126.4-126.6 (m, 1 C, C^{6'}), 127.8 (C^{4a}), 130.6 (C⁹), 133.8 (C^{5a}), 134.6 (C⁴), 139.9 (C^{9a}), 145.7 (C^{11a}), 149.4 (C²), 153.4-156.5 (m, 1 C, C^{4'}), 157.3 (C⁷), 158.3-161.3 (m, 1 C, C^{2'}), 190.8 (C⁵).

### Beispiel 38

### 2-(2-Aminoanilino-7-[2R-, 3-dihydroxypropoxy] -10,11-dihydro-dibenzo[a,d]-cyclohepten-5-on (14 g)

Zu einer Lösung von 1,0 g (2,1 mmol) 2-(2-Aminoanilino)-7-(S-1,2-isopropylidenglycer-3-yl)-10,11-dihydrodibenzo[a,d]-cyclohepten-5-on in 40 ml MeOH werden 10 ml H₂O und 0,50 g (2,6 mmol) p-Toluolsulfonsäure zugefügt. Die Lösung wird unter Argon-Schutzgasatmosphäre auf 50°C erhitzt. Nach 6 h wird die Lösung auf Raumtemperatur abgekühlt und im Vakuum eingeengt. Man erhält ein gelbes Öl, das in Ethylacetat und 5%iger Na₂HCO₃- Lösung (50 ml) wieder gelöst wird. Die organisch Phase wird abgetrennt und i. Vak. eingeengt. Das Diol fällt als weißer Feststoff aus. Der Feststoff wird in Hexan/ Ethylacetat (1:1) gelöst und 1 h lang gerührt. Es wird abfiltriert und i. Vak. eingeengt.
Ausbeute: 96 %; C₂₄H₂₄N₂O₄ (Mr = 404,47)
¹H-NMR (DMSO-d6) δ in ppm: 2.96 (s, 4 H, -CH₂-CH₂-), 3.77-3.82 (m, 2 H, -OCH₂-, -CH-), 3.86-4.02 (m, 3 H, -OCH₂-, -CH₂O-), 4.65 (t, 1 H, J = 5.2, -OH), 4.83 (s, 2 H, -NH₂), 4.93 (d, 1 H, J = 4.5, -OH), 6.45 (s, 1 H, C¹-H), 6.54-6.62 (m, 2 H, C^{3'}-H, C^{6'}-H), 6.77 (d, 1 H, J = 7.2, C^{4'}-H), 6.90-7.03 (m, 3 H, C³-H, C⁸-H, C^{5'}-H), 7.20 (d, 1 H, J = 8.0, C⁹-H), 7.40 (s, 1 H, C⁶-H), 7.91-7.95 (m, 2 H, C⁴-H, -NH-).
¹³C-NMR (DMSO-d6) δ in ppm: 33.6 (C¹⁰), 36.6 (C¹¹), 63.1 (C³-Propoxy), 70.2 (C¹-Propoxy), 70.3 (C²-Propoxy), 112.1 (C⁶), 113.1 (C³), 115.5 (C¹), 115.8 (C^{3'}), 116.8 (C^{4'}), 118.9 (C⁸), 125.2 (C^{5'}), 126.2 (C^{6'}), 126.3 (C^{1'}), 126.4 (C^{4a}), 130.4 (C⁹), 134.0 (C^{5a}), 134.5 (C⁴), 140.2 (C^{9a}), 144.0 (C²), 145.8 (C^{11a}), 151.1 (C2), 157.6 (C⁷), 190.2 (C⁵).

### Beispiel 39

### 2-(2-Aminoanilino-7-[2S-, 3-dihydroxypropoxy]-10,11-dihydro-dibenzo[a,d]-cyclohepten-5-on (14 h)

Zu einer Lösung von 1,0 g (2,1 mmol) 2-(2-Aminoanilino)-7-(R-1,2-isopropylidenglycer-3-yl)-10,11-dihydrodibenzo[a,d]-cyclohepten-5-on in 40 ml MeOH werden 10 ml H₂O und 0,50 g (2,6 mmol) p-Toluolsulfonsäure zugefügt. Die Lösung wird unter Argon-Schutzgasatmosphäre auf 50°C erhitzt. Nach 6 h wird die Lösung auf Raumtemperatur abgekühlt und im Vakuum eingeengt. Man erhält ein gelbes Öl, das in Ethylacetat und 5%iger Na₂HCO₃- Lösung (50 ml) wieder gelöst wird. Die organisch Phase wird abgetrennt und i. Vak. eingeengt. Das Diol fällt als weißer Feststoff aus. Der Feststoff wird in Hexan/ Ethylacetat (1:1) gelöst und 1 h lang gerührt. Es wird abfiltriert und i. Vak. eingeengt.
Ausbeute: 95 %; C₂₄H₂₄N₂O₄ (Mr = 404,47)
¹H-NMR (DMSO-d6) δ in ppm: 2.96 (s, 4 H, -CH₂-CH₂-), 3.77-3.90 (m, 2 H, -CH₂-O-, -CH-), 3.98-4.03 (m, 2 H, CH₂OH, -CH₂O-), 4.66 (s, 1 H, -OH), 4.83 (s, 2 H, -NH₂), 4.93 (s, 1 H, - OH), 6.45 (s, 1 H, C¹-H), 6.53-6.63 (m, 2 H, C^{3'}-H, C^{6'}-H), 6.77 (d, 1 H, J = 7.4, C^{4'}-H), 6.90-7.03 (m, 3 H, C⁸-H, C³-H, C^{5'}-H), 7.20 (d, 1 H, J = 8.1, C⁹-H), 7.40 (s, 1 H, C⁶-H), 7.91-7.95, (m, 2 H, C⁴-H, -NH-).
¹³C-NMR (DMSO-d6) δ in ppm: 33.6 (C¹⁰), 36.6 (C¹¹), 65.3 (C³-Propoxy), 70.2 (C¹-Propoxy), 70.3 (C²-Propoxy), 112.1 (C⁶), 113.1 (C³), 115.5 (C¹), 115.8 (C^{3'}), 116.8 (C^{4'}), 118.9 (C⁸), 125.2 (C^{5'}), 126.2 (C^{6'}), 126.3 (C¹), 126.4 (C^{4a}), 130.4 (C⁹), 134.0 (C^{5a}), 134.5 (C⁴), 140.2 (C^{9a}), 144.0 (C^{2'}), 145.8 (C^{11a}), 151.1 (C²), 157.6 (C⁷), 190.2 (C⁵).

### Beispiel 40

### 2-(2, 4-Difluoranilino)-7-(2-hydroxy-ethoxy)-10,11-dihydro-dibenzo[a,d]-cyclohepten-5-on (14i)

Zur Herstellung der Verbindung nach Methode C werden 0,62 g (1,8 mmol) 2-Chlor-7-(2-acetoxyethoxy)-10,11-dihydrodbenzo[a,d]-cyclohepten-5-on, 0,25 g (1,9 mmol) 2, 4-Difluoranilin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70g (6,2 mmol) KOt-Bu, 5 ml Toluol und 1 ml t-BuOH verwendet. Ausbeute: 34 %; C₂₃H₁₉F₂NO₃ (Mr = 395,41)
¹H-NMR (DMSO-d6) δ in ppm: 2.99 (s, 4 H, -CH₂-CH₂-), 3.66-3.74 (m, 2 H, -CH₂-OH), 3.97-4.02 (m, 2 H, -OCH₂-), 4.85 (t, 1 H, J = 5.5, -OH), 6.60 (s, 1 H, C¹-H), 6.73 (d, 1 H, J = 8.6, C³-H), 7.01-7.42 (m, 6 H, C⁶-H, C⁸-H, C⁹-H, C^{3'}-H, C^{5'}-H, C^{6'}-H), 7.94 (d, 1 H, J = 8.8, C⁴-H), 8.53 (s, 1 H, -NH-).
¹³C-NMR (DMSO-d6) δ in ppm: 33.6 (C¹⁰), 36.2 (C¹¹), 59.9 (C²-Ethoxy), 70.0 (C¹-Ethoxy), 105.3 (t, 1 C, J = 24.2, C^{3'}), 112-0-112.4 (m, 1 C, C^{5'}), 112.6 (C⁶), 113.8 (C³), 115.4 (C¹), 119.1 (C⁸), 125.1-125.4 (m, 1 C, C^{1'}), 126.3-126.5 (m, 1 C, C^{6'}), 127.7 (C^{4a}), 130.6 (C⁹), 133.8 (C^{5a}), 134.6 (C⁴), 140.0 (C^{9a}), 145.7 (C^{11a}), 149.4 (C²), 153.4-156.5 (m, 1 C, C^{4'}), 149.4 (C⁷), 158.3-161.3 (m, 1 C, C^{2'}), 190.8 (C⁵).

### Beispiel 41

### 2-(2, 4-Difluoranilino)-7-(3-hydroxy-propoxy)-10,11-dihydro-dibenzo[a,d]-cyclohepten-5-on (14 j)

Zur Herstellung der Verbindung nach Methode C werden 0,65 g (1,8 mmol) 2-Chlor-7-(3-acetoxypropoxy)-10,11-dihydrodbenzo[a,d]-cyclohepten-5-on, 0,25 g (1,9 mmol) 2, 4-Difluoranilin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70g (6,2 mmol) KOt-Bu, 5 ml Toluol und 1 ml t-BuOH verwendet. Ausbeute: 40 %; C₂₄H₂₁F₂NO₃ (Mr = 409,44)
¹H-NMR (DMSO-d6) δ in ppm: 1.85 (quin, 2 H, J = 6.3, -CH₂-), 2.99 (s, 4 H, -CH₂-CH₂-), 3.55 (q, 2 H, J = 6.1, -CH₂-OH), 4.04 (t, 2 H, J = 6.4 -OCH₂-), 4.54 (t, 1 H, J = 5.1, -OH), 6.60 (s, 1 H, C¹-H), 6.73 (d, 1 H, J = 8.6, C³-H), 7.00-7.42 (m, 6 H, C⁶-H, C⁸-H, C⁹-H, C^{3'}-H, C^{5'}-H, C^{6'}-H), 7.94 (d, 1 H, J = 8.8, C⁴-H), 8.54 (s, 1 H, -NH-).
¹³C-NMR (DMSO-d6) δ in ppm: 32.5 (C²-Propoxy), 33.6 (C¹⁰), 36.2 (C¹¹), 57.6 (C³-Propoxy), 65.1 (C¹-Propoxy),105.3 (t, 1 C, J = 24.2, C³),11.9-112.4 (m, 1 C, C^{5'}), 112.6 (C⁶), 113.8 (C³), 115.3 (C¹), 119.1 (C⁸), 125.1-125.4 (m, 1 C, C^{1'}), 126.3-126.6 (m, 1 C, C^{6'}), 127.8 (C^{4a}), 130.6 (C⁹), 133.8 (C^{5a}), 134.5 (C⁴), 140.0 (C^{9a}), 145.7 (C^{11a}), 149.4 (C²), 153.4-156.5 (m, 1 C, C^{4'}), 157.5 (C⁷), 158.3-161.3 (m, 1 C, C^{2'}), 190.8 (C⁵).

### B. Substitution an Position 8 oder 9

### Methode H

### Bromierung der Methoxymethylbenzoesäure

Die Methoxymethylbenzoesäure und NBS werden in Chlorbenzol gegeben und die Reaktionsmischung auf 70°C erhitzt.

### Variante 1

Nach Zutropfen von AIBN/Chlorbenzol wird weitere 2 h 45 min bei 100°C gerührt. Dann wird die Lösung abgekühlt und das Chlorbenzol entfernt. Das Produkt wird ohne weitere Aufreinigung weiter verwendet. Das Succinimid wird in der folgenden Stufe entfernt.

### Variante 2

Nach Zutropfen von AIBN/Chlorbenzol wird weitere 1 h 30 min bei 70-75°C gerührt. Dann wird die Lösung abgekühlt und das Chlorbenzol entfernt. Das Produkt wird ohne weitere Aufreinigung weiter verwendet. Das Succinimid wird in der folgenden Stufe entfernt.

### Methode I

Wittig-Reaktion der Brommethylmethoxybenzoesäure mit 3-Chlorbenzaldehyd Triphenylphosphin wird in 150 ml MeOH gegeben, unter Rühren die Brommethylmethoxybenzoesäure (gelöst in MeOH) zugetropft und 2 Stunden unter Rückfluss erhitzt. Anschließend tropft man langsam die NaOMe-Lösung (30% in MeOH) zu. Nach beendigtem Zutropfen wird noch 15 min gewartet, dann wird der 3-Chlorbenzaldehyd zugegeben. Es folgen 6 h Refluxieren. Danach wird der Reaktionsansatz auf ein gerührtes Gemisch aus 75 g Eis und 175 ml Wasser gegossen. Im nächsten Schritt wird 3x mit je 200 ml Dichlormethan gewaschen. Anschließend wird die Wasserphase unter Eiskühlung mit konz. HCl stark angesäuert. Daraufhin bildet sich ein ölig-halbfester Niederschlag, der im Scheidetrichter abgetrennt werden kann. Das restliche Produkt wird mit Dichlormethan extrahiert.

### Methode J

### Reduktion der Chlorphenylvinylmethoxybenzoesäure

### Variante 1

Die Chlorphenylvinylmethoxybenzoesäure wird in Methanol gelöst und mit Salzsäure versetzt. Anschließend wird Pd / BaSO₄ (5 %) zugefügt. Nach Evakuieren wird mit 4 I H₂ gespült. Die Lösung wird unter H₂-Atmosphäre 2 Tage bei Raumtemperatur gerührt. Anschließend wird von der Kohle abfiltriert und im Vakuum eingeengt. Das entstehende Öl wird mit Dichlormethan in einen Scheidetrichter überführt und die organische Phase mit Wasser gewaschen. Nach Einengen erhält man ein hell-gelbes Öl erhält, das beim Stehen lassen kristallisiert.

### Variante 2

Die Chlorphenylvinylmethoxybenzoesäure wird im angegebenen Lösungsmittelgemisch gelöst. Anschließend wird Pd / C (10 %) zugefügt. Nach Evakuieren wird mit 4 Liter H₂ gespült. Die Lösung wird unter H₂-Atmosphäre (weiterer H₂-Ballon) 4 Stunden bei Raumtemperatur gerührt. Anschließend wird von der Kohle abfiltriert und im Vakuum eingeengt.

### Methode K

### Ringschluss mittels Friedel-Crafts Acylierung

Die Chlorphenylethylmethoxybenzoesäure wird in Dichlormethan (trocken) suspendiert und der Ansatz mit Argon gespült. Dann wird der Ansatz auf Rückflusstemperatur erhitzt und unter Rühren innerhalb einer Stunde der Lösung Thionylchlorid in Dichlormethan zugetropft. Anschließend wird noch eine weiter Stunde gerührt. Der Reaktionsansatz wird auf Raumtemperatur abgekühlt und innerhalb von 30 min wird wasserfreies AlCl₃ in zugegeben. Danach wird noch weitere 45 min gerührt und das Reaktionsgemisch dann unter Rühren auf eine Mischung aus 90 g Eis und 150 ml Wasser gegossen. Das Hydrolysegemisch wird ca. 30 min gerührt. Anschließend werden die beiden Phasen getrennt. Die organische Phase wird im Vakuum eingeengt

### Methode L

### Methoxyspaltung am Ringgerüst

Das Methoxydihydrodibenzosuberenon wird in Eisessig gelöst und HBr (48 %, wässrig) zugefügt. Es wird 3 h bei 120-130°C erhitzt.

### Variante 1

Nach Hydrolyse mit ca. 150 g Eis wird vom Niederschlag abfiltriert.

### Variante 2

Nach Hydrolyse mit ca. 150 g Eis wird vom Niederschlag abfiltriert und nach Waschen mit Wasser der Filterrückstand wieder in Dichlormethan gelöst. Anschließend wird eingeengt.

### Methode M

### Bildung des Acetals

Zu der Lösung des Dihydrodibenzosuberenons in DMF wird zuerst der Toluen-4-sulfonsäure-2,2-dimethyl-[1,3]dioxolan-4-ylmethylester und anschließend K₂CO₃ hinzugefügt. Die Reaktionsmischung wird unter Argon auf 80°C erwärmt und nach 24 h wieder auf Raumtemperatur abgekühlt. Anschließend wird der Ansatz in 50 ml Wasser aufgenommen und mit Ethylacetat extrahiert, die vereinigten organischen Phasen werden mit NaOH gewaschen und über Na₂SO₄ getrocknet. Dann wird i.Vak. eingeengt, wobei man ein braunes Öl erhält, das über Flash (SiO₂, Hexan/Ethylacetat) gereinigt wird. Man erhält das reine Acetal.

### Methode N

### Spaltung des Acetals

Zu einer Lösung des Acetals in MeOH wird H₂O und p-Toluensulfonsäure zugefügt. Die Lösung wird unter Schutzgas auf 50°C erhitzt. Nach 6 h wird die Lösung auf Raumtemperatur abgekühlt und i. Vak. eingeengt. Man erhält ein gelbes Öl, das in Ethylacetat und 5%iger Na₂HCO₃- Lösung (50 ml) wieder gelöst wird. Die organisch Phase wird abgetrennt und i. Vak. eingeengt.

### Methode O

### Einführung von Resten über Buchwald - Hartwig - Reaktion

Die Mischung aus Chlordihydrodibenzosuberenon, Pd(OAc)₂, Phosphinligand (2-(Dicyclohexylphosphino)-2',4',6'-triisopropylbiphenyl), NaOtert-Bu, Aminosubstituent, Toluen und tert-BuOH wird bei 100 °C 3-5 h gerührt. Danach wird abgekühlt, die Mischung in 150 ml Wasser aufgenommen und mit Diethylether extrahiert. Die vereinigten org. Phasen werden i. Vak. eingeengt und säulenchromatographisch (Flash; SiO₂; Hexan/Ethylacetat) gereinigt.

### Methode P

### Substitution mit Alkoholen

Unter Schutzgas wird eine Lösung aus Chlorhydroxydibenzodihydrodibenzosuberenon, Alkohol, Triphenylphosphin und THF hergestellt. Nach Zutropfen des Carboxylats wird 1h bei 0°C gerührt. Die Reaktion wird bis zur Beendigung bei Raumtemperatur fortgeführt. Danach wird i. Vak. eingeengt.

### Methode Q

### Substitution mit Alkylhalogeniden

Eine Mischung aus Phenol, Alkylhalogenid und K₂CO₃ wird im angegebenen Lösungsmittel auf 80°C erhitzt, 3 h gerührt, auf RT abgekühlt und wieder in EA und Wasser gelöst. Die org. Phase wird mit Wasser und Lauge gewaschen, über Na₂SO₄ getrocknet und i. Vakuum eingeengt.

### Methode R

### Herstellung des Triflats

Zu einer gerührten Lösung des Phenols in Pyridin wird bei 0 °C (Eisbad) unter Argon langsam Tf₂O über 0,5 h hinzugefügt. Die Reaktionsmischung wird bei Raumtemperatur über Nacht gerührt, mit EA extrahiert und die organische Phase mit 10%iger HCl und 5%iger NaHCO₃-Lösung gewaschen, über Na₂SO₄ getrocknet, abfiltriert und eingeengt.

### Substitution in Position 8 - Verbindungen und Vorstufen

### Beispiel 45

### 4-Methoxy-2-methylbenzoesäure (6)

Zur Synthese der Titelverbindung werden nach Methode H (Variante 2) 6,64 g (0,040 mol) 4-Methoxy-2-methylbenzoesäure, 7,1 g (0,040 mol) NBS und 0,25g AIBN in 150 ml Chlorbenzol umgesetzt. C₉H₁₀O₃ (Mr = 166,18)

### Beispiel 46

### 2-[2-(3-Chlorphenyl)-vinyl]-4-methoxybenzoesäure (7)

Zur Synthese der Titelverbindung werden nach Methode I 45,6 g ( 0,186 mol) 2-Bromomethyl-4-methoxybenzoesäure, 48,8 g (0,186 mol) Triphenylphosphin, 26,1 g (0,186 mol) 3-Chlorbenzaldehyd und 84,0 g (0,435 mol) NaOMe (28%) in 150 ml Methanol umgesetzt. Die Aufreinigung erfolgt durch Umkristallisation aus Methanol bei 4°C.
C₁₆H₁₃ClO₃ (Mr = 288,73)
¹H-NMR (DMSO-d6) δ in ppm: 3,86 (s, 3H, -OCH₃), 6,96 (d, 1H, J=8,78 Hz, C⁵-H), 7,12-7,59 (m, 6H, C³-H und C^{4'}-/C^{5'}-/C^{6'}-H und C¹-/C²-H Vinyl), 7,89 (d, 1 H, J=8,72 Hz, C^{2'}) 8,01-8,09 (m, 1H, C⁶).
¹³C-NMR (DMSO-d6) δ in ppm: 55,9 (-OCH₃), 112,1 (C³), 113,9 (C⁵), 122,0 (C¹), 125,5 (C^{6'}), 126,7 (C^{2'}), 127,9 (C^{4'}), 129,6 (C¹ Vinyl), 129,6 (C² Vinyl), 131,0 (C^{5'}), 133,3 (C⁶), 133,9 (C^{3'}), 139,9 (C^{1'}), 140,7 (C¹), 162,3 (C⁴), 168,2 (-COOH).

### Beispiel 47

### 2-[2-(3-Chlorphenyl)-ethyl]-4-methoxybenzoesäure (8)

Zur Synthese der Titelverbindung werden nach Methode J (Variante 2) 6,0 g 2-[2-(3-Chlorphenyl)-vinyl]-4-methoxybenzoesäure, 0,6 g Pd / C (10 %) und 4l Wasserstoff eingesetzt. Das Lösungsmittelgemisch besteht aus 75 ml Acetonitril, 75 ml Ethylacetat und 20 ml Methanol. C₁₆H₁₅ClO₃ (Mr = 290,75)

### Beispiel 48

### 2-Chlor-8-methoxy-10,11-dihydrodibenzo[a,d]cyclohepten-5-on (9)

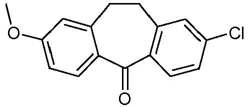

Zur Synthese der Titelverbindung werden nach Methode K 16,6 g (0,057 mol) [2-(3-Chlorphenyl)-ethyl]-4-methoxybenzoesäure, 7,25 g (0,06 mol) SOCl₂, 250 ml Dichlormethan, und 10 g AlCl₃ (0,075 mol) eingesetzt. Die Aufreinigung erfolgt säulenchromatographisch (Flash; SiO₂; Hexan 90% / Ethylacetat 10%). C₁₆H₁₃ClO₂ (Mr = 272,73)
MS m/z (%): 274/272 (34/100, M⁺), 259/257 (2/5, M⁺-CH₃), 246/244 (9/26, M⁺-CO), 237 (19, M⁺-Cl), 231/229 (5/17, 259/257-CO), 208 (17, 244-Cl), 194 (10, 231/229-Cl), 178 (14, 194-O), 165 (43, 4-Methoxy-2-methylbenzoesäure).
IR (ATR) 2921, 2852, 1595, 1266, 1203, 1185, 1110, 1031, 942, 912, 874, 839, 814, 769, 722, 596, 537 cm⁻¹.
¹H-NMR (DMSO-d6) δ in ppm: 3,11 (s, 4H, -CH₂-CH₂-), 3,82 (s, 3H, -OCH₃), 6,87-6,96 (m, 2H, C⁷-/C⁹-H), 7,37-7,46 (m, 2H, C¹-/C³-H), 7,87 (d, 1 H, J=8,37 Hz, C⁶-H), 7,99 (d, 1 H, 8,70 J=8,70 Hz, C⁴-H).
¹³C-NMR (DMSO-d6) δ in ppm: 34,0 (C¹¹), 35,1 (C¹⁰), 55,8 (-OCH₃), 113,1 (C⁷), 114,6 (C⁹), 126,9 (C³), 129,0 (C¹), 130,0 (C^{5a}), 132,7 (C⁶), 133,7 (C⁴), 137,1 (C^{4a}), 137,5 (C²), 144,5 (C^{9a}), 145,7 (C^{11a}), 163,1 (C⁸), 191, (C⁵).

### Beispiel 49

### 2-Chlor-8-hydroxy-10,11-dihydrodibenzo[a,d]cyclohepten-5-on (10)

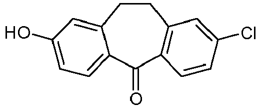

Zur Synthese der Titelverbindung werden nach Methode L 1,0 g (4,87 mmol) 2-Chlor-8-methoxy-10,11-dihydrodibenzo[a,d]cyclohepten-5-on, 10 ml HBr (48%, wässrig) und 10 ml Eisessig eingesetzt. C₁₅H₁₁ClO₂ (Mr = 258,71)

### Beispiel 50

### (S)-2-Chlor-8-(2,2-dimethyl-[1,3]dioxolan-4-ylmethoxy)-10,11-dihydro-dibenzo[a,d]cyclohepten-5-on (14)

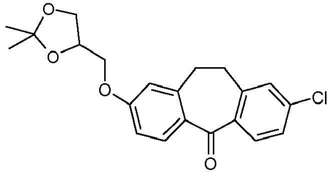

Zur Darstellung der Titelverbindung werden nach Methode M 0,50 g (1,9 mmol) 2-Chlor-8-hydroxy-10,11-dihydrodibenzo[a,d]cyclohepten-5-on, 0,75 g (2,6 mmol) (R)-Toluen-4-sulfonsäure-2,2-dimethyl-[1,3]dioxolan-4-ylmethylester und 0,80 g (5,8 mmol) K₂CO₃ in 10 ml trockenem DMF umgesetzt. C₂₁H₂₁ClO₄ (Mr = 372,85)

### Beispiel 51

### (S)-2-(2,4-Difluorophenylamino)-8-(2,2-dimethyl-[1,3]dioxolan-4-ylmethoxy)-10,11-dihydro-dibenzo[a,d]cyclohepten-5-on (12a)

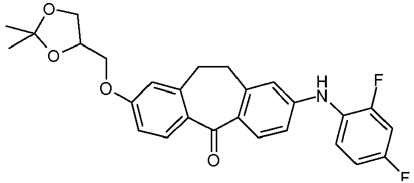

Zur Synthese der Titelverbindung werden nach Methode O 0,81 g (0,0022 mol) (S)-2-Chlor-1-(2,2-dimethyl-[1,3]dioxolan-4-ylmethoxy)-10,11-dihydrodibenzo[a,d]cyclohepten-5-on, 0,25 g (0,0019 mol) 2,4-Difluoranilin, 2 Spatelspitzen Pd(OAc)₂, 0,14 g Phosphinligand und 0,70g (0,0073 mol) NaOtert-Bu in 10 ml Toluen und 2 ml tert-BuOH umgesetzt. Die Aufreinigung erfolgt säulenchromatographisch (Flash; SiO₂; Hexan 80% / Ethylacetat 20%). C₂₇H₂₅F₂NO₄ (Mr = 465,50)

### Beispiel 52

### (R)-2-(2,4-Difluorophenylamino)-8-(2,3-dihydroxypropoxy)-10,11-dihydrodibenzo[a,d]cyclohepten-5-on (12c)

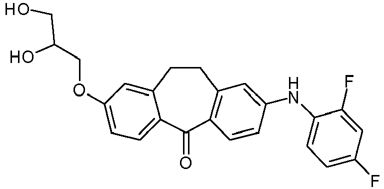

Zur Synthese der Titelverbindung werden nach Methode N 0,10 g (0,215 mmol) (S)-8-(2,4-Difluorophenylamino)-1-(2,2-dimethyl-[1,3]dioxolan-4-ylmethoxy)-10,11-dihydro-dibenzo[a,d]cyclohepten-5-on und 0,0054 g (0,0284 mmol) p-Toluensulfonsäure-Monohydrat in 6 ml Methanol und 1,5 ml Wasser umgesetzt. C₂₄H₂₁F₂NO₄ (Mr = 425,44)
¹H-NMR (DMSO-d6) δ in ppm: 2,95-3,09 (m, 4H, -CH₂-CH₂-), 3,42-3,45 (m, 2H, Dihydroxypropoxy), 3,76-3,81 (m, 1 H, Dihydroxypropoxy), 3,89-3,97 (m, 1 H, Dihydroxypropoxy), 4,04-4,11 (m, 1 H, Dihydroxypropoxy), 4,86 (s, 2H, C²-/C³-OH Dihydroxypropoxy), 6,61 (s, 1 H, C¹-H), 6,72 (d, 1 H, J=9,55 Hz, C³-H), 6,85-6,87 (m, 2H, C^{3'}-/C^{6'}-H), 7,05-7,12 (m, 1H, C^{5'}-H), 7,30-7,47 (m, 2H, C⁷-/C⁹-H), 7,95 (d, 2H, J=8,59 Hz, C⁶-/C⁴-H), 8,49 (s, 1H, -NH₂).

### Beispiel 53

### (S)-2-(2-Aminophenylamino)-8-(2,2-dimethyl-[1,3]dioxolan-4-ylmethoxy)-10,11-dihydrodibenzo[a,d]cyclohepten-5-on (12b)

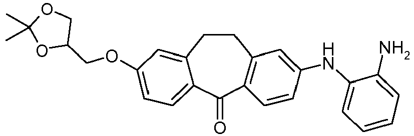

Zur Synthese der Titelverbindung werden nach Methode O 0,91 g (0,0024 mol) (S)-2-Chlor-8-(2,2-dimethyl-[1,3]dioxolan-4-ylmethoxy)-10,11-dihydrodibenzo[a,d]cyclohepten-5-on, 1,00 g (0,0092 mol) Phenylendiamin, 2 Spatelspitzen Pd(OAc)₂, 0,18 g Phosphinligand und 1,60 g (0,0166 mol) NaOtert-Bu in 10 ml Toluen und 2 ml tert-BuOH umgesetzt.
Die Aufreinigung erfolgt säulenchromatographisch (Flash; SiO₂; Hexan 70% / Ethylacetat 30%). C₂₇H₂₈N₂O₄ (Mr = 444,54)
¹H-NMR (DMSO-d6) δ in ppm: 1,30 (s, 3H, -CH₃), 1,35 (s, 3H, -CH₃), 2,92-3,05 (m, 4H, - CH₂-CH₂-), 3,71-3,78 (m, 1H, Dioxolan), 3,99-4,13 (m, 3H, Dioxolan), 4,38-4,44 (m, 1H, Dioxolan), 4,83 (s, 2H, -NH₂), 6,46-7,03 (m, 9H, C¹-/C³-/C⁴-/C⁷-/C⁹-H und C^{3'}-/C^{4'}-/C^{5'}-/C^{6'}-H), 7,91 (s, 1 H, C⁶-H), 7,96 (s, 1 H, -NH-).
¹³C-NMR (DMSO-d6) δ in ppm: 25,7 (-CH₃), 27,0 (-CH₃), 35,1 (C¹¹), 36,1 (C¹⁰), 66,0 (C³ Dioxolan), 69,2 (C¹ Dioxolan), 73,9 (C² Dioxolan), 109,3 (-C-(CH₃)₂-), 112,0 (C⁷), 112,9 (C⁹), 113,0 (C³), 114,3 (C¹), 115,8 (C^{3'}), 116,8 (C^{4'}), 125,3 (C^{4a}), 126,1 (C^{5'}), 126,3 (C^{6'}), 126,7 (C^{5a}) 131,8 (C^{1'}), 133,6 (C⁶), 133,9 (C⁴), 144,0 (C^{2'}), 145,0 (C^{9a}), 145,4 (C²), 150,8 (C^{11a}), 161,3 (C⁸), 188,7 (C⁵).

### Beispiel 54

### (R)-2-(2-Aminophenylamino)-8-(2,3-dihydroxypropoxy)-10,11-dihydro-dibenzo[a,d]cyclohepten-5-on (12d)

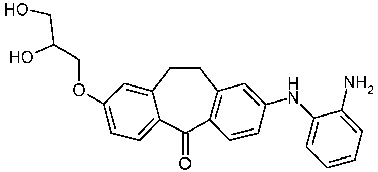

Zur Synthese der Titelverbindung werden nach Methode N 0,10 g (0,225 mmol) (S)-2-(2-Amino-phenylamino)-8-(2,2-dimethyl-[1,3]dioxolan-4-ylmethoxy)-10,11-dihydrodibenzo[a,d]cyclohepten-5-one und 0,0537 g (0,282 mmol) p-Toluensulfonsäure-Monohydrat in 6 ml Methanol und 1,5 ml Wasser umgesetzt. C₂₄H₂₄N₂O₄ (Mr = 404,47) ¹H-NMR (DMSO-*d6*) δ in ppm: 2,83-3,00 (m, 4H, -CH₂-CH₂-), 3,43 (d, 2H, J=4,80 Hz, C³-H Dihydroxypropoxy), 3,93-4,05 (m, 3H, Dihydroxypropoxy), 4,68 (s, 1 H, C³-OH), 4,83 (s, 2H, - NH₂), 4,96 (s, 1 H, C³-OH), 6,44 (s, 1 H, C^{6'}), 6,54-7,03 (m, 8H, C¹-/C³-/C⁴-/C⁷-/C⁹-H und C^{3'}-/C^{4'}-/C^{5'}-H), 7,89-7,96 (m, 2H, C⁶-H, -NH-)
¹³C-NMR (DMSO-*d6*) δ in ppm: 35,2 (C¹¹), 36,1 (C¹⁰), 63,0 (C³ Dihydroxypropoxy), 70,1 (C¹ Dihydroxypropoxy), 70,2 (C² Dihydroxypropoxy), 112,0 (C⁹), 112,8 (C⁷), 113,0 (C³), 114,3 (C¹), 115,8 (C³), 116,8 (C⁴), 125,4 (C^{4a}), 126,1 (C⁶), 126,3 (C^{5'}), 126,7 (C^{5a}), 131,5 (C^{1'}), 133,9 (C⁴), 144,0 (C^{2'}), 145,0 (C^{9a}), 145,4 (C²), 150,8 (C^{11a}), 161,8 (C⁸), 188,7 (C⁵)

### Beispiel 55

### 2-Chlor-8-(2-morpholin-4-ylethoxy)-10,11-dihydrodibenzo[a,d]cyclohepten-5-on (17)

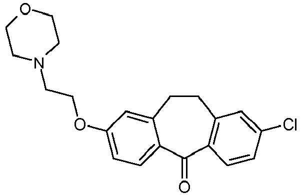

Zur Synthese der Titelverbindung werden nach Methode Q 0,44 g (1,69 mmol) 2-Chlor-8-hydroxy-10,11-dihydrodibenzo[a,d]cyclohepten-5-on, 0,345 g (1,86 mmol) 4-(2-Chlorethyl)morpholinhydrochlorid und 0,93 g (6,75 mmol) K₂CO₃ in 15 ml Acetonitril umgesetzt. C₂₁H₂₂ClNO₃ (Mr = 371,87)
¹H-NMR (DMSO-d6) δ in ppm: 2,47 (unter DMSO, 4H, C²-/C⁶-H Morpholin), 2,68 (t, 2H, J=5,73 Hz, C²-H Ethylmorphin), 3,10 (s, 4H, -CH₂-CH₂- Cyclohexan), 3,53-3,58 (m, 4H, C³-/C⁵-H Morpholin), 4,15 (t, 2H, J=5,69 Hz, C¹-H Ethylmorpholin), 6,88-6,96 (m, 2H, C⁷-/C⁹-H), 7,36-7,44 (m, 2H, C¹-/C³-H), 7,87 (d, 1 H, J=8,37 Hz, C⁶-H), 7,98 (d, 1 H, J=8,64 Hz, C⁴-H). ¹³C-NMR (DMSO-d6) δ in ppm: 34,0 (C¹¹), 35,1 (C¹⁰), 54,0 (2C, C²/C⁶ Morpholin), 57,2 (C² Ethylmorpholin), 66,0 (C¹ Ethylmorpholin), 66,5 (2C, C³/C⁵ Morpholin), 113,5 (C⁷), 115,2 (C⁹), 126,9 (C³), 129,0 (C¹), 130,0 (C^{5a}), 132,7 (C⁶), 133,7 (C⁴), 137,1 (C^{4a}), 137,5 (C²), 144,5 (C^{9a}), 145,7 (C^{11a}), 162,3 (C⁸), 191,0 (C⁵).

### Beispiel 56

### 2-(2,4-Difluorphenylamino)-8-(2-morpholin-4-yl-ethoxy)-10,11-dihydrodibenzo[a,d]cyclohepten-5-on (12e)

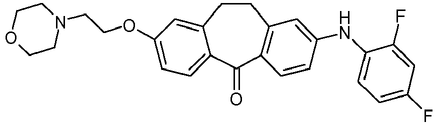

Zur Synthese der Titelverbindung werden nach Methode O 0,45 g (0,0013) mol 8-Chlor-1-(2-morpholin-4-yl-ethoxy)-10,11-dihydrodibenzo[a,d]cyclohepten-5-on, 0,20 g (0,0015 mol) 2,4-Difluoranilin, 2 Spatelspitzen Pd(OAc)₂, 0,14 g Phosphinligand und 0,70g (0,0073 mol) NaOtert-Bu in 10 ml Toluen und 2 ml tert-BuOH umgesetzt. Die Aufreinigung erfolgt säulenchromatographisch (Flash; SiO₂; Hexan 80% / Ethylacetat 20%).
C₂₇H₂₆F₂N₂O₂ (Mr = 464,52)
¹H-NMR (DMSO-d6) δ in ppm: 2,49 (unter DMSO, 4H, C²-/C⁶-H Morpholin), 2,69 (t, 2H, J=5,51 Hz, C²-H Ethylmorpholin), 2,96-3,07 (m, 4H, -CH₂-CH2- Cycloheptan), 3,56 (t, 4H, J=4,50 Hz, C³-/C⁵-H Morpholin), 4,15 (t, 2H, J=5,44 Hz, C¹-H Ethylmorpholin), 6,61 (s, 1 H, C¹-H), 6,73 (d, 1 H, J=8,43 Hz, C³-H), 6,86-6,91 (m, 2H, C^{3'}-/C^{6'}-H), 7,05-7,13 (m, 1 H, C^{5'}-H), 7,30-7,47 (m, 2H, C⁷-/C⁹-H), 7,95 (d, 2H, J=8,66 Hz, C⁴-/C⁶-H), 8,49 (s, 1H, -NH-).
¹³C-NMR (DMSO-d6) δ in ppm: 35,1 (s, C¹¹), 35,8 (s, C¹⁰), 53,9 (s, 2C, C²/C⁶ Morpholin), 57,3 (s, C² Ethylmorpholin), 65,9 (s, C¹, Ethylmorpholin), 66,5 (s, 2C, C³/C⁵ Morpholin), 105,3 (dd, J₁=25,5 Hz, J₂=24,2 Hz, C^{3'}), 112,2 (dd, J₁=21,9 Hz, J₂=3,8 Hz, C^{5'}), 112,5 (s, C⁷), 113,1 (s, C⁹), 113,6 (s, C⁹), 114,5 (s, C¹), 125,5 (dd, J₁=12,0 Hz, J₂=3,4 Hz, C^{1'}), 126,3 (dd, J₁=9,5 Hz, J₂=3,1 Hz, C^{6'}), 128,2 (s, C^{4a}), 131,3 (s, C^{5a}), 133,6 (s, C⁶), 133,8 (s, C⁴), 145,1 (s, C²), 145,3 (s, C^{11a}), 149,1 (s, C^{9a}), 154,9 (dd, J₁=141,3 Hz, J₂=11,0, C^{4'}), 159,7 (dd, J₁=129,7 Hz, J₂=12,0 Hz, C^{2'}), 161,6 (s, C⁸), 189,1 (s, C⁵).

### Substitution in Position 9 - Verbindungen und Vorstufen

Beispiel 57 2-Bromomethyl-3-methoxybenzoesäure (6) zur Synthese der Titelverbindung werden nach Methode H (Variante 1) 6,64 g (0,040 mol) 3-Methoxy-2-methylbenzoesäure, 7,1 g (0,040 mol) NBS und 0,25g AIBN in 150 ml Chlorbenzol umgesetzt.
C₉H₉BrO₃ (Mr = 245,07); Ausbeute: 55 %
¹H-NMR (DMSO-d6) 5 in ppm: 3,89 (s, 3H, -OCH₃), 5,03 (s, 2H, -CH₂-Br), 7,23-7,28 (m, 1H, C⁵-H), 7,41-7,46 (m, 2H, C⁴-/C⁵-H), 13,25 (s, 1H, -COOH).
¹³C-NMR (DMSO-d6) δ in ppm: 56,1 (-OCH₃), 68,4 (-CH₂Br), 116,3 (C⁴), 116,8 (C⁶), 126,9 (C²), 131,4 (C⁵), 135,3 (C¹), 154,3 (C³), 170,9 (-COOH).

### Beispiel 58

### 2-[2-(3-Chlorphenyl)-vinyl]-3-methoxybenzoesäure (7)

### Beispiel 58

### 2-[2-(3-Chlorphenyl)-vinyl]-3-methoxybenzoesäure (7)

Zur Synthese der Titelverbindung werden nach Methode I 45,6 g (0,186 mol) 2-Bromomethyl-3-methoxybenzoesäure, 48,8 g (0,186 mol) Triphenylphosphin, 26,1 g (0,186 mol) 3-Chlorbenzaldehyd und 84,0 g (0,435 mol) NaOMe (28%) in 150 ml Methanol umgesetzt. Die Aufreinigung erfolgt durch Umkristallisation aus Methanol mit Wasser.
C₁₆H₁₃ClO₃ (Mr = 288,73); Ausbeute: 51 %; Schmp.:119 °C.
IR (ATR) 2923, 2360, 2342, 1685, 1587, 1451, 1300, 1275, 1260, 1220, 1197, 178, 1049, 973, 964, 914, 889, 872, 810, 783, 761, 749, 729, 705, 684, 667, 621, 530, 457, 432 cm⁻¹.
¹H-NMR (DMSO-d6) δ in ppm: 3,86 (s, 3H, -OCH₃), 7,04-7,55 (m, 9H, Diphenylethen), 13,03 (s, 1H, -COOH).
¹³C-NMR (DMSO-d6) δ in ppm: 56,3 (O-CH₃), 114,2 (C⁴), 121,4 (C⁶), 124,8 (C²), 125,2 (C¹/C² Vinyl), 126,2 (C^{6'}), 127,6 (C^{2'}), 128,8 (C^{4'}), 130,9 (C⁵), 132,0 (C^{5'}), 133,9 (C¹), 134,0 (C^{3'}), 140,4 (C^{1'}), 157,9 (C³), 169,9 (-COOH).

### Z Beispiel 59

### 2-[2-(3-Chlorphenyl)-ethyl]-3-methoxybenzoesäure (8) Z

### Beispiel 59

### 2-[2-(3-Chlorphenyl)-ethyl]-3-methoxybenzoesäure (8)

Zur Synthese der Titelverbindung werden nach Methode J (Variante 1) 6,0 g 2-[2-(3-Chlorphenyl)-vinyl]-3-methoxybenzoesäure, 0,6 g Pd / BaSO₄ (5 %), 4 I Wasserstoff und 150 ml Methanol eingesetzt. Ausbeute: 87 %; Schmp.: 91 °C; C₁₆H₁₅ClO₃ (Mr = 290,75) IR (ATR) 2947, 2359, 2342, 1691, 1461, 1436, 1276, 1250, 1214, 1089, 1058, 1000, 782, 753, 696, 681 cm⁻¹.
¹H-NMR(DMSO-d6) δ in ppm: 2,74 (t, 2H, J=8,07 Hz, C¹ Ethyl), 3,11 (t, 2H, J=8,08, C² Ethyl), 3,79 (s, 3H, -OCH₃), 7,15 (d, 2H, C⁴-H und C^{6'}-H), 7,22-7,25 (m, 2H, C⁵-H und C^{5'}-H), 7,27 (s, 1 H, C^{2'}-H), 7,30- 7,37 (m, 2H, C⁶-H und C^{4'}-H).
¹³C-NMR (DMSO-d6) δ in ppm: 28,7 (C¹ Ethyl), 35,7 (C² Ethyl), 56,3 (-OCH₃), 114,2 (C⁴), 122,0 (C⁶), 126,1 (C^{6'}), 127,2 (C^{4'}), 128,3 (C^{2'}), 130,3 (C^{5'}), 130,4 (C¹), 132,6 (C²), 133,2 (C^{3'}), 145,0 (C^{1'}), 157,8 (C³), 169,4 (-COOH).

### Z Beispiel 60

### 8-Chlor-1-methoxy-10,11-dihydrodibenzo[a,d]cyclohepten-5-on (9)

### Beispiel 60

### 8-Chlor-methoxy-10,11-dihydrodibenzo[a,d]cyclohepten-5-on (9)

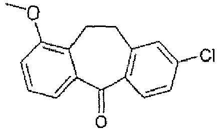

Zur Synthese der Titelverbindung werden nach Methode K 16,6 g (0,057 mol) [2-(3-Chlorphenyl)-ethyl]-3-methoxybenzoesäure, 7,25 g (0,06 mol) SOCl₂, 250 ml Dichlormethan, und 10 g AlCl₃ eingesetzt. Die Aufreinigung erfolgt säulenchromatographisch (Flash; SiO₂; Hexan 90% / Ethylacetat 10%).
C₁₆H₁₃ClO₂(Mr = 272,73); Ausbeute: 47 %; Schmp.:75 °C; GC 13,5 min
MS m/z (%): 274/272 (35/100, M⁺), 259/257 (6/18, M⁺-CH₃), 245/243 (5/15, M⁺-CO), 231/229 (3/11, 259/257-CO), 208 (17, 243-Cl), 194 (20, 231/229-Cl), 178 (23, 194-O), 165 (44, 3-Methoxy-2-methylbenzoesäure).
IR (ATR) 2946, 2901, 2359, 1659, 1589, 1575, 1312, 1284, 1254, 1228, 1184, 1087, 1062, 960, 862, 795, 754, 722, 706 cm⁻¹.
¹H-NMR (DMSO-d6) δ in ppm: 3,08 (s, 4H, -CH₂-CH₂-) 3,82 (s, 3H, -OCH₃), 7,18-7,42 (m, 5H, C²-/C³-/C⁴-/C⁷-/C⁹-H), 7,68 (d, 1H, J=8,28 Hz, C⁶-H).
¹³C-NMR (DMSO-d6) δ in ppm: 26,2 (C¹¹), 32,9 (C¹⁰), 56,4 (-OCH₃), 114,9 (C²), 121,7 (C⁴), 127,0 (C⁷), 127,6 (C³), 129,0 (C⁹), 130,3 (C^{5a}), 131,3 (C⁶), 137,1 (C^{4a}), 138,1 (C^{11a}), 139,4 (C⁸), 143,7 (C^{9a}), 156,7 (C¹), 195,2 (C⁵).

### Beispiel 61 8-Chlor-1-hydroxy-10,11-dihydrodibenzo[a,d]cyclohepten-5-on (10)

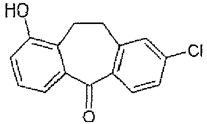

Zur Synthese der Titelverbindung werden nach Methode L 1,0 (4,87 mol) g 8-Chlor-1-methoxy-10,11-dihydrodibenzo[a,d]cyclohepten-5-on, 10 ml HBr (48%, wässrig) und 10 ml Eisessig eingesetzt. Ausbeute: 95 %; Schmp.: 185 °C; GC 15,2 min; C₁₅H₁₁ClO₂ (Mr = 258,71)
MS m/z (%): 260/258 (34/100, M⁺), 243/241 (2/7, M⁺-OH ), 232/230 (9/27, M⁺-CO), 223 (21, M⁺-Cl), 217/215 (2/7), 207/205 (2/6), 195 (28, 223-CO), 177 (12), 165 (39).
IR (ATR) 3251, 2360, 1637, 1573, 1307, 1282, 1241, 1221, 1176, 1159, 886, 759 cm⁻¹. ¹H-NMR (DMSO-d6) δ in ppm: 3,07 (s, 4H, -CH₂-CH₂-), 7,04 (d, 1 H, J=7,84 Hz, C²-H), 7,15 (t, 1H, J=7,78 Hz, C³-H), 7,28 (d, 1H, J=7,69 Hz, C⁷-H), 7,38 (d, 1H, J=8,31 Hz, C⁴-H), 7,45 (s, 1H, C⁹-H), 7,67 (d, 1H, J=8,32, C⁶-H), 9,84 (s, 1H, -OH).
¹³C-NMR (DMSO-d6) δ in ppm: 26,5 (C¹¹), 33,0 (C¹⁰), 119,1 (C²), 120,6 (C⁴), 126,9 (C⁷), 127,2 (C³), 128,7 (C⁹), 129,0 (C^{5a}), 131,3 (C⁶), 136,9 (C^{4a}), 138,4 (C^{11a}), 139,5 (C⁸), 143,8 (C^{9a}), 155,0 (C¹), 195,4 (C⁵).

### Beispiel 62

### 8-(2,4-Difluorphenylamino)-1-hydroxy-10,11-dihydro-dibenzo[a,d]cyclohepten-5-on (11 c)

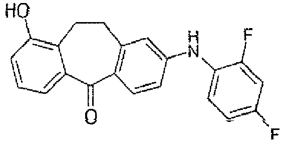

Zur Synthese der Titelverbindung werden nach Methode O 0,52 g (0,0020 mol) 8-Chlor-1-hydroxy-10,11-dihydro-dibenzo[a,d]cyclohepten-5-on, 0,26 g (0,0020 mol) 2,4-Difluoranilin, 2 Spatelspitzen Pd(OAc)₂, 0,14 g Phosphinligand und 0,90g (0,0094 mol) NaOtert-Bu in 10 ml Toluen und 2 ml tert-BuOH umgesetzt. Die Aufreinigung erfolgt Säulenchromatographisch (Flash; Kieselgel, 90% Hexan + 10% Ethylacetat). C₂₁H₁₅F₂NO₂(Mr =351,36); GC 35,3 min MS m/z (%): 351 (100, M+), 336 (4, M+-O), 323 (10, M+-CO), 308 (3, 323-O), 223 (2, 336-Difluorbenzen), 194 (12, 223-CO), 165 (13, 3-Methoxy-2-methylbenzoesäure), 129 (1, Difluoranilin), 115 (1, Difluoranilin).
¹H-NMR (DMSO-d6) δ in ppm: 2,93-3,06 (m, 4H, -CH₂-CH₂-), 6,61 (s, 1H, C⁹-H), 6,71 (d, 1H, J=8,04 Hz, C^{6'}-H), 6,98 (d, 1H, J=7,84 Hz, C⁷-H), 7,05-7,13 (m, 2H, C^{3'}-/C^{5'}-H), 7,24 (d, 1 H, J=7,62 Hz, C²-H), 7,34-7,39 (m, 2H, C³-/C⁴-H), 7,41-7,78 (m, 1 H, C⁶-H), 8,46 (s, 1 H, -NH-), 9,69 (s, 1H, -OH).

### Beispiel 63

### 8-(2,4-Difluorphenylamino)-1-methoxy-10,11-dihydrodibenzo[a,d]cyclohepten-5-on (11a)

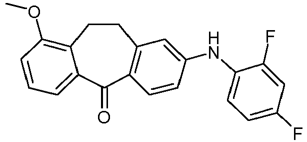

Zur Synthese der Titelverbindung werden nach Methode O 0,54 g (0,0020 mol) 8-Chlor-1-methoxy-10,11-dihydro-dibenzo[a,d]cyclohepten-5-on, 0,26 g (0,0020 mol) 2,4-Difluoranilin, 2 Spatelspitzen Pd(OAc)₂, 0,14 g Phosphinligand und 0,70g (0,0073 mol) NaOtert-Bu in 10 ml Toluen und 2 ml tert-BuOH umgesetzt. Die Aufreinigung erfolgt säulenchromatographisch (Flash; SiO₂; Hexan 90% / Ethylacetat 10%). C₂₂H₁₇F₂NO₂ (Mr = 365,38)
Ausbeute: g (%); Schmp.: 216 °C; GC 31,6 min
MS m/z (%): 365 (100, M⁺), 350 (6, M⁺-CH₃), 336(8), 322 (5, 350-CO), 306 (3, 322-O), 208 (11, M⁺-CO -NH-Phe), 194 (8, 208-CH₃), 178 (9, 194-O), 165 (17).
IR (ATR) 3276, 1299, 1286, 1269, 1255, 1193, 1073, 966, 863, 828, 764, 727, 603, 534, 496, 460, 446 cm⁻¹.
¹H-NMR (DMSO-d6) δ in ppm: 2,94-3,08 (m, 4H, -CH₂-CH₂-), 3,82 (s, 3H, -OCH₃), 6,61 (s, 1 H, C⁹-H), 6,72 (d, 1 H, J=8,72 Hz, C⁷-H), 7,03-7,17- (m, 2H, C^{3'}-/C^{6'}-H), 7,23-7,45- (m, 4H, C²-/C³-/C⁴-H und C^{5'}-H), 7,83 (d, 1 H, J=8,69, C⁶-H), 8,49 (s, 1 H, NH).
¹³C-NMR (DMSO-d6) δ in ppm: 25,0 (s, C¹¹), 35,1 (s, C¹⁰), 56,4 (s, -OCH₃), 105,5 (d, J=26,6 Hz, C^{3'}), 112,2 (d, J=25,6 Hz, C^{5'}), 112,6 (s, C⁷), 113,8 (s, C⁹), 114,3 (s, C²), 121,9 (s, C⁴), 125,4 (d, J=12,1 Hz, C^{1'}), 126,3 (d, J=9,6 Hz, C^{6'}), 127,3 (s, C³), 128,4 (s, C^{5a}), 130,1 (s, C^{4a}), 132,9 (s, C⁶), 141,2 (s, C^{11a}), 145,2 (s, C⁸), 149,3 (s, C^{9a}), 154,9 (d, J=141,2 Hz, C^{4'}), 155,8 (s, C¹), 159,8 (d, J=137,1 Hz, C^{2'}), 192,6 (s, C⁵).

### Beispiel 64

### 8-(2-Aminophenylamino)-1-methoxy-10,11-dihydrodibenzo[a,d]cyclohepten-5-on (11b)

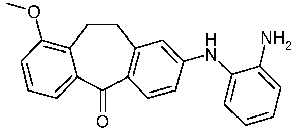

Zur Synthese der Titelverbindung werden nach Methode O 0,54 g (0,0020 mol) 8-Chlor-1-methoxy-10,11-dihydro-dibenzo[a,d]cyclohepten-5-on, 1,08 g (0,010 mol) Phenylendiamin, 2 Spatelspitzen Pd(OAc)₂, 0,14 g Phosphinligand und 1,60g (0,0166 mol) NaOtert-Bu in 10 ml Toluen und 2 ml tert-BuOH umgesetzt. Die Aufreinigung erfolgt säulenchromatographisch (Flash; SiO₂; Hexan 80% / Ethylacetat 20%). C₂₂H₂₀N₂O₂ (Mr = 344,42)
Ausbeute: 46 (%): Schmp.: 132 °C; GC 54,5 min
MS m/z (%): 344 (100, M⁺), 329 (13, M⁺-CH₃), 315 (6, M⁺-CO), 301 (5, 315-CH₂), 285 (5, 301-O), 195 (9, 301-1,2-Diaminobenzen), 182 (6), 165 (8, 3-Methoxy-2-methylbenzoesäure), 107 (13, 1,2-Diaminobenzen), 80 (7).
IR (ATR) 3356, 3282, 2852, 1616, 1587, 1563, 1553, 1497, 1335, 1293, 1272, 1255, 1222, 1192, 1075, 826, 759, 749, 706, 540, 505, 438 cm⁻¹.
¹H-NMR (DMSO-d6) δ in ppm: 2,93-3,02 (m, 4H, -CH₂-CH₂-), 3,81 (s, 3H, -OCH₃), 4,82 (s, 2H, -NH₂), 6,45 (s, 1 H, C⁹-H), 6,59 (d, 2H, J=8,22 Hz, C^{3'}-/C^{6'}-H), 6,76 (d, 1 H, J=7,66 Hz, C⁷-H), 6,89-7,02 (m, 2H, C^{4'}-/C^{5'}-H), 7,13 (d, 1H, J=7,47 Hz, C²-H), 7,21-7,37 (m, 2H, C³-/C⁴-H), 7,80-8,01 (m, 2H, -NH, C⁶-H).
¹³C-NMR (DMSO-d6) δ in ppm: 24,8 (C¹¹), 35,5 (C¹⁰), 56,4 (-OCH₃), 112,0 (C⁷), 113,0 (C^{3'}), 114,1 (C⁹), 115,8 (C²), 116,8 (C^{4'}), 122,0 (C^{5'}), 125,3 (C^{5a}), 126,1 (C^{6'}), 126,3 (C⁴), 126,8 (C^{4a}), 127,2 (C³), 130,0 (C^{11a}), 133,2 (C⁶), 141,5 (C^{1'}), 144,0 (C^{2'}), 145,4 (C^{9a}), 151,0 (C⁸), 155,6 (C¹), 191,9 (C⁵).

### Beispiel 65

### (S)-8-Chlor-1-(2,2-dimethyl-[1,3]dioxolan-4-ylmethoxy)-10,11-dihydrodibenzo[a,d]cyclohepten-5-on (13)

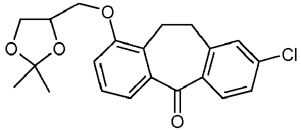

Zur Darstellung der Titelverbindung werden nach Methode M 0,50 g (1,9 mmol) 8-Chlor-1-hydroxy-10,11-dihydrodibenzo[a,d]cyclohepten-5-on, 0,75 g (2,6 mmol) (R)-Toluen-4-sulfonsäure-2,2-dimethyl-[1,3]dioxolan-4-ylmethylester und 0,80 g (5,8 mmol) K₂CO₃ in 10 ml trockenem DMF umgesetzt. Ausbeute: 53 %; C₂₁H₂₁ClO₄ (Mr = 372,85); GC 22,8 min MS m/z (%): 374/372 (35/100, M⁺), 359/357 (6/17, M⁺-CH₃), 337 (2, M⁺-Cl), 316/314 (10/26, 359/357-CH₃), 299/297 (16/39, 316/314-OH), 285/283 (3/8, 299/297-CH₂), 269 (283), 258/256 (15/6 M⁺-Dioxolan), 194 (12, 258/256-CI-CO), 178 (23, 194-CO), 165 (51, 3-Methoxy-2-methylbenzoesäure).
IR (ATR) 2962, 2359, 1650, 1586, 1449, 1370, 1310, 1284, 1257, 1213, 1177, 1156, 1057, 974, 873, 790, 761, 694, 665, 555 cm⁻¹.
¹H-NMR (DMSO-d6) δ in ppm: 1,31 (s, 3H, -CH₃), 1,36 (s, 3H, -CH₃), 3,11 (s, 4H, -CH₂-CH₂), 3,78-3,86 (m, 1H, Dioxolan), 4,00-4,11 (m, 3H, Dioxolan), 4,41-4,46 (m, 1H, Dioxolan), 7,25-7,44 (m, 4H, C²-/C³-/C⁴-/C⁷-H), 7,47 (s, 1 H, C⁹-H), 7,69 (d, 1 H, J=8,34 Hz, C⁶-H). ¹³C-NMR (DMSO-d6) δ in ppm: 25,7 (-CH₃), 26,3 (-CH₃), 26,9 (C¹¹), 32,8 (C¹⁰), 66,0 (C³ Dioxolan), 69,8 (C¹ Dioxolan), 74,1 (C² Dioxolan), 109,2 (-C(CH₃)₂), 116,3 (C²), 122,1 (C⁴), 127,0 (C⁷), 127,6 (C³), 129,1 (C⁹), 130,7 (C^{5a}), 131,3 (C⁶), 137,1 (C^{4a}), 138,2 (C^{11a}), 139,5 (C⁸), 143,7 (C^{9a}), 155,9 (C¹), 195,2 (C⁵).

### Beispiel 66

### (S)-8-(2,4-Difluorphenylamino)-1-(2,2-dimethyl-[1,3]dioxolan-4-ylmethoxy)-10,11-dihydrodibenzo[a,d]cyclohepten-5-on (11 d)

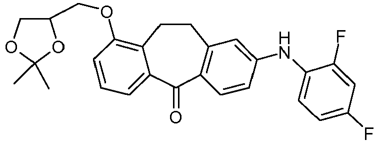

Zur Synthese der Titelverbindung werden nach Methode O 0,50 g (0,0013 mol (S)-8-Chlor-1-(2,2-dimethyl-[1,3]dioxolan-4-ylmethoxy)-10,11-dihydrodibenzo[a,d]cyclohepten-5-on, 0,17 g (0,0013 mol) 2,4-Difluoranilin, 2 Spatelspitzen Pd(OAc)₂, 0,14 g Phosphinligand und 0,70 g (0,0073 mol) NaOtert-Bu in 10 ml Toluen und 2 ml tert-BuOH umgesetzt.
Die Aufreinigung erfolgt säulenchromatographisch (Flash; SiO₂; Hexan 80% / Ethylacetat 20%). C₂₇H₂₅F₂NO₄ (Mr = 465,50); Ausbeute:37 %; Schmp.: 127 °C;
IR (ATR) 3307, 2981, 1503, 1287, 1257, 1207, 1139, 1062, 1041, 864, 843, 832, 810, 757, 541, 515 cm⁻¹.
¹H-NMR (DMSO-d6) δ in ppm: 1,31 (s, 3H, -CH₃), 1,36 (s, 3H, -CH₃), 2,96-3,11 (m, 4H, - CH₂-CH₂-), 3,78-3,85 (m, 1 H, Dioxolan), 4,04-4,14 (m, 3H, Dioxolan), 4,38-4,46 (m, 1 H, Dioxolan), 6,62-6,73 (m, 2H, C7-/C9-H), 7,08-7,43 (m, 6H, C²-/C³-/C⁴-H und C^{3'}-/C^{5'}-/C^{6'}-H), 7,81 (d, 1 H, J=8,72 Hz, C⁶), 8,49 (s, 1 H, -NH-).
¹³C-NMR (DMSO-d6) δ in ppm: 25,1 (s, C¹⁰), 25,7 (s, -CH₃), 26,9 (s, -CH₃), 35,0 (s, C¹¹), 66,0 (s, C³ Dioxolan), 69,9 (s, C¹ Dioxolan), 74,1 (s, C² Dioxolan), 105,0 (d, J=26,4 Hz, C^{3'}), 109,2 (s, -C(CH₃)₂), 112,2 (d, J=21,8 Hz, C^{5'}), 112,6 (s, C⁷), 113,9 (s, C⁹), 115,7 (s, C²), 122,3 (s, C⁴), 125,4 (d, J=15,8 Hz, C^{1'}), 126,2 (d, J=6,7 Hz, C^{6'}), 127,2 (s, C³), 128,5 (s, C^{5a}), 130,4 (s, C^{4a}), 132,9 (s, C⁶), 141,3 (s, C^{11a}), 145,1 (s, C⁸), 149,3 (s, C^{9a}), 154,9 (d, J=138,5 Hz, C^{4'}), 155,0 (s, C¹), 159,7 (d, J=148,2 Hz, C^{2'}), 192,6 (s, C⁵).

### Beispiel 67

### (R)-8-(2,4-Difluorphenylamino)-1-(2,3-dihydroxypropoxy)-10,11-dihydrodibenzo-[a,d]cyclohepten-5-on (11f)

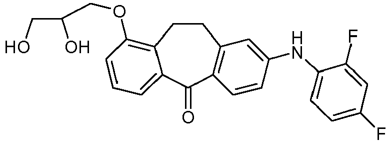

Zur Synthese der Titelverbindung werden nach Methode N 0,10 g (0,215 mmol) (S)-8-(2,4-Difluorophenylamino)-1-(2,2-dimethyl-[1,3]dioxolan-4-ylmethoxy)-10,11-dihydro-dibenzo[a,d]cyclohepten-5-on und 0,0054 g (0,0284 mmol) p-Toluensulfonsäure-Monohydrat in 6 ml Methanol und 1,5 ml Wasser umgesetzt. Ausbeute: 88 %; Schmp.: 125 °C C₂₄H₂₁F₂NO₄ (Mr = 425,44)
IR (ATR) 3307, 1541, 1305, 1257, 1217, 1138, 1116, 1098, 1061, 967, 847, 833, 812, 758, 601, 570, 539, 458 cm⁻¹.
¹H-NMR (DMSO-d6) δ in ppm: 3,00-3,08 (m, 4H, -CH₂-CH₂-), 3,46-3,49 (m, 2H, Dihydroxypropoxy), 3,78-4,03 (m, 3H, Dihydroxypropoxy), 6,63-6,73 (m, 2H, C⁷-/C⁹-H), 7,04-7,41 (m, 6H, C²-/C³-/C⁴-H und C^{3'}-/C^{5'}-/C^{6'}-H), 7,80 (d, 1 H, J=8,73 Hz, C⁶), 8,48 (s, 1 H, -NH).
¹³C-NMR (DMSO-d6) δ in ppm: 25,2 (s, C¹¹), 35,0 (s, C¹⁰), 63,1 (s, C³ Dihydroxypropoxy), 70,4 (s, C² Dihydroxypropoxy), 70,9 (s, C¹ Dihydroxypropoxy), 105,3 (t, J=25,3 Hz, C^{3'}), 112,1 (d, J=25,9 Hz, C^{5'}), 112,5 (s, C⁷), 113,9 (s, C⁹), 115,5 (s, C²), 121,9 (s, C⁴), 125,4 (d, J=15,6 Hz, C¹), 126,1 (d, J=9,6 Hz, C^{6'}), 127,2 (s, C³), 128,6 (s, C^{5a}), 130,5 (s, C^{4a}), 132,8 (s, C⁶), 141,2 (s, C^{11a}), 145,1 (s, C⁸), 149,2 (s, C^{9a}), 154,8 (d, J=141,5 Hz, C^{4'}), 155,5 (s, C¹), 159,7 (d, J=136,2 Hz, C^{2'}), 192,7 (s, C⁵).

### Beispiel 68

### (S)-8-(2-Aminophenylamino)-1-(2,2-dimethyl-[1,3]dioxolan-4-ylmethoxy)-10,11-dihydrodibenzo[a,d]cyclohepten-5-on (11 e)

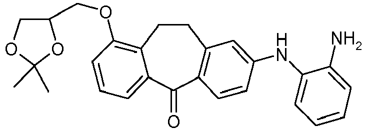

Zur Synthese der Titelverbindung werden nach Methode O 0,50 g (0,0013 mol) (S)-2-Chlor-8-(2,2-dimethyl-[1,3]dioxolan-4-ylmethoxy)-10,11-dihydrodibenzo[a,d]cyclohepten-5-on, 0,70 g (0,0065 mol) Phenylendiamin, 2 Spatelspitzen Pd(OAc)₂, 0,14 g Phosphinligand und 1,04 g (0,0108 mol) NaOtert-Bu in 10 ml Toluen und 2 ml tert-BuOH umgesetzt.
Die Aufreinigung erfolgt säulenchromatographisch (Flash; SiO₂; Hexan 70% / Ethylacetat 30%). Ausbeute: 23 %; Schmp.: < 35 °C; C₂₇H₂₈N₂O₄ (Mr = 444,54)
IR (ATR) 3347, 2927, 1566, 1499, 1450, 1254, 1212, 1155, 1069, 969, 908, 832, 745, 703, 599, 512, 448 cm⁻¹.
¹H-NMR (DMSO-d6) δ in ppm: 1,31 (s, 3H, -CH₃), 1,36 (s, 3H, -CH₃), 2,91-3,10 (m, 4H, - CH₂-CH₂-), 3,78-3,85 (m, 1 H, Dioxolan), 4,00-4,03 (m, 3H Dioxolan), 4,40-4,43 (m, 1 H, Dioxolan), 4,82 (s, 2H, -NH₂), 6,47- 6,62 (m, 3H, C⁹-H und C^{5'}-/C^{6'}-H), 6,78 (d, 1H, J=7,29 Hz, C^{3'}-H), 6,92 (d, 1 H, J=7,17 Hz, C⁷-H), 7,01 (d, 1 H, J=7,76 Hz, C²-H), 7,16-7,24 (m, 2H, C³-H und C^{4'}-H), 7,38 (d, 1H, J=7,55 Hz, C⁴-H), 7,82 (d, 1 H, J=8,74, C⁶-H), 7,91 (s, 1 H, -NH-).
¹³C-NMR (DMSO-d6) δ in ppm: 24,9 (C¹¹), 25,7 (-CH₃), 26,9 (-CH₃), 35,4 (C¹⁰), 66,0 (C³ Dioxolan), 69,9 (C¹ Dioxolan), 74,1 (C² Dioxolan), 109,2 (-C(CH₃)₂), 112,0 (C⁷), 113,1 (C⁹), 115,5 (C^{3'}), 11.5,8 (C²), 116,8 (C^{4'}), 122,4 (C^{5'}), 125,3 (C^{5a}), 126,0 (C^{6'}), 126,3 (C⁴), 126,9 (C³), 127,2 (C^{4a}), 130,4 (C^{11a}), 133,1 (C⁶), 141,6 (C^{1'}), 193,9 (C^{2'}), 145,3 (C⁸), 150,9 (C^{9a}), 154,9 (C¹), 191,9 (C⁵).

### Beispiel 69

### (R)-8-(2-Aminophenylamino)-1-(2,3-dihydroxypropoxy)-10,11-dihydrodibenzo[a,d]cyclohepten-5-on (11g)

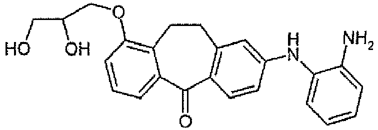

Zur Synthese der Titelverbindung werden nach Methode N 0,10 g (0,225 mmol) (S)-8-(2-Aminophenylamino)-1-(2,2-dimethyl-[1,3]dioxolan-4-ylmethoxy)-10,11-dihydrodibenzo[a,d]cyclohepten-5-on und 0,0537 g (0,282 mmol) p-Toluensulfonsäure-Monohydrat in 6 ml Methanol und 1,5 ml Wasser umgesetzt. C₂₄H₂₄N₂O₄ (Mr = 404,47); Ausbeute: 40% IR (ATR) 3305, 2919, 1566, 1498, 1449, 1255, 1216, 1189, 1155, 1111, 1043, 967, 908, 831, 749, 703, 448, 405 cm⁻¹.
¹H-NMR (DMSO-d6) δ in ppm: 2,93-3,11 (m, 4H, -CH₂-CH₂-), 3,47 (d, 2H, J=5,48 Hz, C³-H Dihydroxypropoxy), 3,83 (q, 1H, J=5,80 Hz, C²-H Dihydroxypropoxy), 3,85-4,06 (m, 2H, C¹-H Dihydroxypropoxy), 4,82 (s, 2H, -NH₂), 6,48 (s, 1 H, C⁹-H), 6,59 (d, 2H, J=7,39 Hz, C^{3'}-/C^{6'}-H), 6,76 (d, 1H, J=7,41 Hz, C⁷-H), 6,90-7,02 (m, 2H, C⁴-H und C^{5'}-H), 7,12 (d, 1H, J=7,37 Hz, C²-H), 7,23 (t, 1H, J=7,80 Hz, C³-H), 7,34 (d, 1H, J=7,09 Hz, C⁴-H), 7,80 (d, 1H, J=8,74 Hz, C⁶-H), 7,91 (s, 1H, -NH-).
¹³C-NMR (DMSO-d6) δ in ppm: 25,0 (C¹¹), 35,4 (C¹⁰), 63,1 (C³ Dihydroxypropoxy), 70,4 (C²Dihydroxypropoxy), 70,9 (C¹ Dihydroxypropoxy), 112,0 (C⁷), 113,2 (C⁹), 115,4 (C^{3'}), 115,8 (C²), 116,8 (C^{4'}), 122,0 (C^{6'}), 125,4 (C^{5a}), 126,0 (C^{5'}), 126,3 (C⁴), 127,0 (C³), 127,1 (C^{4a}), 130,5 (C^{11a}), 133,1 (C⁶), 141,5 (C^{1'}), 143,9 (C^{2'}), 145,3 (C⁷), 150,9 (C^{9a}), 155,3 (C¹), 192,1 (C⁵).

### Beispiel 72

### 8-Chlor-1-(2-morpholin-4-yl-ethoxy)-10,11-dihydrodibenzo[a,d]cyclohepten-5-on (16)

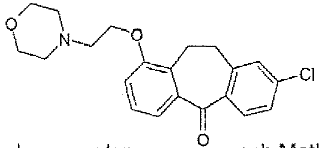

Zur Synthese der Titelverbindung werden nach Methode Q 0,44 g (1,69 mmol) 8-Chlor-1-hydroxy-10,11-dihydrodibenzo[a,d]cyclohepten-5-on, 0,345 g (1,86 mmol) 4-(2-Chlorethyl)morpholinhydrochlorid und 0,93 g (6,75 mmol) K₂CO₃ in 15 ml Acetonitril ungesetzt. C₂₁H₂₁ClNO₃ (Mr = 371,87); GC 29,7min
MS m/z (%): 373/371 (1/4, M⁺), 165 (4, 3-Methoxy-2-methyl-benzoesäure), 114 (1, Ethylmorpholin), 100 (100, 114-CH₂), 87 (1, 100-CH₂).
IR (ATR) 2854, 1585, 1451, 1282, 1252, 1115, 1090, 1062, 909, 860, 757 cm^{-1 1}H-NMR (DMSO-d6) δ in ppm: 2,50 (unter DMSO, 4H, C²-/C⁶-H Morpholin), 2,71 (t, 2H, J=5,61 Hz, C²-H Ethylmorpholin), 3,09 (s, 4H, -CH₂-CH₂- Cycloheptan), 3,47-3,58 (m, 4H, C³-/C⁵-H Morpholin), 4,12 (t, 2H, J=5,59 Hz, C¹-H Ethylmorpholin), 7,23-7,70 (m, 6H, C²-/C³-/C⁴-/C⁶-/C⁷-/C⁹-H).
¹³C-NMR (DMSO-d6) δ in ppm: 26,4 (C¹¹), 32,9 (C¹⁰), 53,9 (C²/C⁶ Morpholin), 57,3 (C² Ethylmorpholin), 66,6 (C³/C⁵ Morpholin), 67,0 (C¹ Ethylmorpholin), 116,3 (C²), 121,9 (C⁴), 127,0 (C⁷), 127,5 (C³), 129,1 (C⁹), 130,7 (C⁶), 131,3 (C^{5a}), 137,1 (C^{4a}), 138,1 (C^{11a}), 139,5 (C⁸), 143,7 (C^{9a}), 156,0 (C¹), 195,2 (C⁵).

### Beispiel 73

### 8-(2,4-Difluorphenylamino)-1-(2-morpholin-4-yl-ethoxy)-10,11-dihydrodibenzo[a,d]cyclohepten-5-on (11 j)

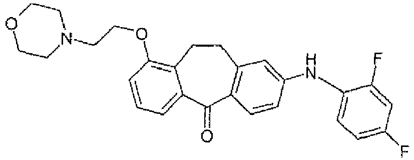

Zur Synthese der Titelverbindung werden nach Methode O 0,48 g (0,0013) mol 8-Chlor-1-(2-marpholin-4-yl-ethoxy)-10,11-dihydrodibenzo[a,d]cyclohepten-5-on, 0,17 g (0,0013 mol) 2,4-Difluoranilin, 2 Spatelspitzen Pd(OAc)₂, 0,14 g Phosphinligand und 0,70g (0,0073 mol) NaOtert-Bu in 10 ml Toluen und 2 ml tert-BuOH umgesetzt. Die Aufreinigung erfolgt säulenchromatographisch (Flash; SiO₂; Hexan 60% / Ethylacetat 40%).
C₂₇H₂₆F₂N₂O₃ (Mr = 464,52)
¹H-NMR (DMSO-d6) δ in ppm: 2,49 (unter DMSO, 4H C²-/C⁶-H Morpholin), 2,72 (t, 2H, J=5,62 Hz, C²-H Ethylmorpholin), 2,99-3,10 (m, 4H, -CH₂-CH₂- Cycloheptan), 3,56 (t, 4H, J=4,58 Hz, C³-/C⁵-H Morpholin), 4,12 (t, 2H, J=5,62 Hz, C¹-H Ethylmorpholin), 6,62 (s, 1H, C⁹-H), 6,71 (d, 1 H, J=8,58 Hz, C⁷-H), 7,08-7,41 (m, 6H, C²-/C³-/C⁴-H und C^{3'}-/C^{5'}-/C^{6'}-H), 7,82 (d, 1H, J=8,87 Hz, C⁶-H), 8,48 (s, 1H, -NH-).
¹³C-NMR (DMSO-d6) δ in ppm): 25,2 (s, C¹¹), 35,1 (s, C¹⁰), 53,9 (s, 2C, C²/C⁶ Morpholin), 57,4 (s, C² Ethylmorpholin), 66,6 (s, 2C, C³/C⁵ Morpholin), 67,1 (s, C¹ Ethylmorpholin), 105,3 (dd, J₁=26,6 Hz, J₂= 24,1 Hz, C^{3'}), 112,1 (dd, J₁=21,8 Hz, J₂=3,7 Hz, C^{5'}), 112,6 (s, C⁷), 113,9 (s, C⁹), 115,8 (s, C²), 122,1 (s, C⁴), 125,4 (dd, J₁=12,1 Hz, J₂=3,6 Hz, C^{1'}), 126,2 (dd, J₁=9,6 Hz, J₂=3,2 Hz, C^{6'}), 127,2 (s, C^{5a}), 128,5 (s, C³), 130,5 (s, C^{4a}), 132,9 (s, C⁶), 141,3 (s, C^{11a}), 145,1 (s, C⁸), 149,2 (s, C^{9a}), 154,8 (dd, J₁=140,7 Hz, J₂=12,6 Hz, C^{4'}), 155,1 (s, C¹), 159,7 (dd, J₁=136,5 Hz, J₂=11,8 Hz, C^{2'}), 196,6 (s, C⁵).

### Beispiel 74

### Trifluormethansulfonsäure-8-chlor-5-oxo-10,11-dihydro-5H-dibenzo[a,d]cyclohepten-1-ylester

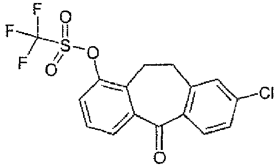

Zur Herstellung der Titelverbindung werden nach Methode R 0,517 g (0,0020 mol) 8-Chlor-1-hydroxy-10,11-dihydrodibenzo[a,d]cyclohepten-5-on und 0,846 g (0,0030 mol) Tf₂O in 5 ml Pyridin umgesetzt. C₁₆H₁₀ClF₃O₄S (Mr = 390,77); GC 11,9 min
MS m/z (%): 392/390 (39/100, M⁺), 364/362 (9/24, M⁺-CO), 259/257 (17/50, M⁺-SO₂CF₃), 231/229 (10/31, 259/257-CO), 194 (54, 231/229-Cl).

### Verbindungen Position 8

| | |
|---|---|
| 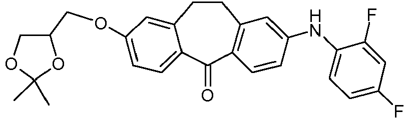 | 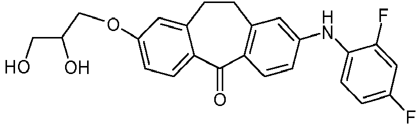 |
| 2-(2,4-Difluoro-phenylamino)-8-(2,2 -dimethyl-[1,3]dioxolan-4-ylmethoxy )-10,11-dihydro-dibenzo[a,d]cyclohe pten-5-one | 2-(2,4-Difluoro-phenylamino)-8-(2,3 -dihydroxy-propoxy)-10,11-dihydro-d ibenzo[a,d]cyclohepten-5-one |
| 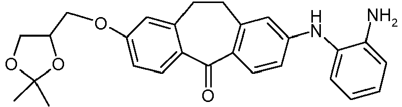 | 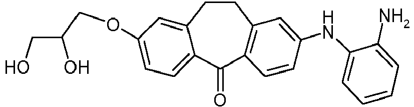 |
| 2-(2-Amino-phenylamino)-8-(2,2-dime thyl-[1,3]dioxolan-4-ylmethoxy)-10, 11-dihydro-dibenzo[a,d]cyclohepten-5-one | 2-(2-Amino-phenylamino)-8-(2,3-dihy droxy-propoxy)-10,11-dihydro-dibenz o[a,d]cyclohepten-5-one |
| 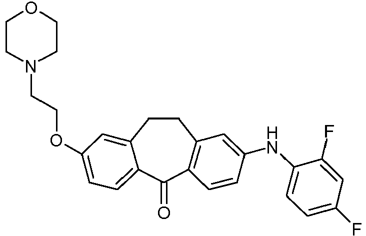 | |
| 2-(2,4-Difluorphenylamino)-8-(2-m orpholin-4-yl-ethoxy)-10,11-dihydro dibenzo[a,d]cyclohepten-5-on | |

### Verbindungen Position 9

| | |
|---|---|
| | 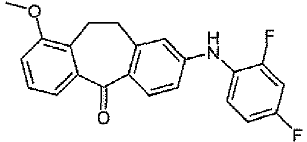 |
| | 8-(2,4-Difluorphenylamino)-1-meth oxy-10,11-dihydrodibenzo[a,d]cyclo hepten-5-on |
| 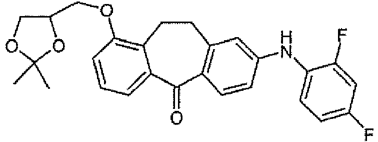 | 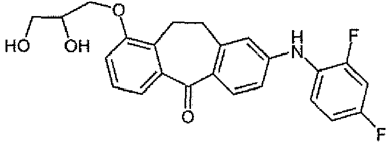 |
| (S)-8-(2,4-Difluorophenylamino)-1-(2,2 -dimethyl-[1,3]dioxolan-4-ylmethoxy )-10,11-dihydrodibenzo[a,d]cyclohe pten-5-on | (R)-8-(2,4-Difluorphenylamino)-1-(2,3 -dihydroxy-propoxy)-10,11-dihydrod ibenzo[a,d]cyclohepten-5-on |
| | 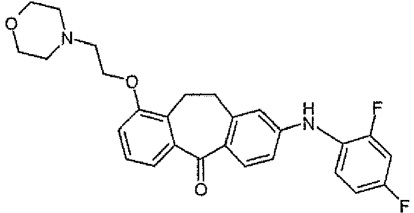 |
| | 8-(2,4-Difluorphenylamino)-1-(2-m orpholin-4-yl-ethoxy)-10,11-dihydro dibenzo[a,d]cyclohepten-5-on |
| 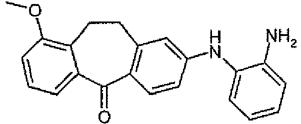 | |
| 8-(2-Aminophenylamino)-1-methoxy-1 0,11-dihydrodibenzo[a,d]cyclohepte n-5-on | |
| 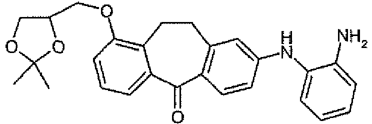 | 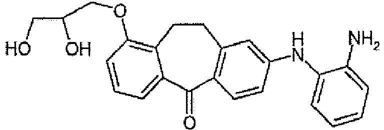 |
| (S)-8-(2-Aminophenylamino)-1-(2,2-dime thyl-[1,3]dioxolan-4-ylmethoxy)-10, 11-dihydrodibenzo[a,d]cyclohepten-5-on | (R)-8-(2-Aminophenylamino)-1-(2,3-dihy droxy-propoxy)-10,11-dihydrodibenz o[a,d]cyclohepten-5-on |

### Allgemeine Methode AA

Zur Herstellung der Testverbindungen wird eine Mischung aus Arylhalogenid, Anilinderivat, Pd(OAc)₂, Phosphinligand, Na-O-tert-Butylat bzw. K-O-tert-Butylat, Toluol und tert-BuOH unter Argon-Schutzgasatmosphäre auf 100 °C erhitzt und die angegebene Zeit bei dieser Temperatur gerührt. Danach wird auf Raumtemperatur abgekühlt, mit 150 ml H₂O hydrolysiert, mit je 3x 200 ml Diethylether extrahiert, die organische Phase filtriert und im Vakuum eingeengt. Die Aufreinigung erfolgt über Flash-Chromatographie.

### Allgemeine Methode AB

Zur Hydrolyse des Acetals in MeOH wird die angegebene Menge H₂O und p-Toluolsulfonsäure zugefügt. Die Lösung wird unter Argon-Schutzgasatmosphäre auf 50 °C erhitzt. Nach 6 h wird die Lösung auf Raumtemperatur abgekühlt und im Vakuum eingeengt. Man erhält ein gelbes Öl, das in Ethylacetat und 5%iger Na₂HCO₃- Lösung (50 ml) wieder gelöst wird. Die organische Phase wird abgetrennt und im Vakuum eingeengt. Das Diol fällt in Form eines weißen Feststoffs aus, der aus Dichlormethan/Hexan umkristallisiert wird.

### Allgemeine Methode AC

Zur Herstellung des sekundären Amins wird in einem trockenen 100 ml Dreihalskolben mit Rückflusskühler und Blasenzähler unter Argonatmosphäre die angegebenen Mengen der Aminokomponente im Toluol - tert-Butanol Gemisch unter Erwärmen gelöst. Nun werden die angegebenen Mengen des Phosphinliganden (2-(Dicyclohexylphosphino)-2',4',6'-triisopropylbiphenyl, K-O-tert-Butylat, Halogenkomponente und Pd(OAc)₂ hinzugefügt und die Mischung unter Argon Atmosphäre bei 110 °C unter DC Kontrolle für 2-6 h refluxiert. Nach Beendigung der Reaktion wird auf RT abgekühlt und abfiltriert. Der Filterrückstand wird mehrmals mit Dichlormethan, Methanol und Ethylacetat gewaschen und die vereinigten organischen Phasen werden unter Vakuum eingeengt. Das dabei zurückbleibende braune Produktgemisch wird chromatographisch mit Hexan:Ethylacetat (3:1) über Kieselgel aufgereinigt.

### Beispiel 107

### 2-(2,4-Difluorphenylamino)-7-(3-morpholin-4-yl-propoxy)-10,11-dihydro-dibenzo¬[a,d]-cyclohepten-5-on (55k)

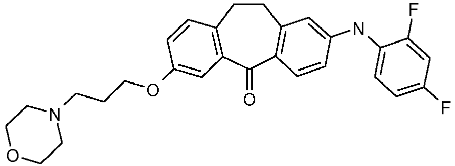

Nach der allgemeinen Methode AA werden 0,67 g (1,8 mmol) 2-Chlor-7-(3-morpholin-4-yl-propoxy-10,11-dihydrodibenzo[a,d]-cyclohepten-5-on (36i), 0,25 g (1,9 mmol) 2,4-Difluoranilin, 0,05 g (0,22 mmol) Pd(OAc)₂, 0,10 g (0,21 mmol) 2-(Dicyclohexylphosphino)-2'-,4'-,6'-triisopropyl-biphenyl, 0,60 g (6,2 mmol) Na-O-tert-Butylat, 5 ml Toluol und 1 ml tert-BuOH verwendet. Reaktionszeit: 1 h. Die Aufreinigung erfolgt über Flash-Chromatographie (SiO₂, Hexan/Ethylacetat 7+3). C₂₈H₂₈F₂N₂O₃ (Mr = 478,54); Ausbeute 43%; Schmp. 114 °C IR (ATR)* 3313 (N-H), 2852, 1567, 1527, 1496, 1270, 1196, 1146, 1116, 1093, 961, 863, 816 cm⁻¹
¹H-NMR (DMSO-d6) δ in ppm: 1.79-1.92 (m, 2 H, C2Propoxy-H), 2.34-2.43 (m, 6 H, C3/5Morpholinyl-H, C3Propoxy-H), 2.99 (s, 4 H, -CH2-CH2-), 3.55 (t, 4 H, J = 3.6 Hz, C2/6Morpholinyl-H), 4.01 (t, 2 H, J = 5.8 Hz, C1Propoxy-H); 6.60 (s, 1 H, C1-H), 6.73 (d, 1 H, J = 8.7 Hz, C3-H), 7.00-7.38 (m, 6 H, C6-H, C8-H, C9-H, C3'-H, C5'-H, C6'-H), 7.94 (d, 1 H, J = 8.6 Hz, C4-H), 8.53 (s, 1 H, -NH-)
¹³C-NMR (DMSO-d6) δ in ppm: 26.2 (C2Propoxy), 33.5 (C10), 36.2 (C11), 53.7 (2 C, C3/5Morpholinyl), 55.1 (C3Propoxy), 65.3 (C1Propoxy), 66.5 (2 C, C2/6Morpholinyl), 105.3 (dd, 1 C, J1 = 24.1 Hz, J2 = 26.8 Hz, C3'), 112.2 (dd, 1 C, J1 = 3.8 Hz, J2 = 22.4 Hz, C5'), 112.6 (C6), 113.8 (C3), 115.2 (C1), 119.2 (C8), 125.2 (dd, 1 C,J1 = 4.2 Hz, J2 = 11.9 Hz, C1'), 126.4 (dd, 1 C, J1 = 3.4 Hz, J2 = 9.8 Hz, C6'), 127.7 (C4a), 130.6 (C9), 133.8 (C5a), 134.5 (C4), 140.0 (C9a), 145.7 (C11a), 149.4 (C2), 156.0 (dd, 1 C, J1 = 12.7 Hz, J2 = 248.3 Hz, C4'), 158.3 (C7), 158.8 (dd, 1 C, J1 = 12.2 Hz, J2 = 242.2 Hz, C2'), 190.8 (C5)

### Beispiel 108

### 2-(2,4-Difluorphenylamino)-8-(3-hydroxypropoxy)-10,11-dihydrodibenzo[a,d]-cyclohepten-5-on (12j)

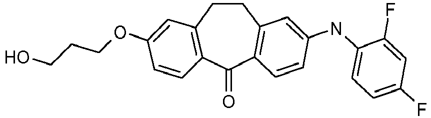

Nach der allgemeinen Methode O werden 0,65 g (1,8 mmol) Essigsäure-3-(8-chlor-5-oxo-10,11-dihydro-5H-dibenzo[a,d]cyclohepten-2-yloxy)-propylester, 0,25 g (1,9 mmol) 2,4-Difluoranilin, 0,04 g Pd(OAc)₂, 0,17 g Phosphinligand und 0,74 g (7,7 mmol) Na-O-tert-Butylat in 10 ml Toluen und 2 ml tert-BuOH umgesetzt. Ausbeute: 42 %; Schmp.: 154 °C C₂₄H₂₁F₂NO₃ (Mr = 409,44); HPLC 8,7 min; 98,3%
IR (ATR) (cm⁻¹): 3359, 2918, 1605, 1581, 1529, 1270, 1240, 1118, 1098, 1055, 960, 843,772,692,455.
¹H-NMR (DMSO-d6) δ in ppm: 1.86 (q, 2H, J = 6.28 Hz, C2-H Hydroxy-propoxy), 3.01 (dd, 4H, J1 = 12.45 Hz, J2 = 9.23Hz, -CH2-CH2-), 3.55 (dd, 2H, J1 = 11.44 Hz, J2 = 5.97 Hz, C3-H Hydroxypropoxy), 4.11 (t, 2H, J = 6.30 Hz, C1-H Hydroxypropoxy), 4.56 (t, 1 H, J = 5.11 Hz, -OH), 6.61 (s, 1 H, C1-H), 6.73 (d, 1 H, J = 8.90 Hz, C3-H), 6.84-6.91 (m, 2H, C3'-/C6'-H), 7.03-7.14 (m, 1 H, C5'-H), 7.29-7.42 (m, 2H, C7-/C9-H), 7.96 (d, 2H, J = 8.71 Hz, C4-/C6-H), 8.49 (s, 1H, -NH-).
¹³C-NMR (DMSO-d6) δ in ppm: 32.4 (C2 Hydroxypropoxy), 35.2 (C11), 35.8 (C10), 57.5 (C3 Hydroxypropoxy), 65.2 (C1 Hydroxypropoxy), 105.3 (dd, J1 = 26.6 Hz, J2 = 23.9 Hz, C3'), 112.1 (dd, J1 = 26.6 Hz, J2 = 23.9 Hz, C5'), 112.5 (C7), 113.0 (C9), 113.6 (C3), 114.4 (C1), 125.4 (dd, J1 = 12.0 Hz, J2 = 3.6 Hz, C1'), 126.2 (dd, J1 = 9.6 Hz, J2 = 3.3 Hz, C6'), 128.3 (C4a), 131.2 (C5a), 133.7 (C6), 133.8 (C4), 145.2 (C9a), 145.3 (C2), 149.1 (C11a), 155.9 (dd, J1 = 246.3 Hz, J2 = 12.4 Hz, C4'), 158.6 (dd, J1 = 234.8 Hz, J2 = 11.8 Hz, C2'), 161.9 (C8), 189.1 (C5).

### Beispiel 109

### 2-(2,4-Difluorphenylamino)-8-(2-hydroxyethoxy)-10,11-dihydrodibenzo[a,d]-cyclohepten-5-on (12k)

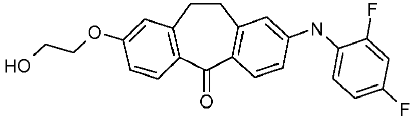

Nach der allgemeinen Methode O werden 0,67 g (0,19 mmol) Essigsäure-2-(8-chlor-5-oxo-10,11-dihydro-5H-dibenzo[a,d]cyclohepten-2-yloxy)-ethylester, 0,26 g (0,0020 mol) 2,4-Difluoranilin, 0,04 g Pd(OAc)₂, 0,14 g Phosphinligand und 0,70g (7,3 mmol) Na-O-tert-Butylat in 10 ml Toluol und 2 ml tert-BuOH umgesetzt. Ausbeute: 20 %; Schmp.: 138 °C C₂₃H₁₉F₂NO₃ (Mr = 395,41); HPLC 8,1 min; 98,1 %
IR (ATR) (cm⁻¹): 3406, 3283, 2919, 1596, 1535, 1495, 1343, 1297, 1265, 1239, 1210, 1186, 1108, 1093, 1077, 1042, 959, 857, 847, 781, 655, 497, 455.
¹H-NMR (DMSO-d6) δ in ppm: 2.97-3.07 (m, 4H, -CH2-CH2-), 3.68-3.76 (m, 2H, C2-H Hydroxyethoxy), 4.06 (t, 2H, J = 4.78 Hz, C1-H Hydroxyethoxy), 4.89 (t, 1 H, J = 5.48 Hz, -OH), 6.61 (s, 1H, C1-H), 6.73 (d, 1H, J = 8.53 Hz, C3), 6.85-6.92 (m, 2H, C3'-/C6'-H), 7.05-7.13 (m, 1 H, C5'-H), 7.29-7.43 (m, 2H, J = 8.71 Hz, C4-/C6-H), 8.47 (s, 1 H, -NH-).

### Beispiel 110

### 2-(2,4-Difluorphenylamino)-8-methoxy-10,11-dihydro-dibenzo[a,d]cyclo-hepten-5-on (12a)

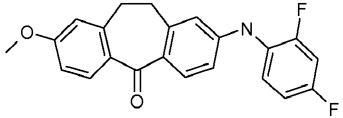

Nach der allgemeinen Methode O werden 0,52 g (1,9 mmol) 2-Chlor-8-methoxy-10,11-dihydro-dibenzo[a,d] cyclohepten-5-on, 0,30 g (2,3 mmol) 2,4-Difluoranilin, 0,04 g Pd(OAc)₂, 0,16 g Phosphinligand und 0,86g (8,9 mmol) Na-O-tert-Butylat in 10 ml Toluol und 2 ml tert-BuOH umgesetzt. Die Aufreinigung erfolgt säulenchromatographisch (Flash; SiO₂; Hexan 90% / Ethylacetat 10 %). Ausbeute: 66 %; Schmp.: 157 °C
C₂₂H₁₇F₂NO₂ (Mr = 365,38); HPLC 9,4 min, 99,9 %; GC 34, 6 min
MS m/z (%): 365 (100, M+), 350 (3, M+-CH3), 337 (5, M+-CO), 322 (14, 337-CH3), 208 (10, 350-O, -Difluoranilin), 194 (3, 322-O, -Difluorbenzen), 178 (3, 322-OH, -Difluoranilin), 165 (14, 4-Methoxy-2-methylbenzoesäure), 152 (4, 165-CH2).
IR (ATR) (cm⁻¹): 3302, 2913, 1581, 1494, 1261, 1238, 1138, 1093, 1033, 965, 954, 862, 846,777,729,695,563,511,444.
¹H-NMR (DMSO-d6) δ in ppm: 3.02 (dd, 4H, J1 = 13.73 Hz, J2 = 8.96 Hz, -CH2-CH2-), 3.82 (s, 3H, -OCH3), 6.61 (s, 1 H, C1-H), 6.73 (d, 1 H, J = 8.64 Hz, C3-H), 6.84-6.92 (m, 2H, C3'-/C6'-H), 7.04-7.13 (m, 1 H, C5'-H), 7.34-7.42 (m, 2H, C7-/C9-H), 7.96 (d, 2H, J = 8.64 Hz, C4-/C6-H), 8.49 (s, 1H, -NH-).
¹³C-NMR (DMSO-d6) δ in ppm: 35.2 (C11), 35.8 (C10), 55.7 (-OCH3), 105.3 (dd, J1 = 26.5 Hz, J2 = 24.0 Hz, C3'), 112.1 (dd, J1 = 21.5 Hz, J2 = 3.7 Hz, C5'), 112.5 (C7), 112.7 (C9), 113.6 (C3), 113.9 (C1), 125.4 (dd, J1 = 12.1 Hz, J2 = 3.6 Hz, C1'), 126.3 (dd, J1 = 9.1 Hz, J2 = 3.3 Hz, C6'), 128.2 (C4a), 131.3 (C5a), 133.7 (C4), 133.8 (C6), 145.1 (C2), 145.3 (C11a), 149.1 (C9a), 155.9 (dd, J1 = 246.4 Hz, J2 = 12.5 Hz, C4'), 158.7 (dd, J1 = 241.6 Hz, J2 = 11.4 Hz, C2'), 162.4 (C8'), 189.1 (C5).

### Beispiel 111

### 2-(2-Aminophenylamino)-8-methoxy-10,11-dihydrodibenzo[a,d]cyclohepten-5-on (12b)

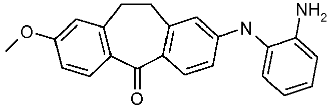

Nach der allgemeinen Methode O werden 0,48 g (1,8 mmol) 2-Chlor-8-methoxy-10,11-dihydro-dibenzo[a,d] cyclohepten-5-on, 1,05 g (9,7 mmol) Phenylendiamin, 0,04 g Pd(OAc)₂, 0,20 g Phosphinligand und 1,65g (17 mmol) Na-O-tert-Butylat in 10 ml Toluol und 2 ml tert-BuOH umgesetzt. Die Aufreinigung erfolgt säulenchromatographisch (Flash; SiO2; Hexan 80% / Ethylacetat 20 %). Ausbeute: 35 %; Schmp.: 200 °C
C₂₂H₂₀N₂O₂ (Mr = 344,42); HPLC 8,2 min; 99,6 %; GC 63,6 min
MS m/z (%): 344 (100, M+), 329 (8, M+-CH3), 315 (5, M+-CO), 301 (9, 315-CH3), 195 (10, 315-O, -Aminobenzen), 158 (11), 107 (7, Diaminobenzen).
IR (ATR) (cm⁻¹): 311, 2921, 2852, 1287, 1262, 1240, 1206, 1153, 1092, 1031, 835, 744, 696, 595, 562, 489, 441.
¹H-NMR (DMSO-d6) δ in ppm: 2.98 (dd, 4H, J1 = 17.6 Hz, J2 = 8.96 Hz, -CH2-CH2-), 3.81 (s, 3H, -OCH3), 4.84 (s, 2H, -NH2), 6.47 (s, 1 H, C1-H), 6.57-6.64 (m, 2H, C3'-/C6'-H), 6.76-6.91 (m, 5H, C3-/C7-/C9-H und C4'-/C5'-H), 7.91-7.98 (m, 3H, -NH- und C4-/C6-H).
¹³C-NMR (DMSO-d6) δ in ppm: 35.2 (C11), 36.1 (C10), 55.7 (-OCH3), 112.0 (C7), 112.6 (C9), 112.8 (C3), 113.8 (C1), 115,8 (C3'), 116,8 (C4'), 125.4 (C4a), 126.1 (C6'), 126.3 (C5'), 126.7 (C5a), 131.6 (C1), 133.7 (C4), 133.9 (C6), 144.0 (C2'), 145.0 (C2), 145.4 (C11a), 150.8 (C9a), 162.2 (C8), 188.7 (C5).

### Beispiel 112

### 2-(2,4-Difluor-phenylamino)-7-[2-((R)-2,2-dimethyl-[1,3]dioxolan-4-yl)-ethoxy]-10,11-dihydro-dibenzo[a,d]cyclohepten-5-on (55h)

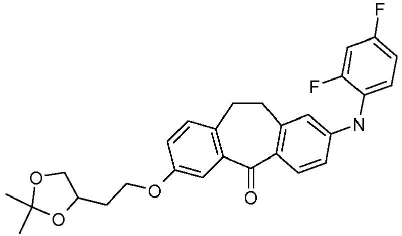

Nach der allgemeinen Methode AA werden 0,70 g (1,8 mmol) 2-Chlor-7-[2-((R)-2,2-dimethyl-[1,3]dioxolan-4-yl)-ethoxy]-10,11-dihydro-dibenzo¬[a,d]cyclohepten-5-on (36f), 0,25 g (1,9 mmol) 2,4-Difluoranilin, 0,05 g (0,22 mmol) Pd(OAc)₂, 0,10 g (0,21 mmol) 2-(Dicyclohexylphosphino)-2'-, 4'-,6'-triisopropyl-biphenyl, 0,60 g (6,2 mmol) Na-O-tert-Butylat, 5 ml Toluol und 1 ml tert-BuOH verwendet. Reaktionszeit: 1 h. Die Aufreinigung erfolgt über Flash-Chromatographie (SiO₂, Hexan/Ethylacetat 7+3). Ausbeute: 45%; Schmelzp.:123 °C C₂₈H₂₇F₂NO₄ (Mr = 479,53)
IR (ATR)* 3307 (N-H), 1607, 1552, 1499, 1355, 1286, 1201, 1142, 1093, 1054, 962, 869, 786 cm⁻¹
¹H-NMR (DMSO-d6) δ in ppm: 1.25 (s, 3 H, -CH3), 1.31 (s, 3 H, -CH3), 1.89-1.98 (m, 2 H, C2Ethoxy-H), 2.99 (s, 4 H, -CH2-CH2-), 3.52-3.59 (m, 1 H, C5Dioxolanyl-H), 4.00-4.23 (m, 4 H, C1 Ethoxy-H, C4Dioxolonyl-H, C5Dioxolanyl-H), 6.60 (s, 1 H, C1-H), 6.73 (d, 1 H, J = 8.7 Hz, C3-H); 7.00-7.46 (m, 6 H, C6-H, C8-H, C9-H, C3'-H, C5'-H, C6'-H), 7.94 (d, 1 H, J = 8.8 Hz, C4-H), 8.54 (s, 1 H, -NH-)
¹³C-NMR (DMSO-d6) δ in ppm: 26.0 (-CH3), 27.2 (-CH3), 33.3 (C2Ethoxy), 33.5 (C10), 36.2 (C11), 65.0 (C1Ethoxy), 69.0 (C5Dioxolanyl), 73.2 (C4Dioxolanyl), 105.3 (dd, 1 C, J1 = 24.5 Hz, J2 = 26.7 Hz, C3'), 108.3 (C2Dioxolanyl), 112.2 (dd, 1 C, J1 = 3.8 Hz, J2 = 22.2 Hz), 112.6 (C6), 113.8 (C3), 115.3 (C1), 119.0 (C8), 125.3 (dd, 1 C, J1 = 3.8 Hz, J2 = 12.0 Hz, C1'), 126.4 (dd, 1 C, J1 = 3.5 Hz, J2 = 9.8 Hz, C6'), 127.7 (C4a), 130.6 (C9), 133.8 (C5a), 134.7 (C4), 140.0 (C9a), 145.7 (C11a), 149.5 (C2), 156.0 (dd, 1 C, J1 = 12.3 Hz, J2 = 248.0 Hz, C4'), 158.6 (C7); 158.8 (dd, 1 C, J1 = 11.5 Hz, J2 = 242.9 Hz, C2'), 190.8 (C5)

### Beispiel 113

### 2-(2,4-Difluorphenylamino)-7-methoxy-10,11-dihydro-dibenzo[a,d]-cyclo-hepten-5-on (52b)

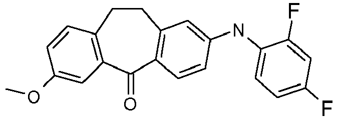

Nach der allgemeinen Methode AA werden 0,50 g (1,8 mmol) 2-Chlor-7-methoxy-10,11-dihydro-dibenzo[a,d]-cyclohepten-5-on (34), 0,25 g (1,9 mmol) 2,4-Difluoranilin, 0,05 g (0,22 mmol) Pd(OAc)₂, 0,10 g (0,21 mmol) 2-(Dicyclohexylphosphino)-2'-,4'-,6'-triisopropyl-biphenyl, 0,70 g (6,2 mmol) K-O-tert-Butylat, 5 ml Toluol und 1 ml tert-BuOH verwendet. Reaktionszeit: 1 h. Die Aufreinigung erfolgt über Flash-Chromatographie (SiO₂, Hexan/Ethylacetat 8+2).
Ausbeute: 87%; Schmelzpunkt: 123-126 °C (Zersetzung)
C₂₂H₁₇F₂NO₂ (Mr = 365,38); GC 31,4 min
MS m/z (%): 365 (100, M+), 350 (7, M⁺-CH₃), 337 (11), 322 (6), 237 (4, M⁺-2-(NH₂-, 4-F-Anilin)), 208 (15), 194 (5), 178 (6), 165 (15), 152 (5)
IR (ATR) 3327 (N-H), 3074 (C-H), 2941-2842 (C-H), 1604, 1552, 1525, 1498, 1338, 1325, 1281, 1238, 854, 832, 786 cm⁻¹
¹H-NMR (DMSO-d6) 5 in ppm: 2.99 (s, 4 H, -CH2-CH2-), 3.76 (s, 3 H, -OCH3), 6.61 (s, 1 H, C1-H), 6.74 (d, 1 H, J = 8.7 Hz, C3-H), 7.00-7.47 (m, 6 H, C6-H, C8-H, C9-H, C3'-H, C5'-H, C6'-H), 7.95 (d, 1 H, J = 8.8 Hz, C4-H), 8.55 (s, 1 H, -NH-)
¹³C-NMR (DMSO-d6) δ in ppm: 33.5 (C10), 36.2 (C11), 55.5 (-OCH3), 105.3 (dd, 1 C, J1 = 24.3 Hz, J2 = 26.7 Hz, C3'), 112.2 (dd, 1 C, J1 = 3.5 Hz, J2 = 21.8 Hz, C5'), 112.6 (C6), 113.9 (C3), 114.7 (C1), 118.6 (C8), 125.3 (dd, 1 C, J1 = 3.8 Hz, J2 = 12.3 Hz, C1'), 126.4 (dd, 1 C, J1 = 3.4 Hz, J2 = 9.6 Hz, C6'), 127.7 (C4a), 130.6 (C9), 133.8 (C5a), 134.6 (C4), 140.0 (C9a), 145.7 (C11a), 149.4 (C2), 156.0 (dd, 1 C, J1 = 12.3 Hz, J2 = 248.1 Hz, C4'), 158.1 (C7), 158.8 (dd, 1 C, J1 = 11.1 Hz, J2 = 143.1 Hz, C2'), 190.8 (C5)

### Beispiel 114

### 7-Methoxy-2-(2,3,4-trifluorphenylamino)-10,11-dihydro-dibenzo[a,d]-cyclo-hepten-5-on (52n)

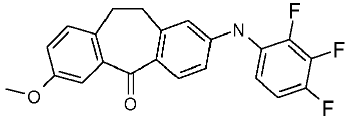

Nach der allgemeinen Methode AA werden 0,50 g (1,8 mmol) 2-Chlor-7-methoxy-10,11-dihydro-dibenzo[a,d]-cyclohepten-5-on (34), 0,27 g (1,8 mmol) 2,3,4-Trifluoranilin, 0,05 g (0,22 mmol) Pd(OAc)₂, 0,10 g (0,21 mmol) 2-(Dicyclohexylphosphino)-2'-,4'-,6'-triisopropylbiphenyl, 0,60 g (6,2 mmol) Na-O-tert-Butylat, 5 ml Toluol und 1 ml tert-BuOH verwendet. Reaktionszeit: 1 h. Die Aufreinigung erfolgt über Flash-Chromatographie (SiO₂, Hexan/Ethylacetat 8+2). Ausbeute: 72%; Schmelzpunkt: 128 °C
C₂₂H₁₆F₃NO₂ (Mr = 383,37); GC 32,0 min
MS m/z (%): 383 (100, M⁺), 368 (7, M⁺-CH₃), 355 (10), 340 (5), 237 (3), 208 (11), 194 (5), 178 (6), 165 (11)
IR (ATR)* 2930, 1578, 1492,1355, 1265, 1035, 1000, 970, 839, 814 cm⁻¹
¹H-NMR (DMSO-d6) δ in ppm: 3.03 (s, 4 H, -CH2-CH2-), 3.77 (s , 3 H, -OCH), 6.95-7.47 (m, 7 H, C1-H, C3-H, C6-H, C8-H, C9-H, C5'-H, C6'-H), 7.92 (d, 1 H, J = 8.6 Hz, C4-H), N-H-Signal nicht sichtbar.
¹³C-NMR (DMSO-d6) δ in ppm: 33.3 (C10), 35.2 (C11), 55.6 (-OCH3), 113.6-114.0 (m, 1 C, C1'); 114.3 (C6); 119.2 (C3), 119.8 (C1), 122.0 (C8), 125.0-125.2 (m, 1 C, C5'), 129.9-130.2 (m, 1C, C6'); 131.0 (C4a), 132.6 (C9), 132.7-135.2 (C4'), 133.0 (C5a), 134.6 (C4), 139.4 (C9a), 145.1 (C11a), 149.0 (C2), 145.3-147.9 (m, 1 C, C3'), 246.8-148.3 (m, 1 C, C2'), 158.1 (C7), 192.3 (C5)

### Beispiel 115

### 2-(2,4-Difluor-phenylamino)-7-((R)-3,4-dihydroxy-butoxy)-10,11-dihydro-dibenzo[a,d]cyclohepten-5-on (55i)

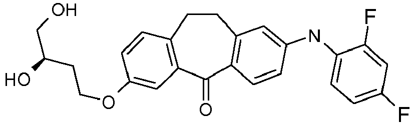

Nach der allgemeinen Methode AB werden 1,00 g (2,1 mmol) 2-(2,4-Difluor-phenylamino)-7-[2-((R)-2,2-dimethyl-[1,3]dioxolan-4-yl)-ethoxy]-10,11-dihydro-dibenzo[a,d]cyclohepten-5-on (55h), 40 ml MeOH, 10 ml H2O und 0,25 g (1,3 mmol) p-Toluolsulfonsäure verwendet.
Ausbeute: 54%; Schmelzpunkt: 123 °C; C₂₅H₂₃F₂NO₄ (Mr = 439,46)
IR (ATR) 3310 (N-H), 2928, 1567,1508, 1261, 1140, 1094, 1055, 964, 845, 808, 780 cm⁻¹
¹H-NMR (DMSO-d6) δ in ppm: 0.85 (s, 1 H, -OH), 1.25 (s, 1 H, -OH), 1.52-1.98 (m, 2 H, C2Butanoxy-H), 2.99 (s, 4 H, -CH2-CH2-), 3.55-3.63 (m, 1 H, C3Butanoxy-H), 4.00-4.08 (m, 2 H, C4Butanoxy-H), 4.53-4.60 (m, 2 H, C1Butanoxy-H), 6.59 (s, 1 H, C1-H), 6.72 (d, 1 H, J = 8.6 Hz, 1 H, C3-H), 6.94-7.46 (m, 6 H, C6-H, C8-H, C9-H, C3'-H, C5'-H, C6'-H), 7.94 (d, 1 H, J = 8.6 Hz, C4-H), 8.58 (s, 1 H, -NH-)
¹³C-NMR (DMSO-d6) δ in ppm: 33.5 (C11), 33.5 (C2Butanoxy), 36.2 (C10), 65.1 (C1Butanoxy), 66.3 (C4Butanoxy), 68.4 (C3Butanoxy), 105.3 (dd, 1 C, J1 = 24.5 Hz, J2 = 27.2 Hz, C3') 112.2 (dd, 1 C, J1 = 3.5 Hz, J2 = 21.8 Hz, C5'), 112.6 (C6), 113.8 (C3), 115.3 (C1), 119.1 (C8), 125.3 (dd, 1 C, J1 = 3.1 Hz, J2 = 11.9 Hz, C1'), 126.4 (dd 1 C, J1 = 3.4 Hz, J2 = 8.6 Hz, C6'), 127.8 (C4a), 130.6 (C9), 133.8 (C5a), 134.5 (C4), 139.9 (C9a), 145.7 (C11a), 149.4 (C2), 156.0 (dd, 1 C, J1 = 19.2 Hz, J2 = 259.8 Hz, C4'), 157.5 (C7), 158.8 (dd, 1 C, J1 = 16.1 Hz, J2 = 246.8 Hz, C2'), 190.8 (C5)

### Beispiel 116

### 7-Chlor-3-(2,4-difluorphenylamino)-6,11-dihydrodibenzo[b,e]oxepin-11-on (13)

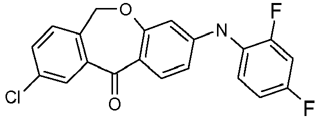

Nach der allgemeinen Methode AC werden 0,5 g (1,92 mmol) 3-Amino-7-chlor-3,11-dihydrodibenzo[b,e]oxepin-11-on in 15 ml Toluol gelöst und mit 0,08 g 2-(Dicyclohexylphoshino)-2', 4', 6'-triisopropylbiphenyl, 0,37 g 1-Brom-2,4-difluorbenzen, 0,70 g K-O-tert-Butylat, 2ml tert-BuOH und zuletzt 2 Spatelspitzen Pd(OAc)₂ versetzt. Der Reaktionsansatz wird nun unter DC-Kontrolle in Argon-Atmosphäre 4 h bei 100°C refluxiert. Das Produktgemisch wird über Kieselgel mit Hexan:EtOAc (3:1) chromatographisch aufgereinigt.
Ausbeute: 52 mg (7,2%); Schmelzpunkt: 207,9 °C
C₂₀H₁₂ClF₂NO₂ (MR= 371,8 g/mol); GC (Methode 1 ): 21,43 min
¹H-NMR (DMSO-d6): δ (in ppm) = 8.77 (s, 1 H, -NH-), 7.93 (d, 1 H, J=8,96 Hz, aryl H), 7.74 - 7.66 (m, 2 H, aryl H), 7.54 - 7.32 (m, 3 H, aryl H), 7.10 (t, 1 H, J= 9,46 Hz, aryl H), 6.62 (d, 1 H, J= 8,08 Hz, aryl H), 6.24 (s, 1 H, aryl H), 5.37 (s, 2 H, CH2-O-)
¹³C-NMR (DMSO-d6): δ (in ppm) = 187.14 (C11), 163.05 (C4a), 160.14 (q, C2 NH-Ph, J1=148.56 Hz, J2=11.80), 155.26 (q, C4 NH-Ph, J1=152.75Hz, J2=11.80), 127,10 (q, C6 NH-Ph, J1=9.52 Hz, J2=3,04 Hz), 124.63 (q, C1 NH-Ph, J1=11,99 Hz, J2= 3,23 Hz), 112.27 (q, C5 NH-Ph, J1= 22.09 Hz, J2=3.81 Hz), 105.37 (q, C3 NH-Ph, J1= 26.28 Hz, J2= 24,00 Hz), 152.53 (C3), 143.11 (C6a), 133.54 (C8), 133.11 (C1), 132.81 (C7), 131.84 (C10a), 130.83 (C9), 128.12 (C10), 116.38 (C11 a), 110.15 (C2), 101.75 (s, C4) ,68.84 (C6)
MS: m/z (%): 371(100), 336(36), 316(6), 243(7), 215(8), 203(7), 152(16), 151(7), 139(8), 89(9), 63 (9)
IR (ATR): v [cm⁻¹]= 3306, 1593, 1552, 1512, 1295, 1269, 1141, 1120, 841, 763

## Patentansprüche

1. Verbindungen der Formel I 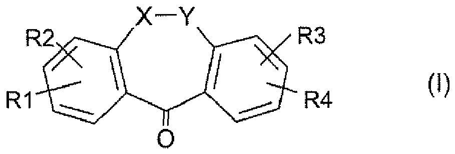 worin
eines der Ringatome X und Y für CH₂ und das andere für O, S, SO, SO₂ oder NR5 steht; oder -X-Y- für -CH₂-CH₂- oder -CH=CH- steht;
R1 für RO- steht, wobei R ausgewählt ist unter:
a) C₁-C₆-Alkyl, das mit 1, 2 oder 3 Hydroxy- oder C₁-C₆-Alkoxygruppen substituiert ist;
b) C₁-C₆-Alkyl, das substituiert ist mit einem gesättigten oder ungesättigten, aromatischen oder nicht-aromatischen Heterocyclus mit 5 oder 6 Ringatomen, der 1, 2 oder 3 Heteroatome aufweist, die unabhängig voneinander ausgewählt sind unter O, N und S, wobei der Heterocyclus gegebenenfalls 1 oder 2 Hydroxy-, C₁-C₆-Alkoxy- oder C₁-C₆-Alkylsubstituenten aufweisen und mit einem Phenylring oder einem gesättigten oder ungesättigten Carbocyclus mit 5 oder 6 Ringatomen kondensiert sein kann;
c) C₁-C₆-Alkyl; und
d) C₁-C₆-Alkyl, das mit NR6R7 substituiert ist;
R2 für H oder C₁-C₆-Alkyl steht;
R3 ausgewählt ist unter:
a) 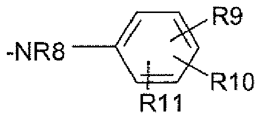
b) 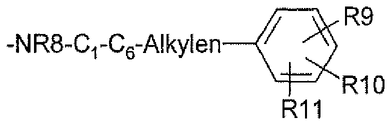
c) 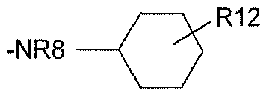
d) 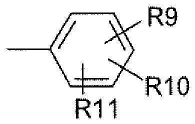
e) -NH-C₁-C₆-Alkylen-NR6R7
f) Tetrazolo;
R4 für H, Halogen oder C₁-C₆-Alkyl steht;
R5 für H oder C₁-C₆-Alkyl, das mit 1, 2 oder 3 Hydroxy- oder C₁-C₆-Alkoxygruppen substituiert ist, steht;
R6 und R7, die gleich oder verschieden sein können, für H oder C₁-C₆-Alkyl, das mit 1, 2 oder 3 Hydroxy- oder C₁-C₆-Alkoxygruppen substituiert ist, stehen;
R8 für H oder C₁-C₆-Alkyl steht;
R9, R10 und R11, die gleich oder verschieden sein können, ausgewählt sind unter:
a) H,
b) NH₂,
c) mono-C₁-C₆-Alkylamino,
d) di-C₁-C₆-Alkylamino,
e) C₁-C₆-Alkyl,
f) C₁-C₆-Alkoxy,
g) Hydroxy,
h) Halogen,
i) C₁-C₆-Alkyl, das mit 1, 2 oder 3 Halogenatomen substituiert ist;
j) CONR6R7; und
k) NO₂;
R12 für H oder NH₂ steht;
R13 und R14, die gleich oder verschieden sein können, für H oder C₁-C₆-Alkyl stehen oder zusammen mit dem Stickstoffatom an das sie gebunden sind, einen nicht-aromatischen Heterocyclus mit 5 oder 6 Ringatomen, der 1 oder 2 Heteroatome aufweist, die unabhängig voneinander ausgewählt sind unter O und N, bilden;
und die optischen Isomeren, physiologisch verträglichen Salze und Solvate davon.

2. Verbindungen nach Anspruch 1, ausgewählt unter einer der Formeln Ia, Ib und Ic 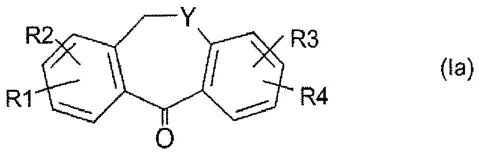 worin Y für O steht und R1, R2, R3 und R4 die in Anspruch 1 angegebenen Bedeutungen besitzen; 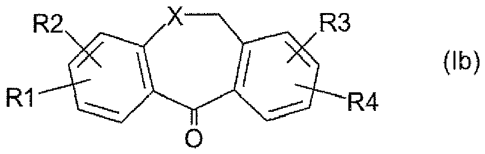 worin X für O steht und R1, R2, R3 und R4 die in Anspruch 1 angegebenen Bedeutungen besitzen; und 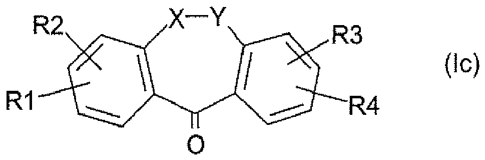 worin -X-Y- für -CH₂ CH₂- oder -CH=CH- steht und R1, R2, R3 und R4 die in Anspruch 1 angegebenen Bedeutungen besitzen.

3. Verbindungen nach einem der vorhergehenden Ansprüche, wobei R1 ausgewählt ist unter C₁-C₆-Alkyl, das mit 1, 2 oder 3 Hydroxygruppen substituiert ist.

4. Verbindungen nach einem der vorhergehenden Ansprüche, wobei R3 ausgewählt ist unter
b) 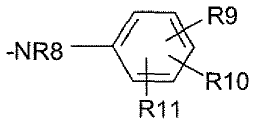
e) Phenyl und
g) Tetrazolo.

5. Verbindungen nach Anspruch 4, wobei R9 und R10 unabhängig voneinander ausgewählt sind unter H, NH₂, mono-C₁-C₆-Alkylamino, di-C₁-C₆-Alkylamino, Halogen, CF₃, C₁-C₆-Alkyl und C₁-C₆-Alkoxy und R11 für H oder Halogen steht.

6. Verbindungen nach Anspruch 4 oder 5, wobei R3 für einen Rest der Formel 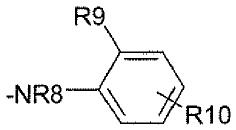 steht.

7. Verbindungen nach einem der vorhergehenden Ansprüche, wobei R4 für H steht.

8. Verbindungen nach einem der vorhergehenden Ansprüche, wobei R6 und R7 für H stehen.

9. Verbindungen nach einem der vorhergehenden Ansprüche der Formel

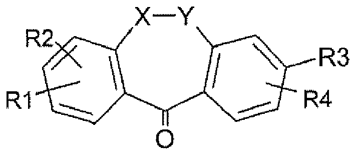

10. Verbindungen nach Anspruch 2 der Formel Iaa 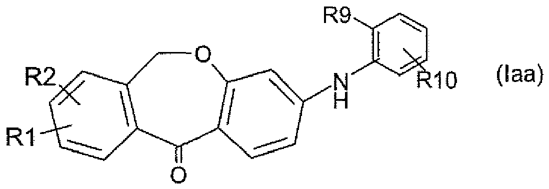 worin R1, R2, R9 und R10 die in Anspruch 1 oder 5 angegebenen Bedeutungen besitzen.

11. Verbindungen nach Anspruch 2 der Formel Ica: 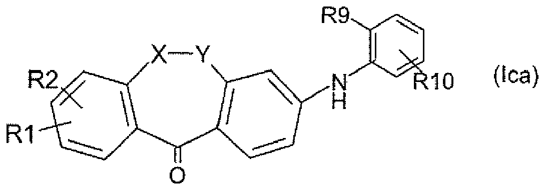 worin
-X-Y- für -CH₂ CH₂ oder -CH=CH- steht und R1, R2, R9 und R10 die in Anspruch 1 oder 5 angegebenen Bedeutungen besitzen.

12. Verbindungen nach Anspruch 1:
(1) 2-(2-Aminoanilino)-7-methoxydibenzosuberon;
(2) 2-(2-Amino-4-fluoranilino)-7-methoxydibenzosuberon;
(3) 2-(2,4-Difluoranilino)-7-methoxydibenzosuberon;
(4) 2-(2-Chlor-4-fluoranilino)-7-methoxydibenzosuberon;
(5) 2-(2,4,5-Trifluoranilino)-7-methoxydibenzosuberon;
(6) 2-(2-Trifluormethylanilino)-7-methoxydibenzosuberon;
(7) 2-(Anilino)-7-methoxydibenzosuberon;
(8) 2-(2-Methoxyanilino)-7-methoxydibenzosuberon;
(9) 2-(3-Methyl-4-fluoranilino)-7-methoxydibenzosuberon;
(10) 2-(2-Amino-4-trifluormethylanilino)-7-methoxydibenzosuberon;
(11) 2-(Phenyl)-7-methoxydibenzosuberon;
(12) 2-(2,4-Difluoranilino)-7-methoxydibenzosuberenon;
(13) 2-(2,4-Difluoranilino)-7-(S-1,2-isopropylidenglycer-3-yl)-10,11-dihydro-dibenzo[a,d]-cyclohepten-5-on;
(14) 2-(2,4-Difluoranilino)-7-(R-1,2-isopropylidenglycer-3-yl)-10,11-dihydro-dibenzo[a,d]-cyclohepten-5-on;
(15) 2-(2-Aminoanilino)-7-(S-1,2-isopropylidenglycer-3-yl)-10,11-dihydro-dibenzo[a,d]-cyclohepten-5-on;
(16) 2-(2-Aminoanilino)-7-(R-1,2-isopropylidenglycer-3-yl)-10,11-dihydro-dibenzo[a,d]-cyclohepten-5-on;
(17) 2-(2,4-Difluoranilino)-7-[2R-,3-dihydroxypropoxy]-10,11-dihydro-dibenzo[a,d]-cyclohepten-5-on;
(18) 2-(2,4-Difluoranilino)-7-[2S-,3-dihydroxypropoxy]-10,11-dihydro-dibenzo[a,d]-cyclohepten-5-on;
(19) 2-(2-Aminoanilino-7-[2R-,3-dihydroxypropoxyl-10,11-dihydro-dibenzo[a,d]-cyclohepten-5-on;
(20) 2-(2-Aminoanilino-7-[2S-,3-dihydroxypropoxy]-10,11-dihydro-dibenzo[a,d]-cyclohepten-5-on;
(21) 2-(2,4-Difluoranilino)-7-(2-hydroxy-ethoxy)-10,11-dihydro-dibenzo[a,d]-cyclohepten-5-on;
(22) 2-(2,4-Difluoranilino)-7-(3-hydroxy-propoxy)-10,11-dihydro-dibenzo[a,d]-cyclohepten-5-on;
(23) (S)-2-(2,4-Difluorophenylamino)-8-(2,2-dimethyl-[1,3]dioxolan-4-ylmethoxy)-10,11-dihydro-dibenzo[a,d]cyclohepten-5-on;
(24) (R)-2-(2,4-Difluorophenylamino)-8-(2,3-dihydroxypropoxy)-10,11-dihydrodibenzo[a,d]cyclohepten-5-on;
(25) (S)-2-(2-Aminophenylamino)-8-(2,2-dimethyl-[1,3]dioxolan-4-ylmethoxy)-10,11-dihydrodibenzo[a,d]cyclohepten-5-on;
(26) (R)-2-(2-Aminophenylamino)-8-(2,3-dihydroxypropoxy)-10,11-dihydrodibenzo[a,d]cyclohepten-5-on;
(27) 2-(2,4-Difluorphenylamino)-8-(2-morpholin-4-yl-ethoxy)-10,11-dihydrodibenzo[a,d]cyclohepten-5-on;
(28) 8-(2,4-Difluorphenylamino)-1-methoxy-10,11-dihydrodibenzo[a,d]cyclohepten-5-on;
(29) 8-(2-Aminophenylamino)-1-methoxy-10,11-dihydrodibenzo[a,d]cyclohepten-5-on;
(30) (S)-8-(2,4-Difluorphenylamino)-1-(2,2-dimethyl-[1,3]dioxolan-4-ylmethoxy)-10,11-dihydrodibenzo[a,d]cyclohepten-5-on;
(31) (R)-8-(2,4-Difluorphenylamino)-1-(2,3-dihydroxypropoxy)-10,11-dihydrodibenzo-[a,d]cyclohepten-5-on;
(32) (S)-8-(2-Aminophenylamino)-1-(2,2-dimethyl-[1,3]dioxolan-4-ylmethoxy)-10,11-dihydrodibenzo[a,d]cycloheplen-5-on;
(33) (R)-8-(2-Aminophenylamino)-1-(2,3-dihydroxypropoxy)-10,11-dihydrodibenzo-[a,d]cyclo-hepten-5-on;
(34) 8-(2,4-Difluorphenylamino)-1-(2-morpholin-4-yl-ethoxy)-10,11-dihydrodibenzo-[a,d]cyclo-hepten-5-on;
(35) 2-(2-Methyl-4-Fluoranilino)-7-methoxydibenzosuberon;
(36) 2-(2-Chloranilino)-7-methoxydibenzosuberon.

13. Pharmazeutisches Mittel, enthaltend wenigstens eine Verbindung der Formel I gemäß einem der Ansprüche 1 bis 12, gegebenenfalls zusammen mit physiologisch verträglichen Exzipienten.

14. Verbindung der Formel I nach einem der Ansprüche 1 bis 12, zur Anwendung in einem Verfahren zur Behandlung von Autoimmunerkrankungen, Krebs, rheumatischer Arthritis, Gicht, septischem Schock, Osteoporose, neuropathischem Schmerz, HIV-Ausbreitung, HIV-Demenz, viraler Myokarditis, insulinabhängiger Diabetes, Periodontalerkrankungen, Restenose, Alopezie, T-Zell-Depletion bei HIV-Infektionen oder AIDS, Psoriasis, akuter Pankreatitis, Abstoßungsreaktionen bei allogenen Transplantaten, allergisch bedingter Lungenentzündung, Arteriosklerose, Multipler Sklerose, Kachexie, Alzheimer Erkrankung, Schlaganfall, Ikterus, Colitis ulcerosa, Morbus Crohn, Inflammatory-Bowel-Disease (IBD), Ischämie, kongestiver Herzinsuffizienz, Lungen-Fibrose, Hepatitis, Glioblastom, Guillain-Barre-Syndrom, systemischem Lupus erythematodes, Adult-respiratory-distress-Syndrom (ARDS) und Atemnotsyndrom.

## Claims

1. Compounds of formula I 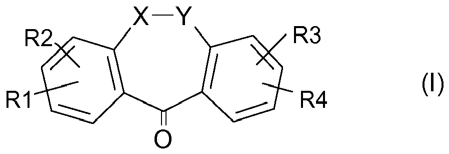 wherein
one of the ring atoms X and Y represents CH₂ and the other represents O, S, SO, SO₂ or NR5; or -X-Y- represents -CH₂-CH₂- or -CH=CH-;
R1 represents RO-, wherein R is selected from:
a) C₁-C₆-alkyl, which is substituted by 1, 2 or 3 hydroxyl or C₁-C₆-alkoxy groups;
b) C₁-C₆-alkyl, which is substituted by a saturated or unsaturated, aromatic or non-aromatic heterocyclic radical having 5 or 6 ring atoms, which contains 1, 2 or 3 hetero atoms which are selected independently of each other from O, N and S, wherein the heterocyclic radical can optionally contain 1 or 2 hydroxy, C₁-C₆-alkoxy or C₁-C₆-alkyl substituents and can be condensed with a phenyl ring or a saturated or unsaturated carbocyclic radical having 5 or 6 ring atoms;
c) C₁-C₆-alkyl; and
d) C₁-C₆-alkyl, which is substituted by NR6R7;
R2 represents H or C₁-C₆-alkyl;
R3 is selected from:
a) 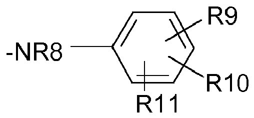
b) 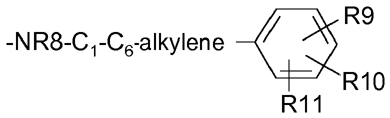
c) 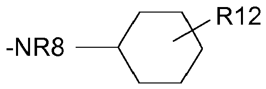
d) 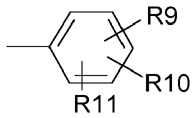
e) -NH-C₁-C₆-alkylene-NR6R7
f) tetrazolo;
R4 represents H, halogen or C₁-C₆-alkyl;
R5 represents H or C₁-C₆-alkyl, which is substituted by 1, 2 or 3 hydroxyl or C₁-C₆-alkoxy groups;
R6 and R7, which can be identical or different, represent H or C₁-C₆-alkyl, which is substituted by 1, 2 or 3 hydroxyl or C₁-C₆-alkoxy groups;
R8 represents H or C₁-C₆-alkyl;
R9, R10 and R11, which can be identical or different, are selected from:
a) H,
b) NH₂,
c) mono-C₁-C₆-alkylamino,
d) di-C₁-C₆-alkylamino,
e) C₁-C₆-alkyl,
f) C₁-C₆-alkoxy,
g) hydroxyl,
h) halogen,
i) C₁-C₆-alkyl, which is substituted by 1, 2 or 3 halogen atoms;
j) CONR6R7; and
k) NO₂;
R12 represents H or NH₂;
R13 and R14, which can be identical or different, represent H or C₁-C₆-alkyl, or together with the nitrogen atom to which they are bonded form a non-aromatic heterocyclic radical having 5 or 6 ring atoms, which contains 1 or 2 hetero atoms which are selected independently of each other from O and N;
and the optical isomers, physiologically acceptable salts and solvates thereof.

2. The compounds according to claim 1 selected from one of the formulae la, Ib and Ic 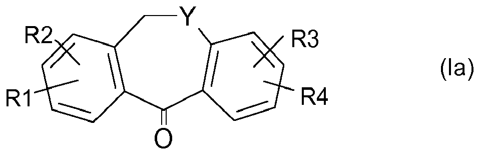 wherein Y represents O and R1, R2, R3 and R4 have the meanings given in claim 1; 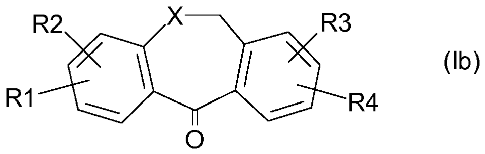 wherein X represents O and R1, R2, R3 and R4 have the meanings given in claim 1; 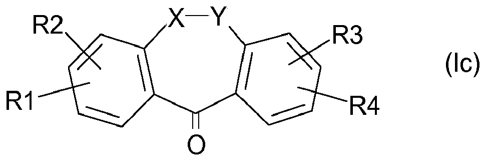 wherein -X-Y- represents -CH₂-CH₂- or -CH=CH- and R1, R2, R3 and R4 have the meanings given in claim 1.

3. The compounds according to one of the preceding claims, wherein R1 is selected from C₁-C₆-alkyl, which is substituted by 1, 2 or 3 hydroxyl groups.

4. The compounds according to one of the preceding claims, wherein R3 is selected from
b) 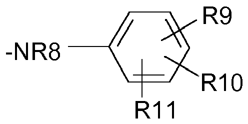
e) phenyl and
g) tetrazolo.

5. The compounds according to claim 4, wherein R9 and R10 independently of each other are selected from H, NH₂, mono-C₁-C₆-alkylamino, di-C₁-C₆-alkylamino, halogen, CF₃, C₁-C₆-alkyl and C₁-C₆-alkoxy and R11 represents H or halogen.

6. The compounds according to claim 4 or 5, wherein R3 represents a radical of the formula

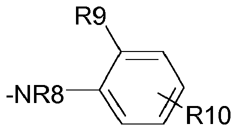

7. The compounds according to one of the preceding claims, wherein R4 represents H.

8. The compounds according to one of the preceding claims, wherein R6 and R7 represent H.

9. The compounds according to one of the preceding claims of the formula

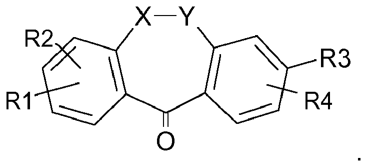

10. The compounds according to claim 2 of the formula laa 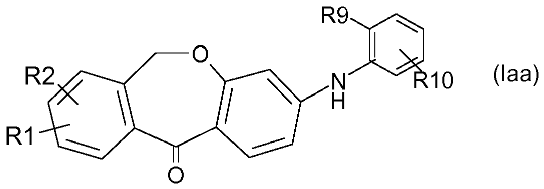 wherein R1, R2, R9 and R10 have the meanings given in claim 1 or 5.

11. The compounds according to claim 2 of the formula Ica: 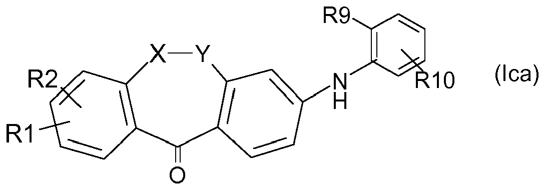 wherein
-X-Y- represents -CH₂-CH₂- or -CH=CH- and R1, R2, R9 and R10 have the meanings given in claim 1 or 5.

12. The compounds according to claim 1:
(1) 2-(2-aminoanilino)-7-methoxydibenzosuberone
(2) 2-(2-amino-4-fluoroanilino)-7-methoxydibenzosuberone
(3) 2-(2,4-difluoroanilino)-7-methoxydibenzosuberone
(4) 2-(2-chloro-4-fluoroanilino)-7-methoxydibenzosuberone
(5) 2-(2,4,5-trifluoroanilino)-7-methoxydibenzosuberone
(6) 2-(2-trifluoromethylanilino)-7-methoxydibenzosuberone
(7) 2-(anilino)-7-methoxydibenzosuberone
(8) 2-(2-methoxyanilino)-7-methoxydibenzosuberone
(9) 2-(3-methyl-4-fluoroanilino)-7-methoxydibenzosuberone
(10) 2-(2-amino-4-trifluoromethylanilino)-7-methoxydibenzosuberone
(11) 2-(phenyl)-7-methoxydibenzosuberone
(12) 2-(2,4-difluoroanilino)-7-methoxydibenzosuberenone
(13) 2-(2,4-difluoroanilino)-7-(S-1,2-isopropylideneglycer-3-yl)-10,11-dihydrodibenzo[a,d]-cyclohepten-5-one
(14) 2-(2,4-difluoroanilino)-7-(R-1,2-isopropylideneglycer-3-yl)-10,11-dihydrodibenzo[a,d]-cyclohepten-5-one
(15) 2-(2-aminoanilino)-7-(S-1,2-isopropylideneglycer-3-yl)-10,11-dihydrodibenzo[a,d]-cyclohepten-5-one
(16) 2-(2-aminoanilino)-7-(R-1,2-isopropylideneglycer-3-yl)-10,11-dihydrodibenzo[a,d]-cyclohepten-5-one
(17) 2-(2,4-difluoroanilino)-7-[2R-,3-dihydroxypropoxy]-10,11-dihydrodibenzo[a,d]-cyclohepten-5-one
(18) 2-(2,4-difluoroanilino)-7-[2S-,3-dihydroxypropoxy]-10,11-dihydrodibenzo[a,d]-cyclohepten-5-one
(19) 2-(2-aminoanilino-7-[2R-,3-dihydroxypropoxy]-10,11-dihydrodibenzo[a,d]-cyclohepten-5-one
(20) 2-(2-aminoanilino-7-[2S-,3-dihydroxypropoxy]-10,11-dihydrodibenzo[a,d]-cyclohepten-5-one
(21) 2-(2,4-difluoroanilino)-7-(2-hydroxy-ethoxy)-10,11-dihydrodibenzo[a,d]-cyclohepten-5-one
(22) 2-(2,4-difluoroanilino)-7-(3-hydroxy-propoxy)-10,11-dihydrodibenzo[a,d]-cyclohepten-5-one
(23) (S)-2-(2,4-difluorophenylamino)-8-(2,2-dimethyl-[1,3]dioxolan-4-ylmethoxy)-10,11-dihydrodibenzo[a,d]cyclohepten-5-one
(24) (R)-2-(2,4-difluorophenylamino)-8-(2,3-dihydroxypropoxy)-10,11-dihydrodibenzo[a,d]cyclohepten-5-one
(25) (S)-2-(2-aminophenylamino)-8-(2,2-dimethyl-[1,3]dioxolan-4-ylmethoxy)-10,11-dihydrodibenzo[a,d]cyclohepten-5-one
(26) (R)-2-(2-aminophenylamino)-8-(2,3-dihydroxypropoxy)-10,11-dihydrodibenzo[a,d]cyclohepten-5-one
(27) 2-(2,4-difluorophenylamino)-8-(2-morpholin-4-yl-ethoxy)-10,11-dihydrodibenzo[a,d]cyclohepten-5-one
(28) 8-(2,4-difluorophenylamino)-1-methoxy-10,11-dihydrodibenzo[a,d]cyclohepten-5-one
(29) 8-(2-aminophenylamino)-1-methoxy-10,11-dihydrodibenzo[a,d]cyclohepten-5-one
(30) (S)-8-(2,4-difluorophenylamino)-1-(2,2-dimethyl-[1,3]dioxolan-4-ylmethoxy)-10,11-dihydrodibenzo[a,d]cyclohepten-5-one
(31) (R)-8-(2,4-difluorophenylamino)-1-(2,3-dihydroxypropoxy)-10,11-dihydrodibenzo-[a,d]cyclohepten-5-one
(32) (S)-8-(2-aminophenylamino)-1-(2,2-dimethyl-[1,3]dioxolan-4-ylmethoxy)-10,11-dihydrodibenzo[a,d]cyclohepten-5-one
(33) (R)-8-(2-aminophenylamino)-1-(2,3-dihydroxypropoxy)-10,11-dihydrodibenzo-[a,d]cyclo-hepten-5-one
(34) 8-(2,4-difluorophenylamino)-1-(2-morpholin-4-yl-ethoxy)-10,11-dihydrodibenzo-[a,d]cyclo-hepten-5-one
(35) 2-(2-methyl-4-fluoroanilino)-7-methoxydibenzosuberone
(36) 2-(2-chloroanilino)-7-methoxydibenzosuberone.

13. A pharmaceutical composition containing at least one compound of formula I according to one of claims 1 to 12, optionally together with physiologically acceptable excipients.

14. The compound of formula I according to one of claims 1 to 12 for use in a method for the treatment of autoimmune diseases, cancer, rheumatic arthritis, gout, septic shock, osteoporosis, neuropathic pain, HIV propagation, HIV dementia, viral myocarditis, insulin-dependent diabetes, periodontal diseases, restenosis, alopecia, T-cell depletion with HIV infections or AIDS, psoriasis, acute pancreatitis, rejection reactions with allogenic transplants, lung inflammation of allergic origin, arteriosclerosis, multiple sclerosis, cachexia, Alzheimer's disease, stroke, jaundice, colitis ulcerosa, Crohn's disease, inflammatory bowel disease (IBD), ischaemia, congestive cardiac insufficiency, pulmonary fibrosis, hepatitis, glioblastoma, Guillain-Barré syndrome, systemic lupus erythematosus, adult respiratory distress syndrome (ARDS) and respiratory distress syndrome.

## Revendications

1. Composés de formule I 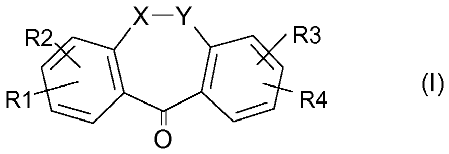 dans laquelle
l'un des atomes X et Y formant le cycle représente CH₂ et l'autre représente O, S, SO, SO₂ ou NR5 ;
ou -X-Y- représente -CH₂-CH₂- ou -CH=CH- ;
R1 représente RO-, R étant choisi parmi :
a) un groupe alkyle en C₁-C₆ qui est substitué par 1, 2 ou 3 groupes hydroxy ou alcoxy en C₁-C₆ ;
b) un groupe alkyle en C₁-C₆ qui est substitué par un hétérocycle saturé ou insaturé, aromatique ou non aromatique, à 5 ou 6 atomes formant le cycle, qui comporte 1, 2 ou 3 hétéroatomes qui sont choisis chacun indépendamment parmi O, N et S, l'hétérocycle comportant éventuellement 1 ou 2 substituants hydroxy, alcoxy en C₁-C₆ ou alkyle en C₁-C₆ et pouvant être soudé à un cycle phényle ou à un cycle carbocyclique saturé ou insaturé ayant 5 ou 6 atomes formant le cycle ;
c) un groupe alkyle en C₁-C₆ ; et
d) un groupe alkyle en C₁-C₆ qui est substitué par NR6R7 ;
R2 représente H ou un groupe alkyle en C₁-C₆ ;
R3 est choisi parmi :
a) 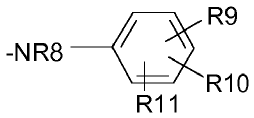
b) 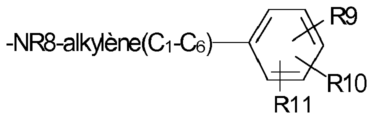
c) 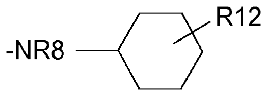
d) 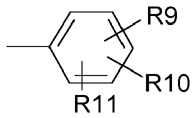
e) -NH-alkylène(C₁-C₆)-NR6R7
f) tétrazolo ;
R4 représente H, un atome d'halogène ou un groupe alkyle en C₁-C₆ ;
R5 représente H ou un groupe alkyle en C₁-C₆ qui est substitué par 1, 2 ou 3 groupes hydroxy ou alcoxy en C₁-C₆ ;
R6 et R7, qui peuvent être identiques ou différents, représentent H ou un groupe alkyle en C₁-C₆ qui est substitué par 1, 2 ou 3 groupes hydroxy ou alcoxy en C₁-C₆ ;
R8 représente H ou un groupe alkyle en C₁-C₆ ;
R9, R10 et R11, qui peuvent être identiques ou différents, sont choisis parmi :
a) H,
b) NH₂,
c) un groupe monoalkyl(C₁-C₆)amino,
d) un groupe dialkyl(C₁-C₆)amino,
e) un groupe alkyle en C₁-C₆,
f) un groupe alcoxy en C₁-C₆,
g) un groupe hydroxy,
h) un atome d'halogène,
i) un groupe alkyle en C₁-C₆ qui est substitué par 1, 2 ou 3 atomes d'halogène ;
j) CONR6R7 ; et
k) NO₂ ;
R12 représente H ou NH₂ ;
R13 et R14, qui peuvent être identiques ou différents, représentent H ou un groupe alkyle en C₁-C₆ ou forment ensemble, avec l'atome d'azote auquel ils sont liés, un hétérocycle non aromatique ayant 5 ou 6 atomes formant le cycle, qui comporte 1 ou 2 hétéroatomes qui sont choisis, indépendamment l'un de l'autre, parmi O et N ;
et leurs isomères optiques, sels et produits de solvatation physiologiquement acceptables.

2. Composés selon la revendication 1, choisis parmi l'une des formules la, Ib et Ic 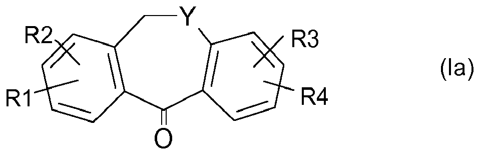 dans laquelle Y représente O et R1, R2, R3 et R4 ont les significations indiquées dans la revendication 1 ; 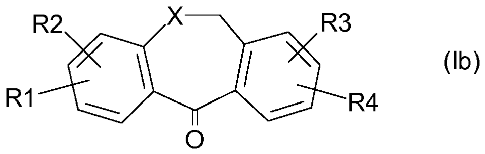 dans laquelle X représente O et R1, R2, R3 et R4 ont les significations indiquées dans la revendication 1 ; et 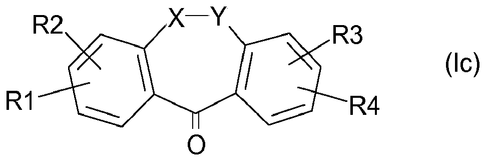 dans laquelle -X-Y- représente -CH₂-CH₂- ou -CH=CH- et R1, R2, R3 et R4 ont les significations indiquées dans la revendication 1.

3. Composés selon l'une quelconque des revendications précédentes, dans lesquels R1 est choisi parmi alkyle en C₁-C₆ qui est substitué par 1, 2 ou 3 groupes hydroxy.

4. Composés selon l'une quelconque des revendications précédentes, dans lesquels R3 est choisi parmi
b) 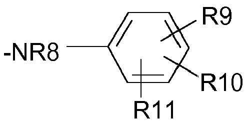
e) phényle et
g) tétrazolo.

5. Composés selon la revendication 4, dans lesquels R9 et R10 sont choisis indépendamment l'un de l'autre parmi H, NH₂, monoalkyl (C₁-C₆) amino, dialkyl(C₁-C₆)amino, un atome d'halogène, CF₃, des groupes alkyle en C₁-C₆ et alcoxy en C₁-C₆ et R11 représente H ou un atome d'halogène.

6. Composés selon la revendication 4 ou 5, dans lesquels R3 représente un radical de formule

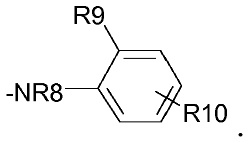

7. Composés selon l'une quelconque des revendications précédentes, dans lesquels R4 représente H.

8. Composés selon l'une quelconque des revendications précédentes, dans lesquels R6 et R7 représentent H.

9. Composés selon l'une quelconque des revendications précédentes, de formule

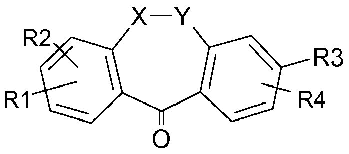

10. Composés selon la revendication 2 de formule Iaa 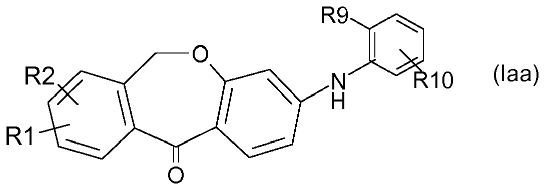 dans laquelle R1, R2, R9 et R10 ont les significations indiquées dans la revendication 1 ou 5.

11. Composés selon la revendication 2, de formule Ica : 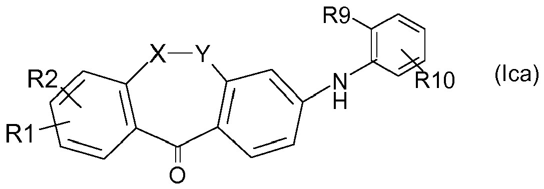 dans laquelle
X-Y- représente -CH₂-CH₂- ou -CH=CH- et R1, R2, R9 et R10 ont les significations indiquées dans la revendication 1 ou 5.

12. Composés selon la revendication 1 :
(1) 2-(2-aminoanilino)-7-méthoxydibenzosubérone ;
(2) 2-(2-amino-4-fluoroanilino)-7-méthoxydibenzosubérone ;
(3) 2-(2,4-difluoroanilino)-7-méthoxydibenzosubérone ;
(4) 2-(2-chloro-4-fluoroanilino)-7-méthoxydibenzosubérone ;
(5) 2-(2,4,5-trifluoranilino)-7-méthoxydibenzosubérone ;
(6) 2-(2-trifluorométhylanilino)-7-méthoxydibenzosubérone ;
(7) 2-(anilino)-7-méthoxydibenzosubérone ;
(8) 2-(2-méthoxyanilino)-7-méthoxydibenzosubérone ;
(9) 2-(3-méthyl-4-fluoroanilino)-7-méthoxydibenzosubérone ;
(10) 2-(2-amino-4-trifluorométhylanilino)-7-méthoxydibenzosubérone ;
(11) 2-(phényl)-7-méthoxydibenzosubérone ;
(12) 2-(2,4-difluoroanilino)-7-méthoxydibenzosubérénone ;
(13) 2-(2,4-difluoroanilino)-7-(S-1,2-isopropylidèneglycér-3-yl)-10,11-dihydro-dibenzo[a,d]-cycloheptén-5-one ;
(14) 2-(2,4-difluoroanilino)-7-(R-1,2-isopropylidèneglycér-3-yl)-10,11-dihydro-dibenzo[a,d]-cyc!oheptén-5-one ;
(15) 2-(2-aminoanilino)-7-(S-1,2-isopropylidèneglycér-3-yl)-10,11-dihydro-dibenzo[a,d]cycloheptén-5-one ;
(16) 2-(2-aminoanilino)-7-(R-1,2-isopropylidèneglycer-3-yl)-10,11-dihydro-dibenzo[a,d]cycloheptén-5-one ;
(17) 2-(2,4-difluoranilino)-7-[2R-,3-dihydroxypropoxy]-10,11-dihydro-dibenzo[a,d]cycloheptén-5-one ;
(18) 2-(2,4-difluoroanilino)7-[2S-,3-dihydroxypropoxy]-10,11-dihydro-dibenzo[a,d]cycloheptén-5-one ;
(19) 2-(2-aminoanilino)-7-[2R-,3-dihydroxypropoxy]-10,11-dihydro-dibenzo[a,d]-cycloheptén-5-one ;
(20) 2-(2-aminoanilino)-7-[2S-,3-dihydroxypropoxy]-10,11-dihydro-dibenzo[a,d]-cycloheptén-5-one ;
(21) 2-(2,4-difluoranilino)-7-(2-hydroxy-éthoxy)-10,11-dihydro-dibenzo[a,d]-cycloheptén-5-one ;
(22) 2-(2,4-difluoranilino)-7-(3-hydroxy-propoxy)-10,11-dihydro-dibenzo[a,d]cycloheptén-5-one ;
(23) (S)-2-(2,4-difluorophénylamino)-8-(2,2-diméthyl-[1,3]dioxolan-4-ylméthoxy)-10,11-dihydro-dibenzo[a,d]-cycloheptén-5-one ;
(24) (R)-2-(2,4-difluorophénylamino)-8-(2,3-dihydroxypropoxy)-10,11-dihydrodibenzo[a,d]cycloheptén-5-one ;
(25) (S)-2-(2-aminophénylamino)-8-(2,2-diméthyl-[1,3]-dioxolan-4-ylméthoxy)-10,11-dihydrodibenzo[a,d]-cycloheptén-5-one ;
(26) (R)-2-(2-aminophénylamino)-8-(2,3-dihydroxypropoxy)-10,11-dihydrodibenzo[a,d]cycloheptén-5-one ;
(27) 2-(2,4-difluorophénylamino)-8-(2-morpholin-4-yl-éthoxy)-10,11-dihydrodibenzo[a,d]cycloheptén-5-one ;
(28) 8-(2,4-difluorophénylamino)-1-méthoxy-10,11-dihydrodibenzo[a,d}cycloheptén-5-one ;
(29) 8-(2-aminophénylamino)-1-méthoxy-10,11-dihydrodibenzo[a,d]cycloheptén-5-one ;
(30) (S)-8-(2,4-difluorophénylamino)-1-(2,2-diméthyl-[1,3]dioxolan-4-ylméthoxy)-10,11-dihydrodibenzo[a,d]-cycloheptén-5-one ;
(31) (R)-8-(2,4-difluorophénylamino)-1-(2,3-dihydroxypropoxy)-10,11-dihydrodibenzo[a,d]cycloheptén-5-one ;
(32) (S)-8-(2-aminophénylamino)-1-(2,2-diméthyl-[1,3]dioxolan-4-ylméthoxy)-10,11-dihydrodibenzo[a,d]-cycloheptén-5-one ;
(33) (R)-8-(2-aminophénylamino)-1-(2,3-dihydroxypropoxy)-10,11-dihydrodibenzo[a,d]cycloheptén-5-one ;
(34) 8-(2,4-difluorophénylamino)-1-(2-morpholin-4-yl-éthoxy)-10,11-dihydrodibenzo[a,d]cycloheptén-5-one ;
(35) 2-(2-méthyl-4-fluoroanilino)-7-méthoxydibenzosubérone ;
(36) 2-(2-chloranilino)-7-méthoxydibenzosubérone.

13. Produit pharmaceutique, contenant au moins un composé de formule I selon l'une quelconque des revendications 1 à 12, éventuellement conjointement avec des excipients physiologiquement acceptables.

14. Composé de formule I selon l'une quelconque des revendications 1 à 12, destiné à l'utilisation dans un procédé pour le traitement de maladies auto-immunes, du cancer, de la polyarthrite rhumatoïde, de la goutte, du choc septique, de l'ostéoporose, de la douleur neuropathique, de la propagation du VIH, de la démence due au VIH, de la myocardite virale, du diabète insulino-dépendant, de maladies parodontales, de la resténose, de l'alopécie, de la déplétion des lymphocytes T dans des infections à VIH ou le SIDA, du psoriasis, de la pancréatite aiguë, de réactions de rejet dans des transplants allogéniques, d'inflammation pulmonaire d'origine allergique, de l'artériosclérose, de la sclérose en plaques, de la cachexie, de la maladie d'Alzheimer, de l'accident vasculaire cérébral, de l'ictère, de la colite ulcéreuse, de la maladie de Crohn, de la maladie intestinale inflammatoire (IBD), de l'ischémie, de l'insuffisance rénale congestive, de la fibrose pulmonaire, de l'hépatite, du glioblastome, du syndrome de Guillain-Barré, du lupus érythémateux disséminé, du syndrome de détresse respiratoire de l'adulte (ARDS) et du syndrome de détresse respiratoire.
